# EUROPEAN PATENT APPLICATION

(11) **EP 2 100 894 A1**
(43) Date of publication of application: **16.09.2009**
(21) Application number: 08102524.9
(22) Date of filing: 12.03.2008
(51) Int. Cl.: C07D 471/04, A61K 31/519, A61P 35/00

(54) **Pyridopyrimidines used as Plk1 (polo-like kinase) inhibitors**

(71) Applicant: 4SC AG, 82152 Planegg - Martinsried (DE)
(72) Inventor: The designation of the inventor has not yet been filed
(74) Representative: Vossius & Partner

(57) **Abstract**

The invention relates to pyridopyrimidinone compounds according to formula (I), 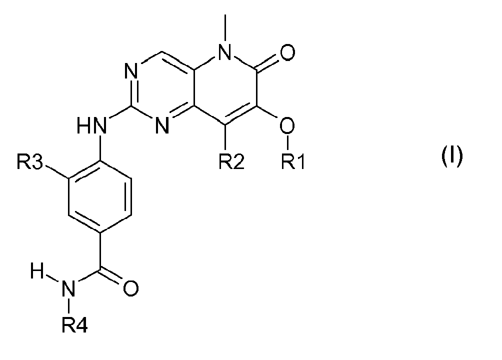 wherein the substituents and symbols are as defined in the description. The compounds are inhibitors of Plk1.

## Description

### Field of application of the invention

The invention relates to pyridopyrimidinone compounds which can be used in the pharmaceutical industry for the manufacture of pharmaceutical compositions. The invention further relates to the contribution made to the art by the finding that said pyridopyrimidinone compounds are potent inhibitors of polo-like kinase 1 (Plk1). This activity is known to result in many cases in a cell cycle-dependent, anti-proliferative and apoptosis inducing activity.

The invention also relates to the use of these compounds for the therapy of hyperproliferative diseases, in particular human cancer.

### Known technical background

Tumor cells are characterized by a partial or complete loss of control of the cell cycle. This loss of cell cycle control results in excessive cell division activity and, thus, in uncontrolled growth. It is known that PIk1, the human orthologue of polo kinase of Drosophila, is an essential regulator of the M-phase of the cell cycle. Targeted interference with Plk1 function in cancer cells such as antisense molecules, siRNA, or antibody microinjection is known to result in mitotic arrest of the cells followed by the onset of cell death. Moreover, it was found that Plk1 is overexpressed in a wide variety of human cancers including, but not limited to breast, prostate, stomach or ovaries.

Inhibitors of PIkl are known from WO 03/20722, WO 03/29248, WO 03/93249,
WO 2004/014899, WO 2004/043936, WO 2004/074244, WO 2004/076454,
WO 2004/087652, WO 2005/005438, WO 2005/019193, WO 2005/037827,
WO 2005/042505, WO 2005/075470, WO 2005/123736, WO 2006/005510,
WO 2006/008028, WO 2006/018182, WO 2006/018185, WO 2006/018220,
WO 2006/018222, WO 2006/021378, WO 2006/021379, WO 2006/021544,
WO 2006/021547, WO 2006/021548, WO 2006/025567, WO 2006/041773,
WO 2006/049339, WO 2006/058876, WO 2007/030359, WO 2007/030361
and WO 2007/030366.

Insufficient susceptibility to known medicines of many tumor types requires the development of novel compounds as chemotherapeutic agents such as, for example, Plk1 inhibiting compounds interfering with cancer cell cycle and/or proliferation. Thus, subject of the present invention are novel PIk1 inhibitors. In particular, the invention relates to pyridopyrimidinone compounds.

The synthesis of a pyrido[3,2-d]pyrimidine derivative is described by W. Pfleiderer and H. Mosthaf in Chemische Berichte 90, 738-45 (1957).

### Description of the invention

It has now been found that the pyridopyrimidinone compounds described in detail below are characterized by surprising and advantageous properties such as, among others, the selective inhibition of Plk1 enzyme. It can be expected that among these Plk1 inhibitors there will be compounds that selectively inhibit proliferation and induce cell death in proliferating cancer cells while being inactive on arrested cells. Moreover it was observed that many of the pyridopyrimidinone compounds arrest proliferating cancer cells in mitosis.

Accordingly, the invention relates to compounds of formula (I) 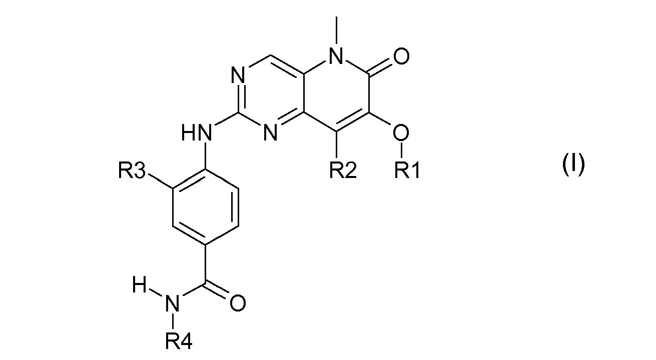 wherein
R1 is C1-C6 alkyl optionally substituted with C1-C4 alkoxy or with 1 to 3 F atoms, C3-C6 cycloalkyl optionally substituted with C1-C4 alkyl or C1-C4 alkoxy, C3-C6 cycloalkyl-C1-C4 alkyl optionally substituted with C1-C4 alkyl, C4-C5 [O]heterocyclyl-C1-C4 alkyl optionally substituted with C1-C4 alkyl, benzyl with phenyl optionally substituted with one or two groups independently selected from R5, or heteroaryl-C1-C2 alkyl with heteroaryl optionally being substituted with one or two groups independently selected from R5;
R2 is H, halogen, C1-C6 alkyl optionally substituted with C1-C4 alkoxy or with 1 to 3 F atoms, C3-C6 cycloalkyl-C1-C4 alkyl, C4-C5 [O]heterocyclylmethyl, C3-C6 cycloalkyl optionally substituted with C1-C4 alkyl or C1-C4 alkoxy, C2-C6 alkenyl, ethinyl optionally substituted with C1-C4 alkyl, C5-C6 cycloalkenyl optionally substituted with C1-C4 alkyl, phenyl optionally substituted with one or two groups independently selected from R5, or heteroaryl optionally substituted with R5;
R3 is halogen, cyano, C1-C4 alkyl optionally substituted with OH or with 1 to 3 F atoms, or C1-C4 alkoxy optionally substituted with OH or with 1 to 3 F atoms;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, cyclopropyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In a preferred embodiment, the invention relates to compounds of formula (I), wherein
R1 is C1-C4 alkyl optionally substituted with C1-C4 alkoxy or with 1 to 3 F atoms, C3-C6 cycloalkyl optionally substituted with C1-C4 alkyl or C1-C4 alkoxy, C3-C6 cycloalkyl-C1-C4 alkyl optionally substituted with C1-C4 alkyl, C4-C5 [O]heterocyclyl-C1-C4 alkyl, benzyl with phenyl optionally substituted with R5, or heteroaryl-C1-C2 alkyl with heteroaryl optionally being substituted with one or two groups independently selected from R5;
R2 is H, halogen, C1-C6 alkyl optionally substituted with C1-C4 alkoxy or with 1 to 3 F atoms, C3-C6 cycloalkyl-C1-C4 alkyl, C4-C5 [O]heterocyclylmethyl, C3-C6 cycloalkyl, C2-C6 alkenyl, ethinyl optionally substituted with C1-C4 alkyl, C5-C6 cycloalkenyl optionally substituted with C1-C4 alkyl, phenyl optionally substituted with R5, or heteroaryl optionally substituted with R5;
R3 is halogen, cyano, C1-C4 alkyl optionally substituted with 1 to 3 F atoms, or C1-C4 alkoxy optionally substituted with 1 to 3 F atoms;
R4 is C1-C4 alkyl, C2-C3 alkyl optionally substituted with -OR6, C2-C3 alkyl optionally substituted with -N(R6)R7, C3-C6 cycloalkyl, C1-C3 alkyl substituted with heteroaryl, the heteroaryl optionally being substituted with one or two groups independently selected from R5, 3-tetrahydropyranyl, 4-tetrahydropyranyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, cyclopropyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl optionally substituted with -F, C3-C6 cycloalkyl, C3-C6 cycloalkyl-C1-C4 alkyl optionally substituted with methyl, C4-C5 [O]heterocyclylmethyl, benzyl or heteroaryl-C1-C2 alkyl with heteroaryl optionally being substituted with one or two groups independently selected from R5;
R2 is H, halogen, C1-C6 alkyl optionally substituted with C1-C4 alkoxy or with 1 to 3 F atoms, C3-C6 cycloalkyl-C1-C4 alkyl, C4-C5 [O]heterocyclylmethyl, C3-C6 cycloalkyl, C2-C6 alkenyl, ethinyl optionally substituted with C1-C4 alkyl, C5-C6 cycloalkenyl optionally substituted with C1-C4 alkyl, phenyl optionally substituted with R5, or heteroaryl optionally substituted with R5;
R3 is F, Cl, C1-C4 alkyl optionally substituted with 1 to 3 F atoms, or C1-C4 alkoxy optionally substituted with 1 to 3 F atoms;
R4 is C2-C3 alkyl optionally substituted with -OR6, C2-C3 alkyl optionally substituted with -N(R6)R7, C3-C6 cycloalkyl, C1-C3 alkyl substituted with heteroaryl, the heteroaryl optionally being substituted with R5, 3-tetrahydropyranyl, 4-tetrahydropyranyl, 4-piperidyl optionally N-substituted by C1-C4 alkyl or C3-C6 cycloalkyl, 4-piperidyl-C1-C2 alkyl, the piperidyl optionally being N-substituted by C1-C4 alkyl or C3-C6 cycloalkyl, or 2-pyrrolidinyl-C1-C2 alkyl, the pyrrolidinyl optionally being N-substituted by C1-C4 alkyl or C3-C6 cycloalkyl;
R5 is halogen, cyano, C1-C4 alkyl, cyclopropyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl optionally substituted with C1-C4 alkoxy or with 1 to 3 F atoms, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is -Cl, C1-C4 alkyl, or C1-C4 alkoxy;
R4 is C1-C3 alkyl substituted with heteroaryl, the heteroaryl optionally being substituted with R5, 3-tetrahydropyranyl, 4-tetrahydropyranyl, 4-piperidyl optionally N-substituted by C1-C4 alkyl or cyclopropyl, 4-piperidyl-C1-C2 alkyl, the piperidyl optionally being N-substituted by C1-C4 alkyl or cyclopropyl, or 2-pyrrolidinyl-C1-C2 alkyl, the pyrrolidinyl optionally being N-substituted by C1-C4 alkyl or C3-C6 cycloalkyl
and
R5 is halogen, cyano, C1-C4 alkyl, cyclopropyl, or C1-C4 alkoxy;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, cyclopropyl, cyclobutyl, cyclopropylmethyl, 1-methylcyclopropylmethyl, cyclobutylmethyl, or 1-cyclopropylethyl;
R2 is cyclopropyl, (1 E)-prop-1-en-1-yl, isopropenyl, cyclohex-1-en-1-yl optionally substituted with C1-C4 alkyl, cycolopent-1-en-1-yl optionally substituted with C1-C4 alkyl, phenyl optionally substituted with C1-C4 alkyl or F, 2-thienyl optionally substituted with C1-C4 alkyl, 3-thienyl optionally substituted with C1-C4 alkyl, 2-furyl optionally substituted with C1-C4 alkyl, 3-furyl optionally substituted with C1-C4 alkyl, 1H-pyrrol-3-yl optionally substituted with C1-C4 alkyl, or 1H-pyrrol-2-yl optionally substituted with C1-C4 alkyl;
R3 is Cl, methyl, ethyl, methoxy, or ethoxy;
and
R4 is 5-imidazolylmethyl, the imidazolyl optionally being N-substituted by C1-C4 alkyl or cyclopropyl, 4-piperidyl optionally N-substituted by C1-C4 alkyl or cyclopropyl, 4-piperidylmethyl, the piperidyl optionally being N-substituted by C1-C4 alkyl or cyclopropyl, or 2-pyrrolidinylethyl, the pyrrolidinyl optionally being N-substituted by C1-C4 alkyl or cyclopropyl;
a salt of one of the compounds, a stereoisomer of one of the compounds, or a salt of a stereoisomer of one of the compounds.

In another preferred embodiment, the invention relates to compounds of formula (I), wherein
R1 is 1-4C-Alkyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is cyclopropylmethyl, cyclobutylmethyl, 1-cyclopropylethyl, 1-methylcyclopropylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, heteroaryl optionally substituted with C1-C4 alkyl or halogen;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is cyclopropyl or cyclobutyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, heteroaryl optionally substituted with C1-C4 alkyl or halogen;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl optionally substituted with F, C3-C6 cycloalkyl, C3-C6 cycloalkyl-C1-C4 alkyl optionally substituted with methyl, C4-C5 [O]heterocyclylmethyl, benzyl, or heteroaryl-C1-C2 alkyl with heteroaryl optionally being substituted with one or two groups independently selected from R5;
R2 is C3-C6 cycloalkyl;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl optionally substituted with F, C3-C6 cycloalkyl, C3-C6 cycloalkyl-C1-C4 alkyl optionally substituted with methyl, C4-C5 [O]heterocyclylmethyl, benzyl, or heteroaryl-C1-C2 alkyl with heteroaryl optionally being substituted with one or two groups independently selected from R5;
R2 is C2-C6 alkenyl;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl optionally substituted with F, C3-C6 cycloalkyl, C3-C6 cycloalkyl-C1-C4 alkyl optionally substituted with methyl, C4-C5 [O]heterocyclylmethyl, benzyl, or heteroaryl-C1-C2 alkyl with heteroaryl optionally being substituted with one or two groups independently selected from R5;
R2 is C5-C6 cycloalkenyl optionally substituted with C1-C4 alkyl;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, cyclopropyl, cyclobutyl, cyclopropylmethyl, 1-methylcyclopropylmethyl, cyclobutylmethyl, 1-cyclopropylethyl;
R2 is phenyl optionally substituted with one or two groups independently selected from R5 or heteroaryl optionally substituted with R5;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, cyclopropyl, cyclobutyl, cyclopropylmethyl, 1-methylcyclopropylmethyl, cyclobutylmethyl, or 1-cyclopropylethyl;
R2 is 2-thienyl optionally substituted with C1-C4 alkyl, 3-thienyl optionally substituted with C1-C4 alkyl, 2-furyl optionally substituted with C1-C4 alkyl, 3-furyl optionally substituted with C1-C4 alkyl, 1H-pyrrol-3-yl optionally substituted with C1-C4 alkyl, or 1 H-pyrrol-2-yl optionally substituted with C1-C4 alkyl;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is methyl or ethyl;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is methoxy or ethoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is C1-C4 alkyl substituted with heteroaryl, the heteroaryl optionally being substituted with one or two groups independently selected from R5;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is 4-piperidyl, 4-piperidylmethyl, the piperidyl optionally being N-substituted by C1-C4 alkyl or cyclopropyl, or 4-piperidylethyl, the piperidyl optionally being N-substituted by C1-C4 alkyl or cyclopropyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is 2-pyrrolidinylmethyl, the pyrrolidinyl optionally being N-substituted by C1-C4 alkyl or cyclopropyl, or 2-pyrrolidinylethyl, the pyrrolidinyl optionally being N-substituted by C1-C4 alkyl or cyclopropyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is N-(1-methylpiperidin-4-yl) or N-(1-methylpiperidin-4-yl)methyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is N-[2-(1-methylpyrrolidin-2-yl)ethyl];
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is 3-tetrahydropyranyl or 4-tetrahydropyranyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is 5-imidazolylmethyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I), wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is 2-pyrrolidinylmethyl, the pyrrolidinyl optionally being N-substituted by C1-C4 alkyl or cyclopropyl, or 2-pyrrolidinylethyl, the pyrrolidinyl optionally being N-substituted by C1-C4 alkyl or cyclopropyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

In another preferred embodiment, the invention relates to compounds of formula (I),
wherein R1 is cyclopropylmethyl and R2 - R7 are defined as described above.

In another preferred embodiment, the invention relates to compounds of formula (I),
wherein R2 is (1 E)-prop-1-en-1-yl and R1 and R3 - R7 are defined as described above.

In another preferred embodiment, the invention relates to compounds of formula (I),
wherein R2 is 3-thienyl and R1 and R3 - R7 are defined as described above.

In another preferred embodiment, the invention relates to compounds of formula (I),
wherein R2 is 1 H-pyrrol-2-yl and R1 and R3 - R7 are defined as described above.

In another preferred embodiment, the invention relates to compounds of formula (I),
wherein R3 is methoxy and R1, R2 and R4 - R7 are defined as described above.

C1-C6 alkyl represents a straight-chain or branched alkyl group having 1 to 6 carbon atoms, including methyl, ethyl, propyl, isopropyl, butyl, isobutyl, sec-butyl, tert-butyl, pentyl and isomers thereof, and hexyl and isomers thereof.

C1-C4 alkyl represents a straight-chain or branched alkyl group having 1 to 4 carbon atoms, including methyl, ethyl, propyl, isopropyl, butyl, isobutyl, sec-butyl, and tert-butyl.

C1-C3 alkyl represents an alkyl group having 1 to 3 carbon atoms, including methyl, ethyl, propyl, and isopropyl.

C2-C3 alkyl represents an alkyl group having 2 or 3 carbon atoms, including ethyl, propyl, and isopropyl.

C2-C6 alkenyl represents a straight-chain or branched alkenyl group having 2 to 6 carbon atoms, including ethenyl, prop-1-enyl, prop-2-enyl, but-1-enyl and its isomers, pent-1-enyl and its isomers, and hex-1-enyl and its isomers.

C2-C4 alkenyl represents a straight-chain or branched alkenyl group having 2 to 4 carbon atoms, including for example ethenyl, prop-1-enyl, prop-2-enyl, and but-1-enyl and its isomers.

C5-C6 cycloalkenyl represents an unsaturated, 5 to 6 membered carbocycle with one double bond, including cyclopentenyl and cyclohexenyl.

C1-C4 alkoxy represents groups which, in addition to the oxygen atom, contain a straight chain or branched C1-C4 alkyl group as outlined above. Accordingly, C1-C4 alkoxy includes methoxy, ethoxy, propoxy, isopropoxy, butoxy, isobutoxy, sec-butoxy, and tert-butoxy.

C3-C6 cycloalkyl represents the cyclopropyl, cyclobutyl, cyclopentyl, and cyclohexyl groups.

C3-C6 cycloalkyl-C1-C4 alkyl represents C1-C4 alkyl as defined above, substituted by C3-C6 cycloalkyl as defined above. For example, C3-C6 cycloalkyl-C1-C4 alkyl can be C3-C6 cycloalkylmethyl such as, e.g., cyclopropylmethyl, cyclobutylmethyl, cyclopentylmethyl or cyclohexylmethyl. Optionally, the C3-C6 cycloalkyl-C1-C4 alkyl can be further substituted by C1-C4 alkyl at either the alkyl or the cycloalkyl moiety.

C4-C5 [O]heterocyclyl-C1-C4 alkyl represents C1-C4 alkyl as defined above, substituted by C4-C5 [O]heterocyclyl. C4-C5 [O]heterocyclyl is a five- or six-membered saturated heterocycle with one ring atom being O. C4-C5 [O]heterocyclyl includes tetrahydrofuranyl and tetrahydropyranyl. For example, C4-C5 [O]heterocyclyl-C1-C4 alkyl can be C4-C5 [O]heterocyclylmethyl such as, e.g., tetrahydrofuranylmethyl or tetrahydropyranylmethyl. Optionally, the C4-C5 [O]heterocyclyl-C1-C4 alkyl can be further substituted by C1-C4 alkyl at either the alkyl or the [O]heterocyclyl moiety.

Heteroaryl represents a 5 or 6 membered aromatic ring with one or two heteroringatoms independently selected from N, O and S. Examples for heteroaryl are furyl, thienyl, pyrrolyl, oxazolyl, isoxazolyl, thiazolyl, isothiazolyl, imidazolyl, pyrazolyl, triazolyl, thiadiazolyl, oxadiazolyl, pyridinyl, pyrazinyl, pyridazinyl and pyrimidinyl.

Heteroaryl-C1-C2 alkyl is methyl or ethyl substituted by heteroaryl as defined above.

C4-C6 [N]heterocyclyl is a saturated or unsaturated 5 to 7 membered heterocycle with one ring atom being N. Examples for C4-C6 [N]heterocyclyl are azepanyl, piperidinyl, and pyrrolidinyl.

C4-C6 [N]heterocyclyl-C1-C3 alkyl represents C1-C3 alkyl as defined above, substituted by C4-C6 [N]heterocyclyl as defined above. Examples for C4-C6 [N]heterocyclyl-C1-C3 alkyl are azepanylmethyl, azepanylethyl, azepanylpropyl, piperidinylmethyl, piperid-inylethyl, piperidinylpropyl, pyrrolidinylmethyl, pyrrolidinylethyl, and pyrrolidinylpropyl.

It is to be understood that the invention covers all combinations of substituent groups referred to hereinabove. In particular, the invention covers all combinations of preferred groups and substitution patterns described herein.

Salts of the compounds according to the invention and the stereoisomers of the salts include all inorganic and organic acid addition salts, particularly all pharmaceutically acceptable inorganic and organic acid addition salts customarily used in pharmacy.
Examples of acid addition salts include, but are not limited to, hydrochlorides, hydrobromides, phosphates, nitrates, sulfates, acetates, trifluoroacetates, citrates, D-gluconates, benzoates, 2-(4-hydroxybenzoyl)benzoates, butyrates, sulfosalicylates, maleates, laurates, malates, lactates, fumarates, succinates, oxalates, tartrates, stearates, benzenesulfonates (besylates), toluenesulfonates (tosylates), methanesulfonates (mesylates), laurylsulfonates, 3-hydroxy-2-naphthoates, lactobionates, galactarates, embonates and ascorbates.

The salts include water-insoluble (or practically insoluble) and, particularly, water-soluble salts.

The compounds according to the invention, the salts thereof and the stereoisomers of the compounds or the salts thereof may contain, e.g., when isolated in crystalline form, varying amounts of solvents. Included within the scope of the invention are, therefore, all solvates of the compounds of formula (I), the salts thereof, and the stereoisomers of the compounds and the salts thereof. Hydrates are a preferred example of said solvates.

The invention further relates to stereoisomers of formula (I). The invention also relates to mixtures of stereoisomers, including the racemates.

Some of the compounds, salts thereof, and stereoisomers of the compounds or the salts thereof may exist in different crystalline forms (polymorphs) as well as in amorphous forms, which are intended to be within the scope of the invention.

Furthermore, derivatives of the compounds of formula (I), the salts thereof, stereoisomers of the compounds or the salts thereof which are converted into compound (I), a salt thereof, or a stereoisomer of the compound or a salt thereof in a biological system (bio-precursors or prodrugs) are covered by the invention. Said biological system is, e.g., a mammalian cell or a mammalian organism, particularly a human subject. The prodrug is, for example, converted into the compound of formula (I), a salt thereof, or a stereoisomer of the compound or a salt thereof by metabolic processes. For example, prodrugs may be esters of the compounds of formula (I) which can be readily hydrolyzed after uptake by a biological systems, e.g., by an esterase, to yield unmodified compound of formula (I).

The compounds according to the invention can be prepared as follows:

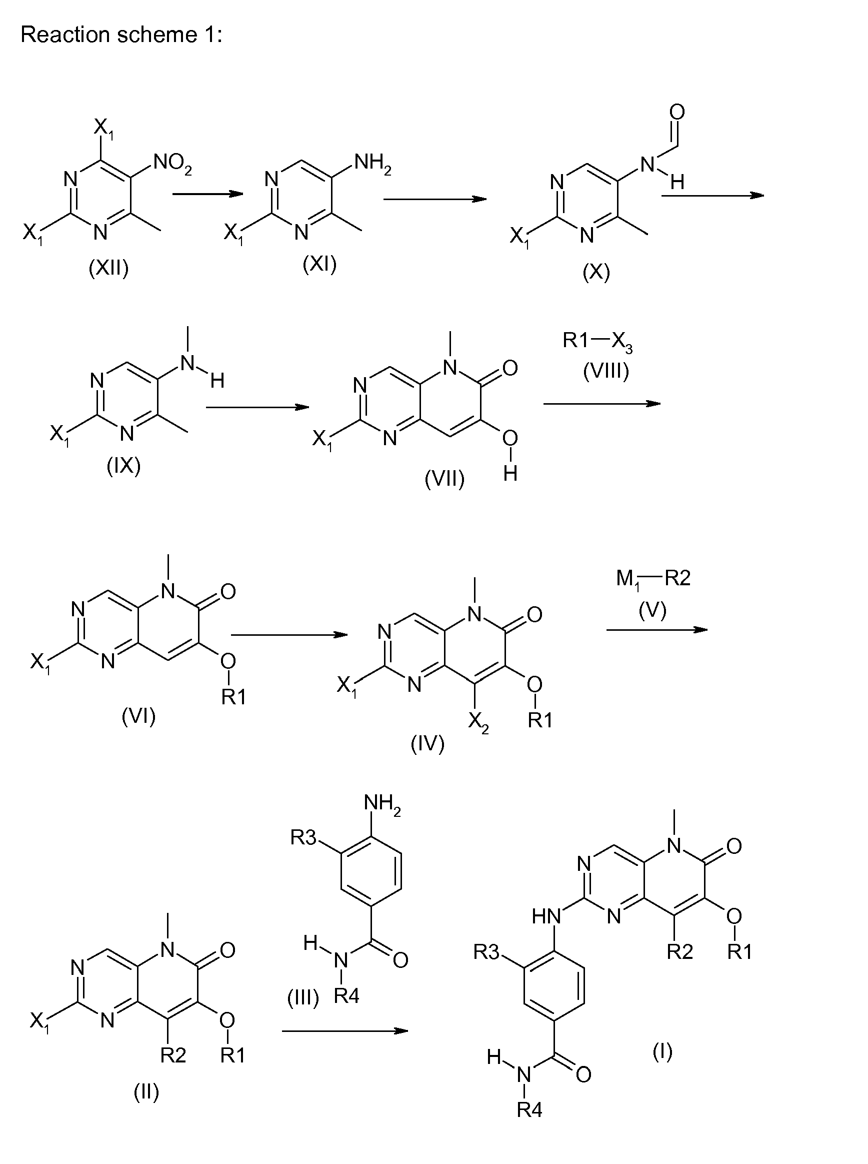

Compound of formula (I) wherein R1, R2, R3 and R4 have the above mentioned meanings can for example be prepared by reaction of a corresponding compound of formula (II) wherein X₁ has the meaning of a halogen, preferably Cl, with compounds of formula (III) wherein R3 and R4 have the above mentioned meanings. If desired, this reaction can be carried out in the presence of a suitable catalyst, e.g. palladium / 2,2'-bis-(diphenylphosphino)-1,1'-binaphthyl or palladium / 4,5-bis(diphenylphosphino)-9,9-dimethylxanthene complexes in solvents such as, e.g., toluene at elevated temperatures (80-110°C) in the presence of a base such as, e.g., Cs₂CO₃ or NaOtBu. Compounds of formula (III) can be prepared as outlined in reaction scheme 2.

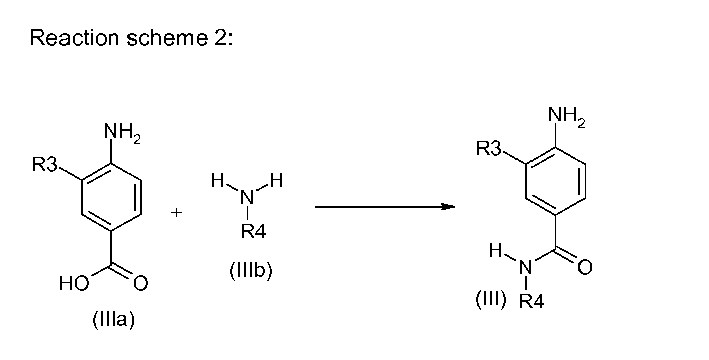

Compounds of formula (III) can be prepared by reaction of an amine of formula (IIIb) with a carboxylic acid of formula (IIIa) in the presence of an appropriate coupling agent such as resin-supported N-[3-(benzyloxy)propyl]-N'-cyclohexylcarbodiimide and 1 H-benzotriazol-1-ol (HOBT) or dicyclohexylcarbodiimide and HOBT in a suitable solvent such as dichloromethane in the presence of a base such as triethylamine. Compounds of formulae (IIIa) and (IIIb) are either commercially available or can be prepared by methods known to the person skilled in the art.

Compounds of formula (II) wherein R1, R2 and X₁ have the meaning described above can be prepared by reaction of a corresponding compound of formula (IV) wherein X₂ has the meaning of a halogen, preferentially of Br, with a compound of formula (V) wherein R2 has the meaning described above and M1 has the meaning of a group suitable for C-C bond formation with aryl halides. Examples of M1-R2 wherein R2 has the meaning described above are boronic acids, boronic esters, R2-Sn(C1-C4-alkyl)₃, R2-ZnCl, R2-ZnBr, R2-Znl, R2-Li, R2-MgCl, R2-MgBr, and R2-Mgl. This reaction is preferably catalyzed by a transition metal compound. Accordingly, if R2-M1 is a boronic acid or boronic ester, this reaction can be carried out by using a palladium catalyst such as, e.g., Pd(OAc)₂, Pd(PPh₃)₄ or Pd(dppf)Cl₂ CH₂Cl₂ with a base such as KF or triethylamine in a solvent such as toluene, MeOH, isopropanol, dioxane or acetonitrile/water at elevated temperatures (60-110°C). Optionally the reaction can be performed at microwave conditions at elevated temperatures (e.g. 120°C). In cases where R2-M1 means R2-Sn(C1-C4-alkyl)₃, this reaction can for example be carried out by using a palladium catalyst such as Pd(OAc)₂ or Pd(PPh₃)₄ in a solvent such as THF at elevated temperatures (60-90°C).

Compounds of formula (IV) wherein R1, X₁ and X₂ have the above mentioned meanings can for example be prepared from corresponding compounds of formula (VI). When X₂ has the meaning of Br, this transformation can be achieved by reaction with a suitable bromination reagent such as, e.g., NBS. The reaction may be carried out in a suitable solvent such as, e.g., DMF and may be heated to a temperature of from about 60 to 90°C.
Compounds of formula (VI) wherein R1 and X₁ have the above mentioned meaning can be prepared by reaction of a corresponding compound of formula (VII) with compounds of formula (VIII) wherein X₃ has the meaning of a halogen, a sulfonester, a hydroxygroup or an aryl triazene. This reaction can for example be performed in DMF in the presence of a base such as, e.g., DBU at elevated temperature (60-100°C) in cases where X₃ is a halogen or sulfonester. If X₃ is a hydroxy group, the reaction can be performed under Mitsunobu conditions (e.g., by the use of diethyl azodicarboxylate and triphenylphosphine in THF). Further details can be found, e.g., in Hughes, D.L., Organic Reactions (1992), Vol. 42, 335-656; and in Mitsunobu, O., Synthesis (1981), 1-28. In cases where X₃ is an aryl triazene such as (1E)-1-(4-bromophenyl)-3-cyclopropyltriaz-1-ene, this reaction can be accomplished by stirring the reaction mixture at room temperature. (1E)-1-(4-bromophenyl)-3-cyclopropyltriaz-1-ene can be prepared by reaction of commercially available 4-bromobenzenediazonium tetrafluoroborate with cyclopropylamine.

Compounds of formula (VII) wherein X₁ has the above mentioned meaning can be prepared from a corresponding compound of formula (IX). This conversion can for example be achieved by reacting compounds of formula (IX) with diethyloxalate in the presence of a base such as KOtBu (potassium tert-butoxide) in DMF at temperatures such as 30-60°C.

Compounds of formula (IX) wherein X₁ has the above mentioned meaning can be prepared by reduction of corresponding compounds of formula (X). This reduction can for example be accomplished by the use of borane-dimethylsulfide complex in THF for example at room temperature.

Compounds of formula (X) wherein X₁ a has the above mentioned meaning can be prepared for example from corresponding compounds of formula (XI). This reaction can for example be achieved by adding the compound of formula (XI) to a mixture of formic acid and acetic acid anhydride which is heated to 50°C for an hour prior to the addition of the compound of formula (XI).

Compounds of formula (XI) wherein X₁ has the meaning described above can be prepared by hydrogenation of corresponding compounds of formula (XII) (which are either commercially available or can be prepared by methods known to the person skilled in the art). Preferably the hydrogenation of compounds of formula XII yielding compounds of formula XI is carried out in two steps. First, a mixture of commercially available 2,4-dichloro-6-methyl-5-nitropyrimidine (X₁ being Cl in formula XII) and 10% Pd/C in ethyl acetate is hydrogenated at a pressure of 40 psi of hydrogen. In a second step, a mixture of the product of the first step (2,4-dichloro-6-methylpyrimidin-5-amine, X₁ being Cl), triethylamine and 10% Pd/C in ethanol is hydrogenated at a pressure of 20 psi of hydrogen to give 2-chloro-4-methylpyrimidin-5-amine (X₁ being Cl in formula XI).

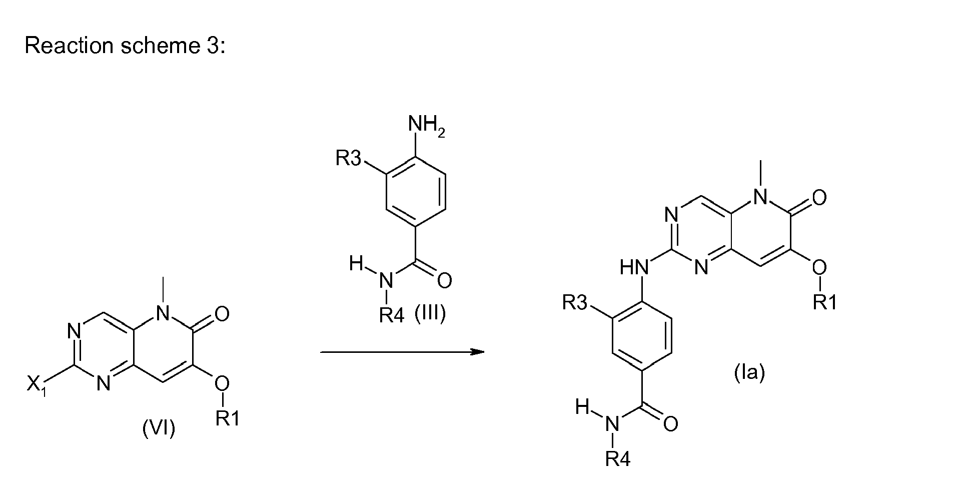

Compounds of formula (Ia) wherein R1, R3 and R4 have the meanings described above are a subtype of compounds of formula (I) with R2 being H. Compounds of formula (la) can be prepared by reaction of compounds of formula (VI) with compounds of formula (III). This reaction can for example be carried out in the presence of a suitable catalyst such as palladium / 2,2'-bis-(diphenylphosphino)-1,1'-binaphthyl or palladium / 4,5-bis(diphenyl-phosphino)-9,9-dimethylxanthene complexes in a solvent such as toluene at elevated temperatures (80-110°C) in the presence of a base such as, e.g., Cs₂CO₃ or NaOtBu. Compounds of formula (III) can be prepared by art-known methods. Compounds of formula (VI) can be prepared as outlined in reaction scheme 1.

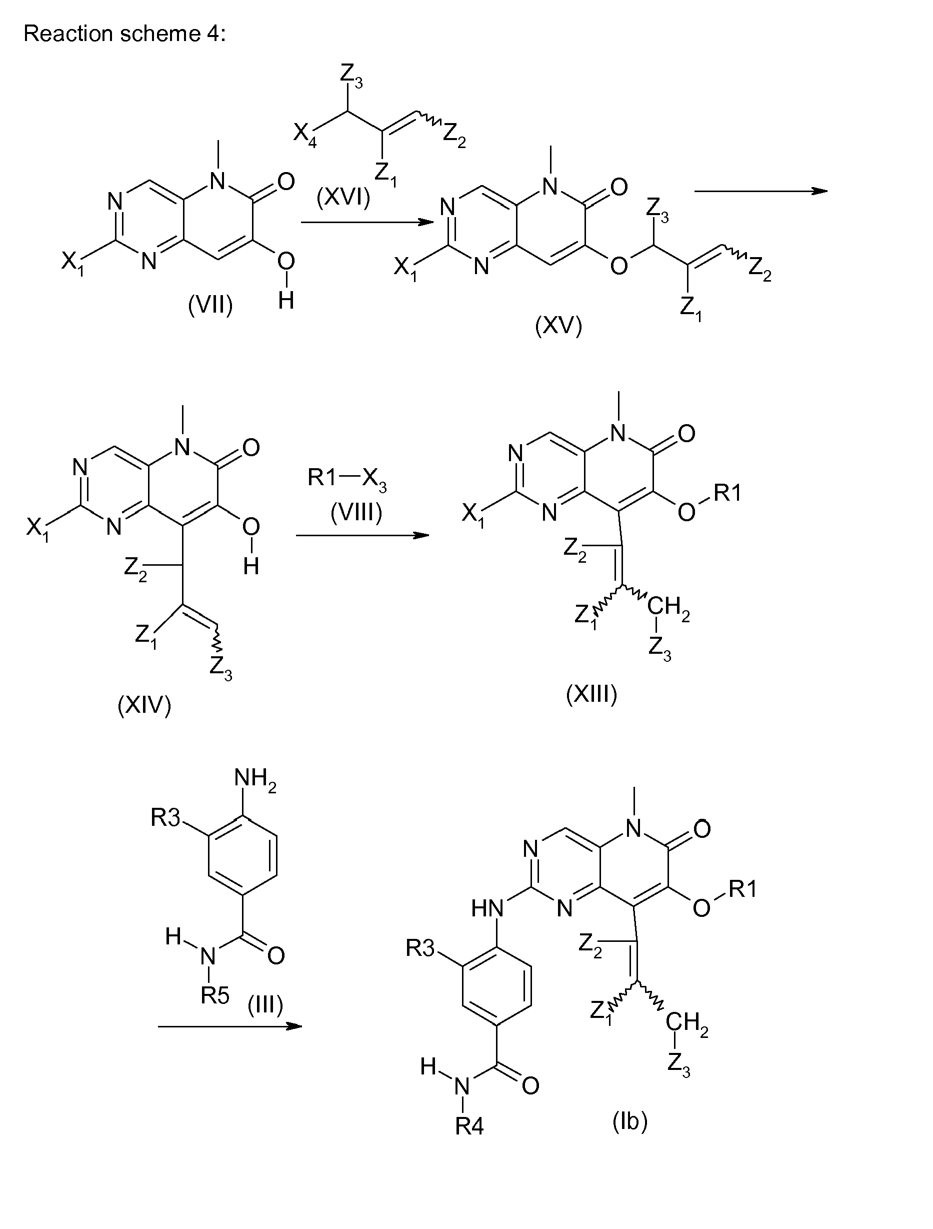

Sinuous lines in scheme 4 indicate either (E) or (Z) configuration of the double bond. Compounds of formula (Ib) wherein R1, R3 and R4 have the meanings described above and either wherein Z1, Z2 and Z3 have the meaning of hydrogen or substituted carbon or
wherein Z1 together with Z2 forms a five- or six-membered carbocycle and Z3 has the meaning of hydrogen or substituted carbon, are a subtype of compounds of formula (I) with R2 of formula (I) being either C5-C6 cycloalkenyl (optionally substituted by C1-C4 alkyl) or C3-C6 alkenyl (those compounds of formula (I) with R2 being C2 alkenyl can be synthesized according to reaction scheme 4).

Compounds of formula (Ib) can be prepared by reaction of compounds of formula (XIII) with compounds of formula (III). This reaction can be carried out in the presence of a suitable catalyst such as, e.g., palladium / 2,2'-bis-(diphenylphosphino)-1,1'-binaphthyl or palladium / 4,5-bis(diphenylphosphino)-9,9-dimethylxanthene complexes in solvents such as toluene at elevated temperatures (80-110°C) in the presence of a base such as, e.g., Cs₂CO₃ or NaOtBu. Compounds of formula (III) can be prepared by art-known methods.

Compounds of formula (XIII) wherein X₁, R1, Z1, Z2 and Z3 have the meaning described above can be prepared from a corresponding compound of formula (XIV). This conversion involves two reactions, an alkylation of the hydroxy group of compounds of formula (XIV) and an isomerisation of the double bond from the position outlined for compound of formula (XIV) to the position outlined for compounds of formula (XIII). The alkylation can be accomplished by reaction of a corresponding compound of formula (XIV) with compounds of formula (VIII) wherein X₃ is a halogen, a sulfonester or a hydroxygroup. In cases where X₃ is a halogen or a sulfonester, suitable conditions for this alkylation include subjecting compounds of formula (XIV) to basic conditions at elevated temperatures (60-100°C) employing, e.g., DBU in DMF. In cases where X₃ is a hydroxy group, alkylation can be achieved under Mitsunobu conditions, for example by the use of diethyl azodicarboxylate and triphenylphosphine in THF. Further details can be found, e.g., in Hughes, D.L., Organic Reactions (1992), 42, 335-656; and in Mitsunobu, O., Synthesis (1981), 1-28. Isomerisation of the double bond can be achieved in the presence of a base such as, e.g., DBU. If the alkylation is performed under basic conditions, both the isomerisation of the double bond and the alkylation reactions can be performed in a one pot procedure, for example by reacting compounds of formula (XIV) with compounds of formula (VIII) in the presence of DBU as a base in DMF at elevated temperatures (60 - 100°C).

Compounds of formula (XIV) wherein X₁, Z1, Z2 and Z3 have the above mentioned meanings can be prepared by rearrangement of corresponding compounds of formula (XV). This rearrangement can be achieved for example by heating (conventional heating or heating via microwave irradiation) compounds of formula (XV) in a suitable solvent such as chlorobenzene at temperatures such as 80-140°C. In certain cases heating at 200 °C without solvent by microwave irradiation is preferred.

Compounds of formula (XV) wherein X₁, Z1, Z2 and Z3 have the above mentioned meanings can be prepared by reactions of corresponding compounds of formula (XVI) wherein X₄ is a halogen, a sulfonester or the hydroxy group with corresponding compounds of formula (VII). In case that X₄ is a halogen or a sulfonester, this reaction can for example be performed in DMF in the presence of a base such as, e.g., DBU at elevated temperatures (60-100°C). When X₄ is a hydroxy group, this reaction can be achieved under Mitsunobu conditions for example by the use of diethyl azodicarboxylate and triphenylphosphine in THF. Further details can be found, e.g., in Hughes, D.L., Organic Reactions (1992), 42, 335-656; and in Mitsunobu, O., Synthesis (1981), 1-28.

Compounds of formula (VII) can be prepared as outlined in reaction scheme 1, and compounds of formula (XVI) are either commercially available or can be prepared by methods known to those skilled in the art.

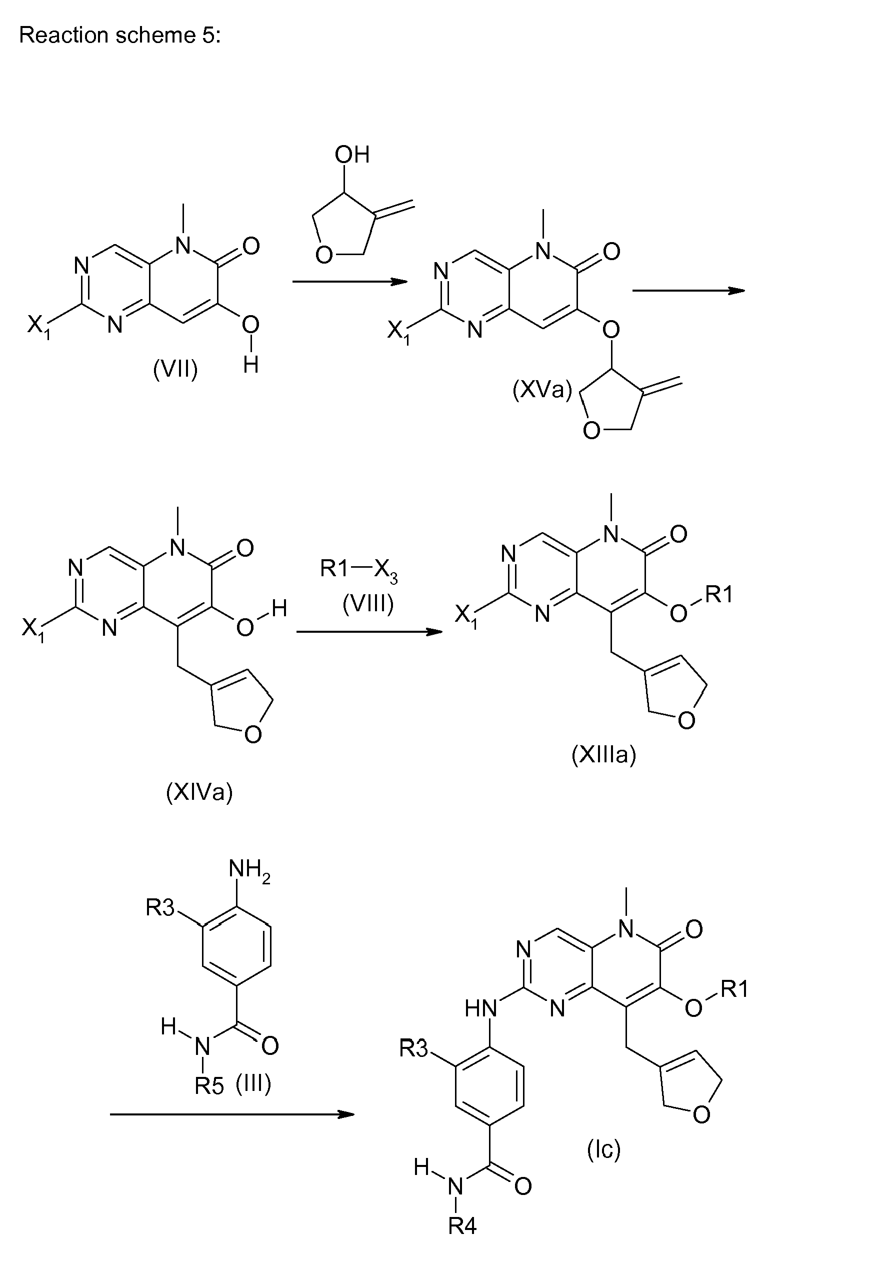

Compounds of formula (Ic) wherein R1, R3 and R4 have the meanings described above can be synthesized by reaction of compounds of formula (XIIIa) with compounds of formula (III), according to scheme 5. This reaction can be carried out in the presence of a suitable catalyst such as, e.g., palladium / 2,2'-bis-(diphenylphosphino)-1,1'-binaphthyl or palladium / 4,5-bis(diphenylphosphino)-9,9-dimethylxanthene complexes in solvents such as toluene at elevated temperatures (80-110°C) in the presence of a base such as, e.g., Cs₂CO₃ or NaOtBu. Compounds of formula (III) can be prepared by art-known methods.

Compounds of formula (XIIIa) wherein X₁ and R1 have the meaning described above can be prepared from a corresponding compound of formula (XIVa) by alkylation of the hydroxy group.The alkylation can be accomplished by reaction of a corresponding compound of formula (XIVa) with compounds of formula (VIII) wherein X₃ is a halogen, a sulfonester or a hydroxygroup. In cases where X₃ is a halogen or a sulfonester, suitable conditions for this alkylation include subjecting compounds of formula (XIVa) to basic conditions at elevated temperatures (60-100°C) employing, e.g., DBU in DMF. In cases where X₃ is a hydroxy group, alkylation can be achieved under Mitsunobu conditions, for example by the use of diethyl azodicarboxylate and triphenylphosphine in THF. Further details can be found, e.g., in Hughes, D.L., Organic Reactions (1992), 42, 335-656; and in Mitsunobu, O., Synthesis (1981), 1-28.

Compounds of formula (XIVa) wherein X₁ has the above mentioned meaning can be prepared by rearrangement of corresponding compounds of formula (XVa). This rearrangement can be achieved for example by heating (conventional heating or heating via microwave irradiation) compounds of formula (XVa) in a suitable solvent such as chlorobenzene at temperatures such as 80-140°C.

Compounds of formula (XVa) wherein X₁ has the above mentioned meaning can be prepared by reaction with 4-methylenetetrahydrofuran-3-ol (synthesized starting from commercially available 3,6-dioxabicyclo[3.1.0]hexane as described in Organic Letters 2001, 4051 - 4053) with corresponding compounds of formula (VII). This reaction can be achieved under Mitsunobu conditions for example by the use of diethyl azodicarboxylate and triphenylphosphine in THF. Further details can be found, e.g., in Hughes, D.L., Organic Reactions (1992), 42, 335-656; and in Mitsunobu, O., Synthesis (1981), 1-28.

Compounds of formula (VII) can be prepared as outlined in reaction scheme 1.

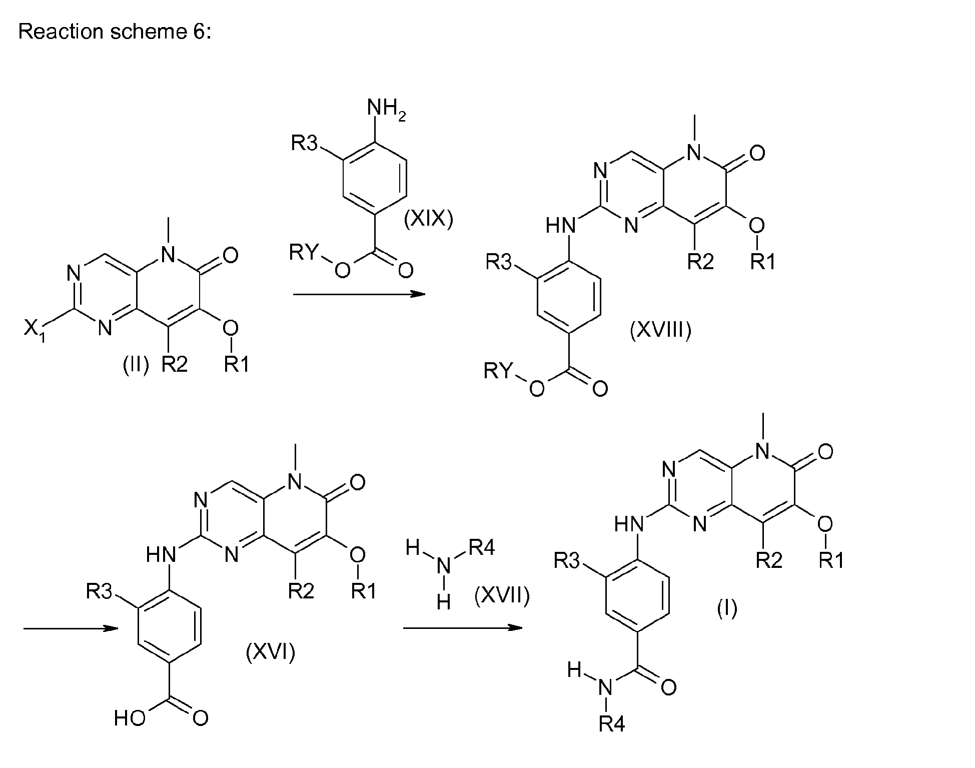

In an alternative scheme (reaction scheme 6), compounds of formula (I) wherein R1, R2, R3 and R4 have the above mentioned meaning can be prepared by reacting compounds of formula (XVI) with amines of formula (XVII) using conventional amide bond coupling reaction conditions. Typically this reaction is carried out in an inert solvent using a suitable coupling reagent such as e.g. N-[3-(dimethylamino)propyl]-N'-ethylcarbodiimide (EDC) and 1H-benzotriazol-1-ol (HOBT) in the presence of a base such as, e.g., triethylamine. Examples of suitable solvents for this reaction include but are not limited to dichloromethane and dimethylformamide.

Compounds of formula (XVII) wherein R4 has the above mentioned meaning are either commercially available or can be prepared by methods known to those skilled in the art.

Compounds of formula (XVI) wherein R1, R2 and R3 have the meanings mentioned above can be prepared from corresponding compounds of formula (XVIII) wherein RY is C1-C4 alkyl. This reaction can be performed at conditions typical for the cleavage of esters such as, e.g., the use of NaOH in a mixture of MeOH and water at elevated temperatures (60-100°C).

Compounds of formula (XVIII) wherein R1, R2 and R3 have the meanings mentioned above can be prepared by reacting compounds of formula (II) wherein X₁ is a halogen, preferably Cl, with compounds of formula (XIX) wherein RY and R3 have the meanings described above. This reaction can for example be carried out in the presence of a suitable catalyst such as, e.g., palladium / 2,2'-bis-(diphenylphosphino)-1,1'-binaphthyl or palladium / 4,5-bis(diphenylphosphino)-9,9-dimethylxanthene complexes in solvents such as toluene at elevated temperatures (80-110°C) in the presence of a base such as, e.g, Cs₂CO₃ or NaOtBu.

Compounds of formula (XIX) can be prepared by art-known methods. Compounds of formula (II) can be prepared as outlined in reaction scheme 1 or as outlined for compounds of formula (XIII) in reaction scheme 4.

Compounds of formula (I) can be converted into different compounds of formula (I) by methods known in the art. For example, a compound of formula (I) wherein R2 is an alkenyl or cycloalkenyl group can be converted to the corresponding compound of formula (I) wherein R2 is an alkyl or a cycloalkyl group. This transformation can be accomplished by hydrogenation, e.g. employing a suitable catalyst such as Pd/C (palladium on carbon).

It is known to the person skilled in the art that, if there are a number of reactive centers on a starting or intermediate compound, it may be necessary to block one or more reactive centers temporarily by protective groups in order to allow a reaction to proceed specifically at the desired reaction center.

The compounds according to the invention are isolated and purified in a manner known *per se,* e.g., by distilling off the solvent in vacuo and recrystallizing the residue obtained from a suitable solvent, or by subjecting it to one of the customary purification methods such as column chromatography employing a suitable support material.

Salts according to the invention of the compounds of formula (I) and of the stereoisomers thereof can be obtained by dissolving the free compound in a suitable solvent (for example a ketone such as acetone, methylethylketone or methylisobutylketone, an ether such as diethyl ether, tetrahydrofurane or dioxane, a chlorinated hydrocarbon such as methylene chloride or chloroform, a low molecular weight aliphatic alcohol such as methanol, ethanol or isopropanol, a low molecular weight aliphatic ester such as ethyl acetate or isopropyl acetate, an amide such as dimethylformamide, a nitril such as acetonitril, or water) which contains the desired acid, or to which the desired acid is then added. The acid can be employed in salt preparation, depending on whether a mono- or polybasic acid is concerned and depending on which salt is desired, in an equimolar quantitative ratio or a ratio differing therefrom. The salts can be obtained by filtering, reprecipitating, precipitating with a non-solvent for the salt or by evaporating the solvent. Salts obtained can be converted into the free compounds which, in turn, can be converted into salts. In this manner, pharmaceutically unacceptable salts which may be obtained, for example, as process products in the manufacturing on an industrial scale, can be converted into pharmaceutically acceptable salts by processes known to the person skilled in the art.

Depending on the particular substitution pattern, certain compounds of formula I are characterized by one or more stereogenic center(s). These stereogenic centers may independently have the absolute configuration R or the absolute configuration S (according to the rules of Cahn, Ingold and Prelog), resulting in a certain number of stereoisomers.

Accordingly, the invention further includes all mixtures of the stereoisomers mentioned above independent of the ratio of different stereoisomers to each other, including the racemates.

Pure diastereomers and pure enantiomers of the compounds of formula (I) and the salts thereof according to the invention can be obtained, e.g., by asymmetric synthesis, by using chiral starting compounds in synthesis and/or by splitting up enantiomeric and diastereomeric mixtures obtained in synthesis. Preferably, the pure diastereomeric and pure enantiomeric compounds of the invention are obtainable by asymmetric synthesis and/or by using chiral starting compounds in synthesis.

Enantiomeric and diastereomeric mixtures can be split up into the pure enantiomers and pure diastereomers by methods known to a person skilled in the art. Preferably, diastereomeric mixtures are separated by crystallization, in particular fractional crystallization, or chromatography. Enantiomeric mixtures can be separated, e.g., by forming diastereomers with a chiral auxiliary agent, resolving the diastereomers obtained and removing the chiral auxiliary agent. As chiral auxiliary agents, for example, chiral acids can be used to separate enantiomeric bases, and chiral bases can be used to separate enantiomeric acids via formation of diastereomeric salts. Furthermore, diastereomeric derivatives such as diastereomeric esters can be formed from enantiomeric mixtures of alcohols or enantiomeric mixtures of acids, respectively, using chiral acids or chiral alcohols, respectively, as chiral auxiliary agents. Additionally, diastereomeric complexes or diastereomeric clathrates may be used for separating enantiomeric mixtures. Alternatively, enantiomeric mixtures can be split up using chiral separating columns in chromatography. Another suitable method for the isolation of enantiomers is the enzymatic separation.

As will be appreciated by persons skilled in the art, the invention is not limited to the particular embodiments described herein, but covers all modifications of said embodiments that are within the spirit and scope of the invention as defined by the appended claims.

All patents, patent applications, publications, test methods and other materials cited herein are incorporated by reference in their entireties.

The following examples illustrate the invention in greater detail, without restricting it. Further compounds according to the invention, of which the preparation is not explicitly described, can be prepared in an analogous way.

The compounds which are mentioned in the examples, the salts thereof, and stereoisomers of the compounds and the salts thereof represent preferred embodiments of the invention.

### Examples

¹H and ¹³C NMR spectra were recorded at 303 K, unless otherwise indicated, on a Bruker ARX300 Spectrometer at 300.13 MHz (¹H) and 75.47 MHz (¹³C). The instrument was equipped with multinuclear inverse probe and temperature controller. The chemical shifts were expressed in ppm (parts per million) with tetramethylsilane (TMS) as an internal standard at zero ppm and the coupling constants (J) in hertz (Hz). The abbreviations s, d, t, q, quin, sxt, spt, m, dd, dt, td, tt, dq and br.s. refer to singlet, doublet, triplet, quartet, quintet, sextet, septet, multiplet, double doublet, double triplet, triple doublet, triple triplet, double quartet and broad, respectively.
LC-MS analyzes were carried out using either a ZQ2000 mass analyzer (Waters) coupled with an UPLC (Sample Organizer and UV detector) (Waters), or a ZQ2000 coupled with a 2777 Sample manager (Waters) and a 2996 Photodiode Array Detector (Waters). Microwave reactions were carried out using either a Biotage Initiator Sixty, a Biotage Initiator, a Biotage Emrys or a CEM Discover.
HPLC runs were carried out by a Shimadzu LC-8A controlled by a SCL-10A, coupled with a UV detector (SDP-6A) using SymmetryPrep^{™} C18 7µm, 19 x 300 mm columns (waters).
The purification on silica gel was performed using either Silica gel Si 60 (40-63 µm), Merck, Cat. No. 1.11567.1000, Silica gel Si 60 (40-63 µm), Merck, Cat. No. 1.09385.2500, or BIOTAGE KP-Sil Cartriges prepacked with KP-Sil^{™}, 32-63 µm, 60 A silica.

The following abbreviations were used:
°C: degrees centigrade, ml: milliliter(s), psi: pound per square inch, DMSO: dimethyl sulfoxide, DMF: N,N-dimethylformamide, EDC: N-[3-(dimethylamino)propyl]-N'-ethylcarbodiimide, HOBT: 1 H-benzotriazol-1-01, MS: mass spectrometry, ¹H NMR: ¹H nuclear magnetic resonance spectroscopy, ¹³C NMR: ¹³C nuclear magnetic resonance spectroscopy, HPLC High Performance Liquid Chromatography

### Example 1: 4-{[7-(benzyloxy)-5-methyl-6-oxo-5,6-dihydropyrido[3,2-d]pyrimidin-2-yl]amino}-3-methoxy-N-(1-methylpiperidin-4-yl)benzamide

### Step 1: 2-chloro-4-methylpyrimidin-5-amine

A mixture of 2,4-dichloro-6-methyl-5-nitropyrimidine (1.100 g) and 10% Pd/C (0.110 g) in ethyl acetate (40 ml) was hydrogenated at room temperature, at a pressure of 40 psi of hydrogen, for 24 hours. The catalyst was filtered off and the solvent was evaporated to give of a white solid. A mixture of 0.200 g of the obtained solid, triethylamine (0.236 ml) and 10% Pd/C (0.02 g) in 96% ethanol (7 ml) was hydrogenated at a pressure of 20 psi of hydrogen for 20 minutes. The catalyst was filtered off and the solvent was evaporated. The residue was purified on silica gel (eluent: ethyl acetate/petroleum ether 1/2 (v/v) to give the title compound as white solid.
¹H NMR (DMSO-d6) δ (ppm): 7.92 (s, 1 H); 5.49 (br. s., 2 H); 2.26 (s, 3 H).

MS (ESI Pos, 3.2 kV, 20 V, 400°C): m/z 144,13 (MH+).

### Step 2: N-(2-chloro-4-methylpyrimidin-5-yl)formamide

A mixture of formic acid (0.69 ml) and acetic anhydride (1.584 ml), under nitrogen atmosphere, was heated to 50°C for 1 hour. The mixture was cooled to room temperature and a solution of 2-chloro-4-methylpyrimidin-5-amine (0.498 g) in dry tetrahydrofuran (8 ml) was added. The mixture was stirred for 15 minutes, then diluted with dichloro-methane and washed with 1 M K₂CO₃. The aqueous phase was separated and extracted with dichloromethane (three times). The combined organic layers were dried over Na₂SO₄ and concentrated to give a white solid that was used in the next step without further purification.
¹H NMR (CDCl₃) δ (ppm): 9.16 (s, 1 H); 8.52 (s, 1 H); 7.30 (br. s., 1 H); 2.54 (s, 3 H).
MS (ESI Pos, 3.2 kV, 25 V, 400°C): m/z 172,13 (MH⁺).

### Step 3: 2-chloro-N,4-dimethylpyrimidin-5-amine

Borane-dimethylsulfide complex (2.76 ml) was added dropwise to a stirred solution of the product of step 2 (2.00 g) in dry tetrahydrofuran (25 ml) at room temperature, under nitrogen atmosphere. The mixture was stirred at room temperature for 2 hours, then, additional borane-dimethylsulfide complex (1.38 ml) was added and the mixture was stirred for 2 hours. Methanol was added until no further reaction was apparent; the mixture was acidified with 1 N HCl and heated to 60°C for 30 min, then cooled to room temperature, diluted with dichloro-methane and washed with 1 N K₂CO₃. The organic phase was dried over Na₂SO₄ and concentrated. The crude was purified on silica gel (eluent: ethyl acetate/petroleum ether 1/1 (v/v)) to give the title compound as white solid.
¹H NMR (DMSO-d6) δ (ppm): 7.81 (s, 1 H); 5.74 (br. s., 1 H); 2.75 (d, 3 H); 2.28 (s, 3 H). MS (ESI Pos, 3.2 kV, 25 V, 400°C): m/z 158,06 (MH+).

### Step 4: 2-chloro-7-hydroxy-5-methylpyrido[3,2-d]pyrimidin-6(5H)-one

A solution of the product of step 3 (0.754 g) in dry dimethylformamide (8 ml) was added dropwise at room temperature and under nitrogen atmosphere to a mixture of potassium *tert*-butoxide (1.61 g) and diethyloxalate (1.95 ml) in dry dimethylformamide (8 ml). The mixture was heated to 45°C for 10 min, cooled to room temperature and water was added until a red solution was obtained. 0.5N HCl was added to the solution until pH 4, obtaining a pale yellow solid that was filtered and washed with water. The title compound was obtained as pale yellow solid.
¹H NMR (DMSO-d₆) δ ppm): 11.09 (br. s., 1 H); 8.89 (s, 1 H); 7.00 (s, 1 H); 3.71 (s, 3 H). MS (ESI Pos, 3.2 kV, 25 V, 400°C): m/z 212,04 (MH⁺).

### Step 5: 7-(benzyloxy)-2-chloro-5-methylpyrido[3,2-d]pyrimidin-6(5H)-one

1,8-Diazabicyclo [5.4.0] undec-7-ene (0.74 ml) was added dropwise at room temperature and under nitrogen atmosphere to a stirred solution of the product of step 4 (0.879 g) in dry dimethylformamide (16 ml). Benzylbromide (0.64 ml) was added after 5 min, and the mixture was heated to 70°C for 1 hour. Additional 1,8-diazabicyclo [5.4.0] undec-7-ene (0.124 ml) and benzylbromide (0.149 ml) were added and the mixture was heated to 70 °C for 30 min, then cooled to room temperature, diluted with dichloromethane and washed with water. The organic phase was dried over Na₂SO₄ and concentrated. The residue was purified on silica gel (eluent: dichloromethane/ethyl acetate 10/1 (v/v)) to give the title compound as a white solid.
¹H NMR (DMSO-d6) δ (ppm): 8.94 (s, 1 H); 7.35 - 7.51 (m, 5 H); 7.32 (s, 1 H); 5.29 (s, 2 H); 3.68 (s, 3 H).
MS (ESI Pos, 3.2 kV, 25 V, 400°C): m/z 302,1 (MH+).

### Step 6: 4-amino-3-methoxy-N-(1-methylpiperidin-4-yl)benzamide

A mixture of 4-amino-3-methoxybenzoic acid (0.334 g), resin supported N-[3-(benzyloxy) propyl]-N'-cyclohexylcarbodiimide (loading 1.2 mmol/g, 2.16 g), HOBT (88%, 0.399 g), triethylamine (0.280 ml) and 1-methylpiperidin-4-amine (0.250 g) in a mixture of dichloromethane (20 ml) and dimethylformamide (4 ml) was shaken for 20 hours. The polymer was filtered off, washed with dichloromethane, acetonitrile and again dichloro-methane. The solution was concentrated and the residue was dissolved in a mixture of ethyl acetate, 1M K₂CO₃ and a saturated solution of NaCl. The organic phase was separated, dried over Na₂SO₄ and concentrated to give the title compound as orange solid. ¹H NMR (DMSO-d₆) δ ppm): 7.75 (d, 1 H); 7.25 - 7.32 (m, 2 H); 6.59 (d, 1 H); 5.17 (s, 2 H); 3.80 (s, 3 H); 3.62 - 3.77 (m, 1 H); 2.76 (d, 2 H); 2.16 (s, 3 H); 1.92 (td, 2 H); 1.66 - 1.81 (m, 2 H); 1.55 (ddd, 2 H).
MS (ESI Pos, 3.2 kV, 20 V, 400°C): m/z 264,24 (MH⁺).

### Step 7: 4-{[7-(benzyloxy)-5-methyl-6-oxo-5,6-dihydropyrido[3,2-d]pyrimidin-2-yl]amino}-3-methoxy-N-(1-methylpiperidin-4-yl)benzamide

Tris(dibenzylideneacetone)dipalladium (0) (0.058 g) was added at room temperature and under nitrogen atmosphere to a mixture consisting of the product of step 5 (0.300 g), 4-amino-3-methoxy-N-(1-methylpiperidin-4-yl)benzamide (product of step 6) (0.248 g), (+/-) 2,2'-bis-(diphenylphosphino)-1,1'-binaphthyl (0.039 g) and Cs₂CO₃ (0.843 g) in dry toluene (1.2 ml). The mixture was heated to 100°C for 20 hours. The mixture was cooled to room temperature and partitioned between water and dichloromethane. The organic phase was separated and the aqueous phase was extracted with dichloromethane. The combined organic layers were dried over Na₂SO₄ and concentrated under vacuum. The residue was purified on silica gel (eluent: dichloromethane/methanol/32% NH₄OH 10/1/0.1 (v/v)) to give a mixture of desired product and unreacted 4-amino-3-methoxy-N-(1-methylpiperidin-4-yl)benzamide. The mixture was purified on a C18 cartridge (Biotage; eluent: water/acetonitrile 95/5 to 5/95 (v/v)). The title compound precipitated from a mixture of water, methanol and acetonitrile by continuous addition of water. The title compound was obtained as yellow solid.
¹H NMR (DMSO-d6) δ (ppm): 8.80 (s, 1 H); 8.50 (d, 1 H); 8.03 - 8.11 (m, 2 H); 7.35 - 7.55 (m, 7 H); 7.17 (s, 1 H); 5.28 (s, 2 H); 3.95 (s, 3 H); 3.70 - 3.81 (m, 1 H); 3.67 (s, 3 H); 2.72 - 2.82 (m, 2 H); 2.17 (s, 3 H); 1.95 (ddd, 2 H); 1.72 - 1.84 (m, 2 H); 1.60 (qd, 2 H). ¹³C NMR (75 MHz, pyridine-d5) δ (ppm): 166.94 (s, 1 C); 155.69 (s, 1 C); 155.64 (s, 1 C); 155.03 (s, 1 C); 147.47 (s, 1 C); 145.26 (s, 1 C); 144.08 (s, 1 C); 135.86 (s, 1 C); 133.07 (s, 1 C); 128.79 (s, 2 C); 128.47 (s, 1 C); 128.14 (s, 1 C); 128.09 (s, 2 C); 124.17 (s, 1 C); 121.04 (s, 1 C); 116.78 (s, 1 C); 110.71 (s, 1 C); 109.84 (s, 1 C); 71.11 (s, 1 C); 55.50 (s, 1 C); 54.08 (s, 2 C); 46.16 (s, 1 C); 44.30 (s, 1 C); 30.83 (s, 2 C); 28.75 (s, 1 C).
MS (ESI Pos, 3.2 kV, 25 V, 400°C): m/z 529,26 (MH+).

### Example 2: 3-methoxy-4-({7-methoxy-5-methyl-6-oxo-8-[(E)-prop-1-en-1-yl]-5,6-dihydropyrido[3,2-d]pyrimidin-2-yl}amino)-N-(1-methylpiperidin-4-yl)benzamide

### Step 1: 7-(allyloxy)-2-chloro-5-methylpyrido[3,2-d]pyrimidin-6(5H)-one

Allylbromide (0.330 ml) was added to a stirred solution of 2-chloro-7-hydroxy-5-methyl-pyrido[3,2-d]pyrimidin-6(5H)-one (0.550 g) (prepared as described under example 1, step 4) and 1,8-diazabicyclo [5.4.0] undec-7-ene (0.466 ml) in dry dimethylformamide (10 ml) at room temperature, under nitrogen atmosphere. The mixture was heated to 60°C for 2 hours, then cooled to room temperature, diluted with dichloromethane and washed with water. The organic phase was separated, dried over Na₂SO₄ and concentrated. The residue was purified on silica gel (eluent: dichloromethane/ethyl acetate 20/3 (v/v)) to give the desired compound as pale yellow solid.
¹H NMR (DMSO-d6) δ (ppm): 8.94 (s, 1 H); 7.22 (s, 1 H); 6.07 (dddd, 1 H); 5.49 (dq, 1 H); 5.36 (dq, 1 H); 4.77 (dt, 2 H); 3.68 (s, 3 H).

MS (ESI Pos, 3.2 kV, 25 V, 400°C): m/z 252,15 (MH+).

### Step 2: 8-allyl-2-chloro-7-hydroxy-5-methylpyrido[3,2-d]pyrimidin-6(5H)-one

A mixture of the product of step 1 7-(allyloxy)-2-chloro-5-methylpyrido[3,2-d]pyrimidin-6(5H)-one (0.523 g) and chlorobenzene (4 ml) was heated to 130°C in a closed vessel under microwave irradiation, for 3 hours. The solvent was evaporated to give the title compound as light brown solid.
¹H NMR (DMSO-d6) δ (ppm): 10.95 (br. s., 1 H); 8.92 (s, 1 H); 5.92 (dddd, 1 H); 4.90 - 5.08 (m, 2 H); 3.73 (s, 3 H); 3.46 - 3.65 (m, 2 H)
MS (ESI Pos, 3.2 kV, 25 V, 400°C): m/z 252,15 (MH⁺).

### Step 3: 2-chloro-7-methoxy-5-methyl-8-[(1E)-prop-1-en-1-yl]pyrido[3,2-d]pyrimidin-6(5H)-one

lodomethane (0.237 ml) was added at room temperature to a solution of the product of step 2 (0.400 g) and 1,8-diazabicyclo [5.4.0] undec-7-ene (0.308 ml) in dry dimethylformamide (3 ml). The mixture was heated to 65 °C in a closed vessel under microwave irradiation for 45 minutes, cooled to room temperature and concentrated under vacuum to eliminate the excess of iodomethane. The residue was partitioned between ethyl acetate and water. The organic phase was dried over Na₂SO₄ and concentrated. The residue was purified on silica gel (eluent: dichloromethane/ethyl acetate 20/1.5 (v/v)) to give a white solid which was dissolved in dioxane (7 ml) and added to 1,8-diazabicyclo [5.4.0] undec-7-ene (0.160 ml). The mixture was heated to 50°C for 6 hours, then cooled to room temperature, diluted with ethyl acetate and washed with water. The organic phase was separated, dried over Na₂SO₄ and concentrated. The residue was purified on silica gel (eluent: dichloromethane/ethyl acetate 10/1 (v/v)) to give the title compound as white solid.
¹H NMR (DMSO-d6) δ (ppm): 9.03 (s, 1 H); 7.11 (dq, 1 H); 6.83 (dq, 1 H); 4.01 (s, 3 H); 3.67 (s, 3 H); 1.99 (dd, 3 H).
MS (ESI Pos, 3.2 kV, 25 V, 400°C): m/z 266,09 (MH+).

### Step 4: 3-methoxy-4-({7-methoxy-5-methyl-6-oxo-8-[(1E)-prop-1-en-1-yl]-5,6-dihydropyrido[3,2-d]pyrimidin-2-yl}amino)-N-(1-methylpiperidin-4-yl)benzamide

Tris(dibenzylideneacetone)dipalladium (0) (0.120 g) was added at room temperature and under nitrogen atmosphere to a stirred mixture of the product of step 3 (0.272 g), 4-amino-3-methoxy-N-(1-methylpiperidin-4-yl)benzamide (example 1, step 6) (0.270 g), Cs₂CO₃ (0.869 g) and (+/-) 2,2'-bis-(diphenylphosphino)-1,1'-binaphthyl (0.081 g) in dry toluene (8 ml).The mixture was heated to reflux for 3 hours, then additional tris(dibenzylideneacetone)di-palladium (0) (0.120 g) and (+/-) 2,2'-bis-(diphenylphosphino)-1,1'-bi-naphthyl (0.081 g) were added. The mixture was heated to reflux for 1 hour, then cooled to room temperature and partitioned between dichloromethane and water. The organic phase was separated, dried over Na₂SO₄ and concentrated. The residue was purified on silica gel (eluent: dichloromethane/methanol/32% NH₄OH 20/1.8/0.18 (v/v)) to give a product that was further purified on a C18 cartridge and finally triturated in a mixture of ethyl ether/dichloromethane 1/1 (v/v) to give the title compound as yellow solid. ¹H NMR (DMSO-d6) δ (ppm): 8.82 (s, 1 H); 8.39 (d, 1 H); 8.03 (s, 1 H); 7.76 (d, 1 H); 7.43 - 7.66 (m, 2 H); 7.05 (dq, 1 H); 6.88 (dq, 1 H); 4.01 (s, 3 H); 3.98 (s, 3 H); 3.70 - 3.85 (m, 1 H); 3.67 (s, 3 H); 2.69 - 2.88 (m, 2 H); 2.21 (s, 3 H); 2.03 (dd, 3 H); 1.95 - 2.08 (m, 2 H); 1.76 - 1.92 (m, 2 H); 1.66 (qd, 2 H).
¹³C NMR (75 MHz, pyridine-d5) δ (ppm): 166.62 (s, 1 C); 156.83 (s, 1 C); 154.97 (s, 1 C); 151.65 (s, 1 C); 147.66 (s, 1 C); 145.21 (s, 1 C); 143.78 (s, 1 C); 137.14 (s, 1 C); 132.80 (s, 1 C); 129.22 (s, 1 C); 128.62 (s, 1 C); 125.31 (s, 1 C); 120.72 (s, 1 C); 120.70 (s, 1 C); 116.77 (s, 1 C); 110.03 (s, 1 C); 59.39 (s, 1 C); 55.42 (s, 1 C); 55.05 (s, 2 C); 47.51 (s, 1
C); 46.03 (s, 1 C); 32.55 (s, 2 C); 28.36 (s, 1 C); 20.06 (s, 1 C). MS (ESI Pos, 3.2 kV, 25 V, 400°C): m/z 493,23 (MH+).

### Example 3: 3-methoxy-4-[(7-methoxy-5-methyl-6-oxo-8-phenyl-5,6-dihydropyrido[3,2-d]pyrimidin-2-yl)amino]-N-(1-methylpiperidin-4-yl)benzamide

### Step 1: 2-chloro-7-methoxy-5-methylpyrido[3,2-d]pyrimidin-6(5H)-one

lodomethane (0.304 ml) was added under a nitrogen atmosphere to a stirred solution of 2-chloro-7-hydroxy-5-methylpyrido[3,2-d]pyrimidin-6(5H)-one (0.430 g, prepared as described for example 1, step 4) and 1,8-diazabicyclo [5.4.0] undec-7-ene (0.394 ml) in dry dimethyl-formamide (5 ml). The vessel was closed and the mixture was heated to 70°C for 1 hour. Additional iodomethane (0.152 ml) and 1,8-diazabicyclo [5.4.0] undec-7-ene (0.197 mLl) were added and the mixture was heated to 70°C for 1 hour. The mixture was cooled to room temperature, diluted with ethyl acetate and washed with water. The aqueous phase was extracted with ethyl acetate. The combined organic layers were dried over Na₂SO₄ and concentrated. The residue was purified on silica gel (eluent: dichloromethane/ethyl acetate 1/1 (v/v)) to give the desired compound as a white solid.
¹H NMR (DMSO-d6) δ (ppm): 8.94 (s, 1 H); 7.22 (s, 1 H); 3.95 (s, 3 H); 3.68 (s, 3 H). MS (ESI Pos, 3.2 kV, 20 V, 400°C): 226.17 m/z (MH+).

### Step 2: 8-bromo-2-chloro-7-methoxy-5-methylpyrido[3,2-d]pyrimidin-6(5H)-one

N-Bromosuccinimide (0.240 g) was added under a nitrogen atmosphere to a stirred mixture of the product of step 1 (0.305 g) in dry dimethylformamide (7 ml) at room temperature. The mixture was stirred at 70°C for 1 hour; additional N-bromosuccinimide (0.240 g) was added and the mixture was stirred at 70°C for 3 additional hours, then cooled to room temperature, diluted with ethyl acetate and washed with 1 N K₂CO₃. The organic phase was dried over Na₂SO₄ and concentrated. The residue was purified on silica gel (eluent: dichloromethane/ ethyl acetate 2/1 (v/v)) to give the desired compound as white solid.
¹H NMR (DMSO-d6) δ (ppm): 9.11 (s, 1 H); 4.12 (s, 3 H); 3.69 (s, 3 H).
MS (ESI Pos, 3.2 kV, 25 V, 350°C): 303.84 m/z (MH+).

### Step 3: 2-chloro-7-methoxy-5-methyl-8-phenylpyrido[3,2-d]pyrimidin-6(5H)-one

Palladium(II) acetate (0.0037 g) was added under a nitrogen atmosphere to a stirred mixture of 8-bromo-2-chloro-7-methoxy-5-methylpyrido[3,2-d]pyrimidin-6(5H)-one (0.025 g), phenylboronic acid (0.010 g) and KF (0.014 g) in degassed methanol. The mixture was heated to reflux; after 1 hour additional phenylboronic acid (0.010 g), KF (0.014 g) and palladium(II) acetate (2 mg) were added; the mixture was refluxed for 4 additional hours. It was then cooled to room temperature, diluted with dichloromethane and washed with 1 N K₂CO₃. The organic phase was separated, dried over Na₂SO₄ and concentrated.

The residue was purified on silica gel (eluent: ethyl acetate/petroleum ether 2/3 (v/v)) and the desired compound was obtained as a white solid.
¹H NMR (DMSO-d6) δ (ppm): 9.10 (s, 1 H); 7.41 - 7.56 (m, 3 H); 7.33 - 7.41 (m, 2 H); 3.87 (s, 3 H); 3.76 (s, 3 H).
MS (ESI Pos, 3.2 kV, 25 V, 400°C): 302.03 m/z (MH+).

### Step 4: 3-methoxy-4-[(7-methoxy-5-methyl-6-oxo-8-phenyl-5,6-dihydropyrido[3,2-d]pyrimidin-2-yl)amino]-N-(1-methylpiperidin-4-yl)benzamide

Tris(dibenzylideneacetone)dipalladium (0) (0.033 g) was added under a nitrogen atmosphere to a stirred mixture of 2-chloro-7-methoxy-5-methyl-8-phenylpyrido[3,2-d]pyrimidin-6(5H)-one (0.085 g), 4-amino-3-methoxy-N-(1-methylpiperidin-4-yl)benz-amide (example 1, step 6) (0.096 g), (+/-) 2,2'-bis-(diphenylphosphino)-1,1'-binaphthyl (0.022 g) and Cs₂CO₃ (0.367 g) in dry toluene (2.5 ml). The mixture was stirred at reflux for 4 hours, then cooled to room temperature, diluted with dichloromethane and washed with water. The organic phase was dried over Na₂SO₄ and concentrated. The residue was purified on silica gel (eluent: dichloromethane/methanol/32% NH₄OH 20/1.8/0.18 (v/v)) to give an impure yellow solid that was triturated in a mixture of acetonitrile/water 1/9 (v/v) to give the title compound as yellow solid.
¹H NMR (DMSO-d6) δ (ppm): 8.95 (s, 1 H); 7.91 - 8.10 (m, 3 H); 7.33 - 7.63 (m, 6 H); 7.12 (dd, 1 H); 3.92 (s, 3 H); 3.86 (s, 3 H); 3.75 (s, 3 H); 3.59 - 3.81 (m, 1 H); 2.68 - 2.91 (m, 2 H); 2.18 (s, 3 H); 1.94 (ddd, 2 H); 1.76 (dd, 2 H); 1.59 (ddd, 2 H).
¹³C NMR (75 MHz, DMSO-d6) δ (ppm): 165.63 (s, 1 C); 156.94 (s, 1 C); 154.62 (s, 1 C); 152.18 (s, 1 C); 146.93 (s, 1 C); 146.85 (s, 1 C); 143.78 (s, 1 C); 134.74 (s, 1 C); 132.45 (s, 1 C); 131.90 (s, 1 C); 130.60 (s, 2 C); 128.42 (s, 1 C); 128.26 (s, 2 C); 127.60 (s, 1 C); 125.63 (s, 1 C); 120.67 (s, 1 C); 116.33 (s, 1 C); 109.42 (s, 1 C); 60.52 (s, 1 C); 56.58 (s, 1 C); 55.13 (s, 2 C); 47.06 (s, 1 C); 46.48 (s, 1 C); 32.09 (s, 2 C); 29.45 (s, 1 C). MS (ESI Pos, 3.2kV, 25V, 350°C): 529.29 m/z (MH+).

### Example 4: 3-methoxy-4-{[7-methoxy-5-methyl-6-oxo-8-(3-thienyl)-5,6-dihydro-pyrido[3,2-d]pyrimidin-2-yl]amino}-N-(1-methylpiperidin-4-yl)benzamide

This compound was prepared in a manner according to example 3 by using 3-thienyl-boronic acid instead of phenylboronic acid in step 3.
¹H NMR (DMSO-d6) δ (ppm): 8.95 (s, 1 H); 8.21 (d, 1 H); 8.06 (d, 1 H); 8.07 (s, 1 H); 7.84 (dd, 1 H); 7.72 (dd, 1 H); 7.47 (d, 1 H); 7.37 (dd, 1 H); 7.31 (dd, 1 H); 3.94 (s, 3 H); 3.90 (s, 3 H); 3.74 (s, 3 H); 3.59 - 3.84 (m, 1 H); 2.75 - 2.89 (m, 2 H); 2.20 (s, 3 H); 1.87 - 2.09 (m, 2 H); 1.70 - 1.86 (m, 2 H); 1.47 - 1.70 (m, 2 H).
MS (ESI Pos, 3.2 kV, 25 V, 350°C): 535,27 m/z (MH+).

### Example 5: 3-methoxy-4-{[7-methoxy-5-methyl-6-oxo-8-(2-thienyl)-5,6-dihydro-pyrido[3,2-d]pyrimidin-2-yl]amino}-N-(1-methylpiperidin-4-yl)benzamide

This compound was prepared in a manner according to example 3 by using 2-thienylboronic acid instead of phenylboronic acid in step 3.
¹H NMR (DMSO-d6) δ (ppm): 8.96 (s, 1 H); 8.24 (s, 1 H); 8.20 (d, 1 H); 8.10 (d, 1 H); 8.04 (dd, 1 H); 7.84 (dd, 1 H); 7.51 (d, 1 H); 7.40 (dd, 1 H); 7.25 (dd, 1 H); 4.00 (s, 3 H); 3.93 (s, 3 H); 3.72 (s, 3 H); 3.61 - 3.88 (m, 1 H); 2.84 (d, 2 H); 2.22 (s, 3 H); 1.89 - 2.15 (m, 2 H); 1.74 - 1.89 (m, 2 H); 1.62 (ddd, 2 H).
¹³C NMR (CDCl₃) δ (ppm): 166.60 (s, 1 C); 156.86 (s, 1 C); 154.69 (s, 1 C); 151.34 (s, 1 C); 147.10 (s, 1 C); 144.57 (s, 1 C); 143.80 (s, 1 C); 132.11 (s, 1 C); 132.07 (s, 1 C); 130.56 (s, 1 C); 128.59 (s, 1 C); 127.37 (s, 1 C); 127.22 (s, 1 C); 126.36 (s, 1 C); 124.93 (s, 1 C); 119.10 (s, 1 C); 116.28 (s, 1 C); 109.02 (s, 1 C); 60.11 (s, 1 C); 55.89 (s, 1 C); 54.47 (s, 2 C); 46.39 (s, 1 C); 45.92 (s, 1 C); 32.00 (s, 2 C); 28.87 (s, 1 C). MS (ESI Pos, 3.2 kV, 25 V, 350°C): 535.23 m/z (MH+).

### Example 6: 4-[(8-cyclopent-1-en-1-yl-7 -methoxy-5-methyl-6-oxo-5,6-dihydropyrido[3,2-d]pyrimidin-2-yl)amino]-3-methoxy-N-(1-methylpiperidin-4-yl)benzamide

This compound was prepared in a manner according to example 3 by using cyclopent-1-en-1-ylboronic acid instead of phenylboronic in step 3.
¹H NMR (DMSO-d6) δ (ppm): 8.89 (s, 1 H); 8.54 (d, 1 H); 8.10 (d, 1 H); 8.10 (s, 1 H); 7.52 (d, 1 H); 7.47 (dd, 1 H); 5.98 - 6.06 (m, 1 H); 3.96 (s, 3 H); 3.92 (s, 3 H); 3.71 - 3.87 (m, 1 H); 3.69 (s, 3 H); 2.56 - 2.86 (m, 6 H); 2.19 (s, 3 H); 2.09 (quin, 2 H); 1.87 - 2.03 (m, 2 H); 1.79 (dd, 2 H); 1.51 - 1.71 (m, 2 H).
MS (ESI Pos, 3.2 kV, 25 V, 350°C): 519.32 m/z (MH+).

### Example 7: 4-{[7-(cyclopropylmethoxy)-5-methyl-6-oxo-5,6-dihydropyrido[3,2-d]pyrimidin-2-yl]amino}-3-methoxy-N-(1-methylpiperidin-4-yl)benzamide

This compound was prepared in a manner according to example 1 by using (bromomethyl)cyclopropane instead of benzylbromide in step 5.
1 H NMR (DMSO-d6, 353 K) δ (ppm): 8.74 (s, 1 H); 8.48 (d, 1 H); 7.91 (s, 1 H); 7.72 (d, 1 H); 7.46 - 7.57 (m, 2 H); 6.98 (s, 1 H); 4.04 (d, 2 H); 3.98 (s, 3 H); 3.70 - 3.85 (m, 1 H); 3.68 (s, 3 H); 2.73 - 2.84 (m, 2 H); 2.20 (s, 3 H); 2.02 (ddd, 2 H); 1.76 - 1.89 (m, 2 H); 1.65 (dddd, 2 H); 1.18 - 1.43 (m, 1 H); 0.58 - 0.71 (m, 2 H); 0.34 - 0.47 (m, 2 H).
¹³C NMR (CDCl₃) δ (ppm): 166.57 (s, 1 C), 156.07 (s, 1 C), 155.49 (s, 1 C), 155.30 (s, 1 C), 147.37 (s, 1 C), 145.63 (s, 1 C), 143.30 (s, 1 C), 132.64 (s, 1 C), 127.27 (s, 1 C), 123.64 (s, 1 C), 118.92 (s, 1 C), 116.12 (s, 1 C), 109.49 (s, 1 C), 109.22 (s, 1 C), 74.58 (s, 1 C), 55.98 (s, 1 C), 54.53 (s, 2 C), 46.51 (s, 1 C), 46.22 (s, 1 C), 32.46 (s, 2 C), 29.24 (s, 1 C), 9.55 (s, 1 C), 3.72 (s, 2 C)
MS (ESI Pos, 3.2 kV, 25 V, 350°C): 493,26 m/z (MH+).

### Example 8: 4-{[7-(cyclopentylmethoxy)-5-methyl-6-oxo-5,6-dihydropyrido[3,2-d]pyrimidin-2-yl]amino}-3-methoxy-N-(1-methylpiperidin-4-yl)benzamide

### Step 1: 2-chloro-7-(cyclopentylmethoxy)-5-methylpyrido[3,2-d]pyrimidin-6(5H)-one

A mixture of 2-chloro-7-hydroxy-5-methylpyrido[3,2-d]pyrimidin-6(5H)-one (0.150 g, prepared as described under example 1, step 4), (iodomethyl)cyclopentane (0.372 g) and K₂CO₃ (0.245 g) in dimethylformamide (3 ml) was heated to 100 °C for 4 hours, under microwave irradiation. Water was added and the mixture was extracted with dichloro-methane. The crude product was purified on silica gel (eluent: dichloromethane, then dichloromethane/ethyl acetate 6/1 (v/v)) to give the desired compound.
¹H NMR (DMSO-d6) δ (ppm): 8.92 (s, 1 H); 7.20 (s, 1 H); 4.03 (d, 2 H); 3.67 (s, 3 H); 2.38 (dt, 1 H); 1.71 - 1.87 (m, 2 H); 1.45 - 1.70 (m, 4 H); 1.26 - 1.45 (m, 2 H).

### Step 2: 4-{[7-(cyclopentylmethoxy)-5-methyl-6-oxo-5,6-dihydropyrido[3,2-d]pyrimidin-2-yl]amino}-3-methoxy-N-(1-methylpiperidin-4-yl)benzamide

Tris(dibenzylideneacetone)dipalladium (0) (0.027 g) was added to a stirred mixture of 2-chloro-7-(cyclopentylmethoxy)-5-methylpyrido[3,2-d]pyrimidin-6(5H)-one (0.068 g), 4-amino-3-methoxy-N-(1-methylpiperidin-4-yl)benzamide (example 1, step 6) (0.061 g), (+/-) 2,2'-bis-(diphenylphosphino)-1,1'-binaphthyl (0.019 g) and Cs₂CO₃ (0.302 g) in dry toluene (2.5 ml). The mixture was refluxed overnight. Water was added and the mixture was extracted with dichloromethane. The crude product was purified on silica gel (eluent: dichloromethane/methanol 10/1 (v/v)) to give the desired compound.
1 H NMR (DMSO-d6) δ (ppm): 8.78 (s, 1 H); 8.53 (d, 1 H); 8.05 (s, 1 H); 8.07 (d, 1 H); 7.53 (dd, 1 H); 7.50 (d, 1 H); 7.05 (s, 1 H); 4.01 (d, 2 H); 3.96 (s, 3 H); 3.68 - 3.86 (m, 1 H); 3.66 (s, 3 H); 2.75 - 2.88 (m, 2 H); 2.32 - 2.45 (m, 1 H); 2.21 (s, 3 H); 1.94 - 2.13 (m, 2 H); 1.71 - 1.88 (m, 4 H); 1.47 - 1.71 (m, 6 H); 1.28 - 1.47 (m, 2 H).
MS (ESI Pos, 3.2 kV, 25 V, 350°C): 521.34 m/z (MH⁺).

### Example 9: 4-{[7-(cyclohexylmethoxy)-5-methyl-6-oxo-5,6-dihydropyrido[3,2-d]pyrimidin-2-yl]amino}-3-methoxy-N-(1-methylpiperidin-4-yl)benzamide

This compound was prepared in a manner according to example 8 by using (iodomethyl)cyclohexane instead of (iodomethyl)cyclopentane in step 1.
¹H NMR (CDCl₃) δ (ppm): 8.69 (d, 1 H); 8.52 (s, 1 H); 7.92 (s, 1 H); 7.47 (d, 1 H); 7.32 (dd, 1 H); 6.85 (s, 1 H); 5.97 (d, 1 H); 4.00 (s, 3 H); 3.95 - 4.13 (m, 1 H); 3.91 (d, 2 H); 3.76 (s, 3 H); 2.76 - 2.95 (m, 2 H); 2.33 (s, 3 H); 2.14 - 2.28 (m, 2 H); 1.87 - 2.13 (m, 5 H); 1.53 - 1.86 (m, 5 H); 1.20 - 1.41 (m, 3 H); 1.04 - 1.18 (m, 2 H).
¹³C NMR (CDCl₃) δ (ppm): 166.59 (s, 1 C); 156.07 (s, 1 C); 155.56 (s, 1 C); 155.50 (s, 1 C); 147.39 (s, 1 C); 145.74 (s, 1 C); 143.24 (s, 1 C); 132.69 (s, 1 C); 127.23 (s, 1 C); 123.63 (s, 1 C); 118.95 (s, 1 C); 116.12 (s, 1 C); 109.34 (s, 1 C); 109.24 (s, 1 C); 75.02 (s, 1 C); 56.00 (s, 1 C); 54.52 (s, 2 C); 46.45 (s, 1 C); 46.16 (s, 1 C); 36.90 (s, 1 C); 32.37 (s, 2 C); 29.80 (s, 2 C); 29.18 (s, 1 C); 26.34 (s, 1 C); 25.59 (s, 2 C).
MS (ESI Pos, 3.2kV, 25V, 350°C): 535,28 m/z (MH+).

### Example 10: 4-{[8-(2-furyl)-7-methoxy-5-methyl-6-oxo-5,6-dihydropyrido[3,2-d]pyrimidin-2-yl]amino}-3-methoxy-N-(1-methylpiperidin-4-yl)benzamide

This compound was prepared in a manner according to example 3 by using 2-furanylboronic acid instead of phenylboronic acid in step 3.
¹H NMR (CDCl₃) δ ppm): 8.63 (s, 1 H); 8.58 (d, 1 H); 7.99 (s, 1 H); 7.78 (dd, 1 H); 7.42 (d, 1 H); 7.18 (dd, 1 H); 7.09 (dd, 1 H); 6.72 (dd, 1 H); 5.94 (d, 1 H); 4.08 (s, 3 H); 4.00 (s, 3 H); 3.97 - 4.07 (m, 1 H); 3.80 (s, 3 H); 2.86 - 3.01 (m, 2 H); 2.38 (s, 3 H); 2.18 - 2.33 (m, 2 H); 2.00 - 2.17 (m, 2 H); 1.67 - 1.78 (m, 2 H).
MS (ESI Pos, 3.2 kV, 25 V, 350°C): 519,21 m/z (MH+).

### Example 11: 4-{[8-(3-furyl)-7-methoxy-5-methyl-6-oxo-5,6-dihydropyrido[3,2-d]pyrimidin-2-yl]amino}-3-methoxy-N-(1-methylpiperidin-4-yl)benzamide

This compound was prepared in a manner according to example 3 by using 3-furanylboronic acid instead of phenylboronic acid in step 3.
¹H NMR (CDCl₃) δ ppm): 8.65 (s, 1 H); 8.53 (d, 1 H); 8.28 (dd, 1 H); 7.93 (s, 1 H); 7.62 (dd, 1 H); 7.48 (d, 1 H); 7.20 (dd, 1 H); 7.12 (dd, 1 H); 5.94 (d, 1 H); 4.06 (s, 3 H); 4.00 - 4.02 (m, 3 H); 3.94 - 4.05 (m, 1 H); 3.79 (s, 3 H); 2.79 - 2.91 (m, 2 H); 2.33 (s, 3 H); 2.13 - 2.26 (m, 2 H); 2.01 - 2.13 (m, 2 H); 1.61 - 1.69 (m, 2 H).
MS (ESI Pos, 3.2 kV, 25 V, 350°C): 519,19 m/z (MH+).

### Example 12: 3-methoxy-4-{[5-methyl-6-oxo-7-(tetrahydrofuran-3-ylmethoxy)-5,6-dihydropyrido[3,2-d]pyrimidin-2-yl]amino}-N-(1-methylpiperidin-4-yl)benzamide

This compound was prepared in a manner according to example 8 by using 3-(iodomethyl)tetrahydrofuran instead of (iodomethyl)cyclopentane in step 1.
¹H NMR (CDCl₃) δ ppm): 8.68 (d, 1 H); 8.54 (s, 1 H); 7.93 (s, 1 H); 7.46 (d, 1 H); 7.34 (dd, 1 H); 6.89 (s, 1 H); 6.10 (d, 1 H); 4.01 (s, 3 H); 3.90 - 4.16 (m, 5 H); 3.78 - 3.86 (m, 2 H); 3.76 (s, 3 H); 2.86 - 3.12 (m, 3 H); 2.46 (s, 3 H); 2.33 - 2.45 (m, 2 H); 2.05 - 2.30 (m, 3 H); 1.70 - 1.92 (m, 3 H).
¹³C NMR (CDCl₃) δ (ppm): 166.63 (s, 1 C); 155.84 (s, 1 C); 155.50 (s, 1 C); 155.08 (s, 1 C); 147.35 (s, 1 C); 145.41 (s, 1 C); 143.45 (s, 1 C); 132.68 (s, 1 C); 127.05 (s, 1 C); 123.73 (s, 1 C); 119.17 (s, 1 C); 116.16 (s, 1 C); 109.83 (s, 1 C); 109.17 (s, 1 C); 71.33 (s, 1 C); 70.57 (s, 1 C); 67.62 (s, 1 C); 56.02 (s, 1 C); 54.46 (s, 2 C); 46.01 (s, 1 C); 45.64 (s, 1 C); 38.21 (s, 1 C); 31.68 (s, 2 C); 29.21 (s, 1 C); 28.90 (s, 1 C).
MS (ESI Pos, 3.2 kV, 25 V, 350°C): 523,27 m/z (MH+).

### Example 13: 3-methoxy-4-{[5-methyl-6-oxo-7-(tetrahydrofuran-2-ylmethoxy)-5,6-dihydropyrido[3,2-d]pyrimidin-2-yl]amino}-N-(1-methylpiperidin-4-yl)benzamide

### Step 1: 2-chloro-5-methyl-7-(tetrahydrofuran-2-ylmethoxy)pyrido[3,2-d]pyrimidin-6(5H)-one

Diethyl azodicarboxylate (0.309 g) was slowly added to a solution of 2-chloro-7-hydroxy-5-methylpyrido[3,2-d]pyrimidin-6(5H)-one (0.250 g, prepared as described for example 1, step 4), tetrahydrofuran-2-ylmethanol (0.172 ml) and triphenylphosphine (0.465 mg) in tetrahydro-furan (25 ml) at 0 °C. The reaction was stirred at 0 °C for 15 minutes and then at room temperature for 2.5 hours. The solvent was evaporated and the residue was purified on silica gel (eluent: dichloromethane to dichloromethane/ethyl acetate 1/1 (v/v)) to give the title compound.

### Step 2: 3-methoxy-4-{[5-methyl-6-oxo-7-(tetrahydrofuran-2-ylmethoxy)-5,6-dihydropyrido[3,2-d]pyrimidin-2-yl]amino}-N-(1-methylpiperidin-4-yl)benzamide

Tris(dibenzylideneacetone)dipalladium (0) (0.042 g) was added to a stirred mixture of the product of step 1 (0.107 g), 4-amino-3-methoxy-N-(1-methylpiperidin-4-yl)benzamide (example 1, step 6) (0.095 g), (+/-) 2,2'-bis-(diphenylphosphino)-1,1'-binaphthyl (0.029 g) and Cs₂CO₃ (0.473 g) in dry toluene (4 ml). The mixture was refluxed for 1.5 hours, then water was added and the mixture was extracted with dichloromethane. The crude was purified on silica gel (eluent: dichloromethane to dichloromethane/methanol/32% NH₄OH 10/1/0.2 (v/v)). The title compound precipitated from a mixture of methanol/diethyl ether by continuous addition of diethyl ether.
¹H NMR (CDCl₃) δ (ppm): 8.69 (d, 1 H); 8.54 (s, 1 H); 7.94 (s, 1 H); 7.48 (d, 1 H); 7.33 (dd, 1 H); 6.96 (s, 1 H); 5.99 (d, 1 H); 4.33 - 4.52 (m, 1 H); 4.02 (s, 3 H); 3.93 - 4.24 (m, 4 H); 3.81 - 3.91 (m, 1 H); 3.77 (s, 3 H); 2.79 - 2.99 (m, 2 H); 2.38 (s, 3 H); 2.21 - 2.33 (m, 2 H); 1.53 - 2.21 (m, 8 H).
¹³C NMR (CDCl₃) δ (ppm): 166.60 (s, 1 C); 155.88 (s, 1 C); 155.50 (s, 1 C); 155.24 (s, 1 C); 147.37 (s, 1 C); 145.50 (s, 1 C); 143.36 (s, 1 C); 132.63 (s, 1 C); 127.28 (s, 1 C); 123.79 (s, 1 C); 118.96 (s, 1 C); 116.14 (s, 1 C); 109.98 (s, 1 C); 109.23 (s, 1 C); 76.28 (s, 1 C); 71.82 (s, 1 C); 68.77 (s, 1 C); 56.00 (s, 1 C); 54.52 (s, 2 C); 46.45 (s, 1 C); 46.15 (s, 1 C); 32.35 (s, 2 C); 29.19 (s, 1 C); 28.52 (s, 1 C); 25.73 (s, 1 C).
MS (ESI Pos, 3.2kV, 25V, 350°C): 523.30 m/z (MH+).

### Example 14: 4-[(8-cyclopentyl-7-methoxy-5-methyl-6-oxo-5,6-dihydropyrido[3,2-d]pyrimidin-2-yl)amino]-3-methoxy-N-(1-methylpiperidin-4-yl)benzamide

4-[(8-cyclopent-1-en-1-yl-7-methoxy-5-methyl-6-oxo-5,6-dihydropyrido[3,2-d]pyrimidin-2-yl)amino]-3-methoxy-N-(1-methylpiperidin-4-yl)benzamide (example 6) (0.240 g) was dissolved in ethanol (84 ml) and dichloromethane (5 ml); Pd/C (0.190 g) was added and the mixture was hydrogenated at room temperature, at a pressure of 30 psi of hydrogen for 1.5 hours. Additional Pd/C (0.190 g) was added and the mixture was hydrogenated at a pressure of 35 psi for 4 hours. The crude was purified by preparative HPLC (acetonitrile/water/trifluoro-acetic acid 200/1800/1 (v/v) / acetonitrile/water/ trifluoroacetic acid 1800/200/1 (v/v), elution gradient 95/5 to 0/100 to 95/5 (v/v) to give the desired compound as a yellow powder.
¹H NMR (CDCl₃) δ ppm): 8.64 (d, 1 H); 8.61 (s, 1 H); 7.96 (s, 1 H); 7.47 (d, 1 H); 7.31 (dd, 1 H); 6.04 (d, 1 H); 4.11 (s, 3 H); 4.04 (s, 3 H); 3.98 - 4.26 (m, 2 H); 3.75 (s, 3 H); 2.96 - 3.25 (m, 2 H); 2.51 (s, 3 H); 2.28 - 2.63 (m, 2 H); 1.74 - 2.21 (m, 12 H).
MS (ESI Pos, 3.2 kV, 25 V, 350°C): 521.41 m/z (MH+).

### Example 15: 3-methoxy-4-{[7-methoxy-5-methyl-8-(2-methylcyclopent-1-en-1-yl)-6-oxo-5,6-dihydropyrido[3,2-d]pyrimidin-2-yl]amino}-N-(1-methylpiperidin-4-yl)benzamide

### Step 1: 2-chloro-7-(cyclopent-1-en-1-ylmethoxy)-5-methylpyrido[3,2-d]pyrimidin-6(5H)-one

Diethyl azodicarboxylate (0.055 ml) was added dropwise to a stirred solution of 2-chloro-7-hydroxy-5-methylpyrido[3,2-d]pyrimidin-6(5H)-one (0.050 g, prepared as described for example 1, step 4), cyclopent-1-en-1-ylmethanol (0.035 g, prepared according to J. Org. Chem. 2005, 70, 1066-1069), triphenylphosphine (0.093 g), in dry tetrahydrofuran, under nitrogen atmosphere, at 0°C. The mixture was stirred at room temperature for 20 hours, the solvent was evaporated and the crude was purified on silica gel (eluent: ethyl acetate/petroleum ether 1/2 (v/v)) to give the desired compound as off-white solid.
¹H NMR (DMSO-d6) δ (ppm): 8.93 (s, 1 H); 7.22 (s, 1 H); 5.85 (br. s., 1 H); 4.82 (s, 2 H); 3.68 (s, 3 H); 2.29 - 2.42 (m, 4 H); 1.76 - 1.98 (m, 2 H).

### Step 2: 2-chloro-7-methoxy-5-methyl-8-(2-methylcyclopent-1-en-1-yl)pyrido[3,2-d]pyrimidin-6(5H)-one

A suspension of the product of step 1 (0.350 g) in chlorobenzene (2.0 ml) was heated in a closed vessel at 120°C for 20 minutes under microwave irradiation. The solvent was evaporated, the residue was dissolved in dimethylformamide (2.5 ml); 1,8-diazabicyclo [5.4.0] undec-7-ene (0.233 ml) and iodomethane (0.179 ml) were added and the mixture was heated in a closed vessel for 45 minutes at 65°C. The mixture was cooled to room temperature and partitioned between ethyl acetate and water; the organic phase was separated, dried over Na₂SO₄ and concentrated. The residue was purified on silica gel (eluent: ethyl acetate/ petroleum ether 1/2 (v/v)) to give the desired compound as white solid.
¹H NMR (CDCl₃) δ (ppm): 8.64 (s, 1 H); 4.02 (s, 3 H); 3.77 (s, 3 H); 2.87 - 3.17 (m, 1 H); 2.43 - 2.71 (m, 3 H); 1.92 - 2.21 (m, 2 H); 1.56 - 1.61 (m, 3 H).

### Step 3: 3-methoxy-4-{[7-methoxy-5-methyl-8-(2-methylcyclopent-1-en-1-yl)-6-oxo-5,6-dihydropyrido[3,2-d]pyrimidin-2-yl]amino}-N-(1-methylpiperidin-4-yl)benzamide

Tris(dibenzylideneacetone)dipalladium (0) (0.034 g) was under a nitrogen atmosphere added to a stirred mixture of the product of step 2 (0.090 g), 4-amino-3-methoxy-N-(1-methyl-piperidin-4-yl)benzamide (example 1, step 6) (0.077 g), Cs₂CO₃ (0.384 g) and (+/-) 2,2'-bis-(diphenylphosphino)-1,1'-binaphthyl (0.023 g) in dry toluene (3 ml). The mixture was heated to reflux for 5 hours, then cooled to room temperature and partitioned between dichloro-methane and water. The aqueous phase was separated and extracted with dichloromethane. The combined organic layers were dried over Na₂SO₄ and concentrated. The residue was purified on silica gel (eluent: dichloromethane/methanol/32% NH₄OH 20/1.5/0.15 (v/v)) to give a yellow solid that was triturated in diethyl ether to give the desired compound as yellow solid.
¹H NMR (CDCl₃) δ (ppm): 8.71 (d, 1 H); 8.59 (s, 1 H); 8.00 (s, 1 H); 7.43 (d, 1 H); 7.25 (dd, 1 H); 5.94 (d, 1 H); 4.04 (s, 3 H); 4.02 (s, 3 H); 3.95 - 4.10 (m, 1 H); 3.78 (s, 3 H); 2.91 (d, 3 H); 2.54 - 2.80 (m, 3 H); 2.37 (s, 3 H); 2.05 - 2.30 (m, 6 H); 1.63 - 1.78 (m, 2 H); 1.62 (s, 3H).
¹³C NMR (CDCl₃) δ (ppm): 166.67 (s, 1 C); 157.22 (s, 1 C); 155.00 (s, 1 C); 152.63 (s, 1 C); 147.16 (s, 1 C); 144.81 (s, 1 C); 144.27 (s, 1 C); 140.17 (s, 1 C); 132.88 (s, 1 C); 132.07 (s, 1 C); 127.29 (s, 1 C); 127.04 (s, 1 C); 124.84 (s, 1 C); 119.03 (s, 1 C); 115.93 (s, 1 C); 108.93 (s, 1 C); 60.24 (s, 1 C); 55.99 (s, 1 C); 54.58 (s, 2 C); 46.55 (s, 1 C); 46.11 (s, 1 C); 38.84 (s, 1 C); 37.58 (s, 1 C); 32.31 (s, 2 C); 28.89 (s, 1 C); 23.19 (s, 1 C); 15.62 (s, 1 C).
MS (ESI Pos, 3.2kV, 25V, 350°C): 533,27 m/z (MH+).

### Example 16: 4-[(8-chloro-7-methoxy-5-methyl-6-oxo-5,6-dihydropyrido[3,2-d]pyrimidin-2-yl)amino]-3-methoxy-N-(1-methylpiperidin-4-yl)benzamide

### Step 1: 2,8-dichloro-7-methoxy-5-methylpyrido[3,2-d]pyrimidin-6(5H)-one

N-Chlorosuccinimide (0.178 g) was added under a nitrogen atmosphere to a stirred mixture of 2-chloro-7-hydroxy-5-methylpyrido[3,2-d]pyrimidin-6(5H)-one (0.300 g, prepared as described for example 1, step 4) in dry dimethylformamide (5 ml). The mixture was heated to 70°C for one hour, then cooled to room temperature, diluted with ethyl acetate and washed with 1 N K₂CO₃. The organic phase was dried over Na₂SO₄ and concentrated. The residue was purified on silica gel (eluent: dichloromethane/ethyl acetate 1/1 (v/v)) to give the title compound as white solid.

### Step 2: 4-[(8-chloro-7-methoxy-5-methyl-6-oxo-5,6-dihydropyrido[3,2-d]pyrimidin-2-yl)amino]-3-methoxy-N-(1-methylpiperidin-4-yl)benzamide

The product of step 1 (0.240 g) was dissolved under a nitrogen atmosphere in dry toluene (5 ml). Cs₂CO₃ (1.212 g), (+/-) 2,2'-bis-(diphenylphosphino)-1,1'-binaphthyl (0.087 g) and Pd₂(dba)₃ (0.128 g) were added, the resulting mixture was heated to 100°C and finally 4-amino-3-methoxy-N-(1-methylpiperidin-4-yl)benzamide (example 1, step 6) (0.317 g) was added. The mixture was heated to reflux for 8 hours; after cooling to room temperature the solvent was evaporated and the residue was dissolved in dichloro-methane and washed with water. The organic phase was dried over Na₂SO₄ and concentrated. The residue was purified by preparative HPLC (acetonitrile/water/trifluoroacetic acid 200/1800/1 (v/v) / acetonitrile/ water/trifluoroacetic acid 1800/200/1 (v/v), elution gradient 95/5 to 0/100 to 95/5 (v/v) to give the title compound as pale yellow powder.
¹H NMR (CDCl₃) δ ppm): 8.92 (d, 1 H); 8.66 (s, 1 H); 8.14 (s, 1 H); 7.49 (d, 1 H); 7.39 (dd, 1 H); 6.09 (d, 1 H); 4.26 (s, 3 H); 4.09 - 4.19 (m, 1 H); 4.04 (s, 3 H); 3.78 (s, 3 H); 3.13 (br. s., 2 H); 2.31 - 2.74 (m, 5 H); 2.06 - 2.26 (m, 2 H); 1.95 (br. s., 2 H).
MS (ESI Pos, 3.2 kV, 25 V, 350°C): 487.27 m/z (MH+).

### Example 17: 3-methoxy-4-{[7-methoxy-5-methyl-8-(2-methylcyclohex-1-en-1-yl)-6-oxo-5,6-dihydropyrido[3,2-d]pyrimidin-2-yl]amino}-N-(1-methylpiperidin-4-yl)benzamide

### Step 1: 2-chloro-7-(cyclohex-1-en-1-ylmethoxy)-5-methylpyrido[3,2-d]pyrimidin-6(5H)-one

Diethyl azodicarboxylate (0.055 ml) was added dropwise under a nitrogen atmosphere and at 0°C to a stirred solution of 2-chloro-7-hydroxy-5-methylpyrido[3,2-d]pyrimidin-6(5H)-one (0.050 g, prepared as described for example 1, step 4), cyclohex-1-en-1-ylmethanol (0.039 g, prepared in analogy to the synthesis of cyclopent-1-en-1-ylmethanol as described in J. Org. Chem. 2005, 70, 1066-1069) and triphenylphosphine (0.093 g), in dry tetrahydrofuran. The mixture was stirred at room temperature for 30 minutes, the solvent evaporated and the residue purified on silica gel (eluent: ethyl acetate/petroleum ether 1/3 (v/v)) to give the title compound as white solid contaminated with a by-product deriving from diethyl azodicarboxylate.
¹H NMR (CDCl₃) δ ppm): 8.60 (s, 1 H); 6.99 (s, 1 H); 5.81 - 5.98 (m, 1 H); 4.59 (s, 2 H); 3.78 (s, 3 H); 1.99 - 2.17 (m, 4 H); 1.57 - 1.79 (m, 4 H).

### Step 2: 2-chloro-7-methoxy-5-methyl-8-(2-methylcyclohex-1-en-1-yl)pyrido[3,2-d]pyrimidin-6(5H)-one

The product of step 1 (100 mg) was heated to 200°C for 5 minutes, under microwave irradiation, without solvent, in a closed vessel. After cooling to room temperature, the product was dissolved in dry DMF (2 ml) and 1,8-diazabicyclo [5.4.0] undec-7-ene (0.063 ml) and then iodomethane (0.049 ml) were added. The mixture was heated in a closed vessel for 45 minutes at 65°C, cooled to room temperature and partitioned between ethyl acetate and water. The organic phase was separated dried over Na₂SO₄ and concentrated. The residue was purified on silica gel (eluent: ethyl acetate/petroleum ether 1/3 (v/v)) to give the title compound as white solid.
¹H NMR (CDCl₃) δ ppm): 8.63 (s, 1 H); 4.06 (s, 3 H); 3.77 (s, 3 H); 2.30 - 2.47 (m, 1 H); 1.97 - 2.29 (m, 3 H); 1.63 - 1.93 (m, 4 H); 1.44 (s, 3 H).

### Step 3: 3-methoxy-4-{[7-methoxy-5-methyl-8-(2-methylcyclohex-1-en-1-yl)-6-oxo-5,6-dihydropyrido[3,2-d]pyrimidin-2-yl]amino}-N-(1-methylpiperidin-4-yl)benzamide

Tris(dibenzylideneacetone)dipalladium (0) (0.021 g) was added under a nitrogen atmosphere to a stirred mixture of the product of step 2 (0.057 g), 4-amino-3-methoxy-N-(1-methyl-piperidin-4-yl)benzamide (example 1, step 6) (0.047 g), Cs₂CO₃ (0.232 g) and (+/-) 2,2'-bis-(diphenylphosphino)-1,1'-binaphthyl (0.014 g) in dry toluene (2 ml). The mixture was heated to reflux for 4 hours. The mixture was cooled to room temperature and partitioned between dichloromethane and water. The aqueous phase was separated and extracted with dichloro-methane. The combined organic layers were dried over Na₂SO₄ and concentrated. The residue was purified on silica gel (eluent: dichloro-methane/methanol/32% NH₄OH 20/1.5/0.15 (v/v)) and further on C18 cartridge (water/acetonitrile 9/1 to 1/9 (v/v)) to give the desired compound as yellow solid.
¹H NMR (CDCl₃) δ ppm): 8.80 (d, 1 H); 8.59 (s, 1 H); 8.03 (s, 1 H); 7.41 (d, 1 H); 7.25 (dd, 1 H); 5.91 (d, 1 H); 4.07 (s, 3 H); 4.02 (s, 3 H); 3.98 - 4.06 (m, 1 H); 3.78 (s, 3 H); 2.76 - 2.98 (m, 2 H); 2.34 (s, 3 H); 2.02 - 2.33 (m, 8 H); 1.78 - 1.99 (m, 4 H); 1.56 - 1.74 (m, 2 H); 1.49 (s, 3 H).
MS (ESI Pos, 3.2 kV, 25 V, 350°C): 547,72 m/z (MH+).

### Example 18: 4-({7-ethoxy-5-methyl-6-oxo-8-[(1E)-prop-1-en-1-yl]-5,6-dihydropyrido[3,2-d]pyrimidin-2-yl}amino)-3-methoxy-N-(1-methylpiperidin-4-yl)benzamide

The title compound was prepared analogously to example 2 using iodoethane instead of iodomethane in step 3.
¹H NMR (CDCl₃) δ ppm): 8.67 (d, 1 H); 8.59 (s, 1 H); 7.96 (s, 1 H); 7.48 (d, 1 H); 7.31 (dd, 1 H); 7.20 (dq, 1 H); 6.99 (dq, 1 H); 5.96 (d, 1 H); 4.43 (q, 2 H); 4.03 (s, 3 H); 3.89 - 4.20 (m, 1 H); 3.74 (s, 3 H); 2.70 - 2.97 (m, 2 H); 2.34 (s, 3 H); 2.21 (td, 2 H); 2.10 (dd, 3 H); 1.98 - 2.15 (m, 2 H); 1.55 - 1.67 (m, 2 H); 1.45 (t, 3 H).
¹³C NMR (CDCl₃) δ ppm): 166.64 (s, 1 C); 157.32 (s, 1 C); 154.65 (s, 1 C); 150.78 (s, 1 C); 147.23 (s, 1 C); 144.33 (s, 2 C); 137.70 (s, 1 C); 132.46 (s, 1 C); 130.04 (s, 1 C); 127.36 (s, 1 C); 124.98 (s, 1 C); 120.26 (s, 1 C); 119.22 (s, 1 C); 115.97 (s, 1 C); 109.21 (s, 1 C); 68.33 (s, 1 C); 55.97 (s, 1 C); 54.61 (s, 2C); 46.67 (s, 1 C); 46.19 (s, 1 C); 32.31 (s, 2 C); 28.76 (s, 1 C); 20.47 (s, 1 C); 15.84 (s, 1 C).
MS (ESI Pos, 3.2 kV, 25 V, 350°C): m/z 507,40 (MH⁺).

### Example 19: 4-[(8-cyclopropyl-7-methoxy-5-methyl-6-oxo-5,6-dihydropyrido[3,2-d] pyrimidin-2-yl)amino]-3-methoxy-N-(1-methylpiperidin-4-yl)benzamide

### Step 1: 2-chloro-8-cyclopropyl-7-methoxy-5-methylpyrido[3,2-d]pyrimidin-6(5H)-one

KF (0.062 g), NaBr (0.036 g), tetrakis(triphenylphosphine)palladium(0) (0.018 g) and cyclopropylboronic acid (0.084 g) were added to a mixture of 8-bromo-2-chloro-7-methoxy-5-methylpyrido[3,2-d]pyrimidin-6(5H)-one (example 3, step 2) (0.100 g) in dry toluene (2 ml), under nitrogen atmosphere. The mixture was stirred at reflux for 18 hours and then cooled to room temperature. Toluene was evaporated and the residue was dissolved in dichloro-methane and washed with water (three times). The organic phase was dried over Na₂SO₄ and concentrated. The residue was purified on silica gel (eluent: dichloromethane/methanol 99.5/0.5 (v/v)) to give the title compound as white solid.
¹H NMR (CDCl₃) δ ppm): 8.64 (s, 1 H); 4.05 (s, 3 H); 3.73 (s, 3 H); 2.50 - 2.68 (m, 1 H); 1.48 - 1.57 (m, 2 H); 1.03 - 1.14 (m, 2 H).

### Step 2: 4-[(8-cyclopropyl-7-methoxy-5-methyl-6-oxo-5,6-dihydropyrido[3,2-d]pyrimidin-2-yl)amino]-3-methoxy-N-(1-methylpiperidin-4-yl)benzamide

4-amino-3-methoxy-N-(1-methylpiperidin-4-yl)benzamide (example 1, step 6) (0.090 g), Cs₂CO₃ (0.340 g), (+/-) 2,2'-bis-(diphenylphosphino)-1,1'-binaphthyl (0.024 g) and Tris(dibenzylideneacetone)dipalladium (0) (0.037 g) were added to a solution of the product of step 1 (0.070 g) in dry toluene (5 ml), under nitrogen atmosphere. The mixture was heated to reflux for 4 hours. After cooling, toluene was evaporated and the residue was diluted with dichloromethane and washed with water. The organic phase was dried over Na₂SO₄ and concentrated. The residue was purified on silica gel (eluent: dichloro-methane/methanol/32% NH₄OH 90/9/1 (v/v)) to give the title compound as yellow powder.
¹H NMR (CDCl₃) δ ppm): 8.71 (d, 1 H); 8.59 (s, 1 H); 7.96 (s, 1 H); 7.49 (d, 1 H); 7.29 (dd, 1 H); 5.99 (d, 1 H); 4.05 (s, 3 H); 4.03 (s, 3 H); 3.95 - 4.04 (m, 1 H); 3.73 (s, 3 H); 2.88 (d, 2 H); 2.52 (tt, 1 H); 2.34 (s, 3 H); 2.22 (td, 2 H); 2.09 (dd, 2 H); 1.55 - 1.72 (m, 2 H); 1.34 - 1.49 (m, 2 H); 1.04 - 1.24 (m, 2 H).
¹³C NMR (CDCl₃) δ ppm): 166.54 (s, 1 C); 156.97 (s, 1 C); 154.73 (s, 1 C); 152.81 (s, 1 C); 147.37 (s, 1 C); 145.67 (s, 1 C); 144.35 (s, 1 C); 136.57 (s, 1 C); 132.66 (s, 1 C); 127.17 (s, 1 C); 124.78 (s, 1 C); 118.84 (s, 1 C); 115.91 (s, 1 C); 109.27 (s, 1 C); 60.58 (s, 1 C); 56.00 (s, 1 C); 54.57 (s, 2 C); 46.56 (s, 1 C); 46.14 (s, 1 C); 32.37 (s, 2 C); 28.73 (s, 1 C); 8.49 (s, 1 C); 7.87 (s, 2 C).
MS (ESI Pos, 3.2 kV, 25 V, 350°C): 493.33 m/z (MH⁺).

### Example 20: 3-methoxy-4-({7-methoxy-5-methyl-6-oxo-8-[(1E)-prop-1-en-1-yl]-5,6-dihydropyrido[3,2-d]pyrimidin-2-yl}amino)-N-[(1-methyl-1H-imidazol-5-yl)methyl]benzamide

### Step 1: 3-methoxy-4-({7-methoxy-5-methyl-6-oxo-8-[(1E)-prop-1-en-1-yl]-5,6-dihydropyrido[3,2-d]pyrimidin-2-yl}amino)benzoic acid

Tris(dibenzylideneacetone)dipalladium (0) (0.440 g) was added to a stirred mixture of 2-chloro-7-methoxy-5-methyl-8-[(1E)-prop-1-en-1-yl]pyrido[3,2-d]pyrimidin-6(5H)-one (example 2, step 3) (1.00 g), 4-amino-3-methoxy-benzoic acid ethyl ester (0.730 g), Cs₂CO₃ (4.90 g) and (+/-) 2,2'-bis-(diphenylphosphino)-1,1'-binaphthyl (0.300 g) in dry toluene (40 ml), at room temperature, under nitrogen atmosphere. The mixture was refluxed for 5 hours, water was added and the mixture was extracted with dichloro-methane. The crude was purified on silica gel (eluent: dichloromethane, then dichloro-methane/ethyl acetate 20/1 (v/v), then dichloromethane/ethyl acetate 10/1 (v/v), then ethyl acetate, then ethyl acetate/methanol 10/1 (v/v)) to give the title compound as a yellow solid.
NaOH (30% aq. sol., 4.8 ml) was added at room temperature to a mixture of the above obtained solid (0.500 g) in methanol (75 ml) at room temperature. The mixture was heated to reflux for 1.5 hours, then methanol (75 ml) was added and the mixture was refluxed for 10 hours. 1 M LiOH (6 ml) was added and the mixture was refluxed for additional 3 hours. 2% citric acid was added to adjust the solution to pH 4-5 and then the mixture was extracted with a mixture ethyl acetate/methanol 10/1 (v/v). The aqueous phase contained a yellow solid that was filtered to give the title compound as yellow solid. The organic phase was concentrated; the residue was triturated in water to give a second batch of title compound as yellow solid.
¹H NMR (DMSO-d6) δ (ppm): 12.65 (br. s., 1 H); 8.89 (s, 1 H); 8.49 (d, 1 H); 8.27 (s, 1 H); 7.61 (dd, 1 H); 7.54 (d, 1 H); 7.06 (dq, 1 H); 6.87 (dq, 1 H); 3.98 (s, 3 H); 3.96 (s, 3 H); 3.66 (s, 3 H); 2.01 (dd, 3 H).

### Step 2: 3-methoxy-4-({7-methoxy-5-methyl-6-oxo-8-[(1E)-prop-1-en-1-yl]-5,6-dihydropyrido[3,2-d]pyrimidin-2-yl}amino)-N-[(1-methyl-1H-imidazol-5-yl)methyl]benzamide

A mixture of the product of step 1 (0.073 g), 1-(1-methyl-1H-imidazol-5-yl)methanamine (0.025 g), EDC hydrochloride (0.053 g), HOBT (88%, 0.042 g) and triethylamine (0.103 ml) in dichloromethane (12.5 ml) and dimethylformamide (2.5 ml) was stirred at room temperature overnight. 2M K₂CO₃ was added and the mixture was extracted with dichloromethane. The crude was purified on silica gel (dichloromethane to dichloro-methane/methanol 10/1 (v/v)). The title compound precipitated from a mixture of methanol/diethyl ether by continuous addition of diethyl ether.
¹H NMR (CDCl₃) δ (ppm): 8.68 (d, 1 H); 8.59 (s, 1 H); 7.98 (s, 1 H); 7.50 (d, 1 H); 7.49 (s, 1 H); 7.30 - 7.36 (m, 1 H); 7.07 (s, 1 H); 7.11 (dq, 1 H); 6.96 (dq, 1 H); 6.19 - 6.40 (m, 1 H); 4.72 (d, 2 H); 4.09 (s, 3 H); 4.02 (s, 3 H); 3.73 (s, 3 H); 3.68 (s, 3 H); 2.09 (dd, 3 H).
MS (ESI Pos, 3.2 kV, 25 V, 350°C): 490.30 m/z (MH⁺).

### Example 21: 3-methoxy-4-[(7-methoxy-5-methyl-6-oxo-5,6-dihydropyrido[3,2-d]pyrimidin-2-yl)amino]-N-(1-methylpiperidin-4-yl)benzamide

The title compound was prepared in a manner according to example 1 using iodomethane instead of benzylbromide in step 5.
¹H NMR (CDCl₃) δ (ppm): 8.71 (d, 1 H); 8.57 (s, 1 H); 7.95 (s, 1 H); 7.48 (d, 1 H); 7.33 (dd, 1 H); 6.92 (s, 1 H); 5.97 (d, 1 H); 4.03 (s, 3 H); 4.02 (s, 3 H); 3.99 - 4.15 (m, 1 H); 3.79 (s, 3 H); 2.72 - 3.06 (m, 2 H); 2.37 (s, 3 H); 2.16 - 2.32 (m, 2 H); 2.00 - 2.17 (m, 2 H); 1.67 - 1.76 (m, 2 H).
MS (ESI Pos, 3.2 kV, 25 V, 350°C): 453,40 m/z (MH⁺).

### Example 22: 4-[(8-isopropenyl-7-methoxy-5-methyl-6-oxo-5,6-dihydropyrido[3,2-d]pyrimidin-2-yl)amino]-3-methoxy-N-(1-methylpiperidin-4-yl)benzamide

### Step 1: 2-chloro-8-isopropenyl-7-methoxy-5-methylpyrido[3,2-d]pyrimidin-6(5H)-one

8-bromo-2-chloro-7-methoxy-5-methylpyrido[3,2-d]pyrimidin-6(5H)-one (0.050 g) was dissolved in 1,2-dimethoxyethane (1 ml) under nitrogen atmosphere. NaHCO₃ (0.040 g dissolved in 0.2 ml of water), tetrakis(triphenylphosphine)palladium(0) (0.019 g) and 2-isopropenyl-4,4,5,5-tetramethyl-1,3,2-dioxaborolane (0.036 ml) were added to the solution and the resulting mixture was heated to reflux for 3 hours. After cooling, the mixture was diluted with dichloromethane and washed with water. The organic phase was dried over Na₂SO₄ and concentrated. The procedure was repeated starting from 0.113 g of 8-bromo-2-chloro-7-methoxy-5-methylpyrido[3,2-d]pyrimidin-6(5H)-one. The crudes obtained were combined and purified on silica gel (dichloromethane/petroleum ether/methanol 20/75/5 (v/v)) to give the title compound as yellow solid.

### Step 2: 4-[(8-isopropenyl-7-methoxy-5-methyl-6-oxo-5,6-dihydropyrido[3,2-d]pyrimidin-2-yl)amino]-3-methoxy-N-(1-methylpiperidin-4-yl)benzamide

The product of step 1 (0.110 g) was dissolved in toluene (3 ml) and then 4-amino-3-methoxy-N-(1-methylpiperidin-4-yl)benzamide (example 1, step 6) (0.141 g), Cs₂CO₃ (0.535 g), (+/-) 2,2'-bis-(diphenylphosphino)-1,1'-binaphthyl (0.038 g) and tris(dibenzylideneacetone)dipalladium (0) (0.055 g) were added. The mixture was heated to reflux for 4 hours and then cooled to room temperature. Toluene was evaporated and the residue was diluted with dichloromethane and washed with water. The organic phase was dried over Na₂SO₄ and concentrated. The crude was purified on silica gel (dichloro-methane/methanol/32% NH₄OH 80/20/2 (v/v)) and further by preparative HPLC (acetonitrile/water/trifluoroacetic acid 200/1800/1 (v/v) / acetonitrile/water/ trifluoroacetic acid 1800/200/1 (v/v), elution gradient 95/5 to 0/100 to 95/5 (v/v) to give the title compound as yellow powder.
¹H NMR (CDCl₃) δ (ppm): 8.80 (d, 1 H); 8.61 (s, 1 H); 8.02 (s, 1 H); 7.48 (d, 1 H); 7.28 (dd, 1 H); 5.99 (d, 1 H); 5.60 (t, 1 H); 5.09 (s, 1 H); 4.11 (s, 3 H); 4.02 (s, 3 H); 3.91 - 4.08 (m, 1 H); 3.78 (s, 3 H); 2.87 - 3.06 (m, 2 H); 2.40 (s, 3 H); 2.24 - 2.36 (m, 2 H); 2.22 (s, 3 H); 2.03 - 2.18 (m, 2 H); 1.70 - 1.86 (m, 2 H).
MS (ESI Pos, 3.2kV, 25V, 350°C): 493.33 m/z (MH⁺).

### Example 23: 4-{[8-(2,5-dihydrofuran-3-ylmethyl)-7-methoxy-5-methyl-6-oxo-5,6-dihydropyrido[3,2-d]pyrimidin-2-yl]amino}-3-methoxy-N-(1-methylpiperidin-4-yl)benzamide

### Step 1: 2-chloro-5-methyl-7-[(4-methylenetetrahydrofuran-3-yl)oxy]pyrido[3,2-d]pyri-midin-6(5H)-one

Diethyl azodicarboxylate (0.055 ml) was added dropwise to a stirred mixture of 2-chloro-7-hydroxy-5-methylpyrido[3,2-d]pyrimidin-6(5H)-one (example 1, step 4) (0.050 g), 4-methylenetetrahydrofuran-3-ol (0.035 g, prepared according to Org. Lett. 2003, 3, 25, 4051-4053) and polymer-supported triphenylphosphine (loading 2.6 mmol/g, 0.272 g), in dry tetrahydro-furan (4 ml), under nitrogen atmosphere, at room temperature. After 1 hour additional diethyl azodicarboxylate (0.055 ml) was added and the mixture was stirred for 1 hour and then kept for 18 hours without stirring. The polymer was filtered off, washed with dichloromethane, acetonitrile and again dichloromethane. The solution was concentrated and the residue was purified on silica gel (eluent: ethyl acetate/petroleum ether 2/1 (v/v)) to give the title compound as white solid.
¹H NMR (CDCl₃) δ (ppm): 8.64 (s, 1 H); 7.03 (s, 1 H); 5.48 - 5.55 (m, 1 H); 5.38 (dd, 1 H); 5.27 - 5.34 (m, 1 H); 4.62 (d, 1 H); 4.39 (dt, 1 H); 4.22 (dd, 1 H); 4.07 - 4.16 (m, 1 H); 3.80 (s, 3 H).

### Step 2: 2-chloro-8-(2,5-dihydrofuran-3-ylmethyl)-7-hydroxy-5-methylpyrido[3,2-d]pyrimidin-6(5H)-one

A mixture of the product of step 1 (0.023 g) in chlorobenzene (1 ml) was heated to 120°C for 15 minutes, under microwave irradiation, in a closed vessel. The solvent was evaporated to give the title compound as white solid.
¹H NMR (CDCl₃) δ (ppm): 8.67 (s, 1 H); 7.71 (s, 1 H); 5.60 (s, 1 H); 4.60 (s, 4 H); 3.85 (s, 3 H); 3.81 (s, 2 H).

### Step 3: 2-chloro-8-(2,5-dihydrofuran-3-ylmethyl)-7-methoxy-5-methylpyrido[3,2-d]pyrimidin-6(5H)-one

lodomethane (0.076 ml) was added to a stirred mixture of the product of step 2 (0.150 g) and 1,8-diazabicyclo [5.4.0] undec-7-ene (0.099 ml), in dry dimethylformamide (3 ml), at room temperature. The mixture was heated in a closed vessel at 65°C, under microwave irradiation for 45 minutes. The mixture was cooled to room temperature and partitioned between water and ethyl acetate. The organic phase was separated and dried over Na₂SO₄. The residue was purified on silica gel (eluent: ethyl acetate/petroleum ether 2/1 (v/v)) to give the title compound as white solid.
¹H NMR (CDCl₃) δ (ppm): 8.66 (s, 1 H); 5.54 (s, 1 H); 4.59 (s, 4 H); 4.18 (s, 3 H); 3.79 (s, 2 H); 3.76 (s, 3 H).

### Step 4: 4-{[8-(2,5-dihydrofuran-3-ylmethyl)-7-methoxy-5-methyl-6-oxo-5,6-dihydro-pyrido[3,2-d]pyrimidin-2-yl]amino}-3-methoxy-N-(1-methylpiperidin-4-yl)benzamide

Tris(dibenzylideneacetone)dipalladium (0) (0.037 g) was added to a stirred mixture of the product of step 3 (0.098 g), 4-amino-3-methoxy-N-(1-methylpiperidin-4-yl)benzamide (example 1, step 6) (0.084 g), Cs₂CO₃ (0.414 g) and (+/-) 2,2'-bis-(diphenylphosphino)-1,1'-binaphthyl (0.025 g) in dry toluene (4 ml), at room temperature, under nitrogen atmosphere. The mixture was heated to reflux for 4 hours, cooled to room temperature and partitioned between water and dichloromethane. The aqueous phase was separated and extracted with dichloromethane. The combined organic layers were dried over Na₂SO₄ and concentrated. The residue was purified on silica gel (eluent: dichloro-methane/methanol/32% NH₄OH 20/3/0.3 (v/v)) and further triturated in water to the title compound as yellow solid.
¹H NMR (CDCl₃) δ (ppm): 8.58 (s, 1 H); 8.57 (d, 1 H); 7.93 (s, 1 H); 7.45 (d, 1 H); 7.31 (dd, 1 H); 6.30 (d, 1 H); 5.38 - 5.49 (m, 1 H); 4.56 - 4.63 (m, 2 H); 4.25 - 4.56 (m, 2 H); 4.09 (s, 3 H); 3.97 (s, 3 H); 3.87 - 3.96 (m, 1 H); 3.77 (s, 2 H); 3.71 (s, 3 H); 2.65 - 2.92 (m, 2 H); 2.27 (s, 3 H); 2.13 (td, 2 H); 1.90 - 2.07 (m, 2 H); 1.48 - 1.71 (m, 2 H).
¹³C NMR (CDCl₃) δ (ppm): 166.50 (s, 1 C); 156.69 (s, 1 C); 155.03 (s, 1 C); 152.96 (s, 1 C); 147.31 (s, 1 C); 144.49 (s, 1 C); 144.23 (s, 1 C); 137.00 (s, 1 C); 132.35 (s, 1 C); 132.26 (s, 1 C); 127.48 (s, 1 C); 125.00 (s, 1 C); 121.07 (s, 1 C); 118.93 (s, 1 C); 115.80 (s, 1 C); 109.39 (s, 1 C); 77.21 (s, 1 C); 75.86 (s, 1 C); 60.35 (s, 1 C); 55.99 (s, 1 C); 54.62 (s, 2 C); 46.72 (s, 1 C); 46.20 (s, 1 C); 32.34 (s, 2 C); 28.79 (s, 1 C); 22.15 (s, 1 C).
MS (ESI Pos, 3.2 kV, 25 V, 350°C): 535.49 m/z (MH⁺).

### Example 24: 4-[(8-isopropyl-7-methoxy-5-methyl-6-oxo-5,6-dihydropyrido[3,2-d]pyrimidin-2-yl)amino]-3-methoxy-N-(1-methylpiperidin-4-yl)benzamide

The title compound was prepared analogously to example 14 starting from 4-[(8-isopropenyl-7-methoxy-5-methyl-6-oxo-5,6-dihydropyrido[3,2-d]pyrimidin-2-yl)amino]-3-methoxy-N-(1-methylpiperidin-4-yl)benzamide (example 22) instead of 4-[(8-cyclopent-1-en-1-yl-7-methoxy-5-methyl-6-oxo-5,6-dihydropyrido[3,2-d]pyrimidin-2-yl)amino]-3-methoxy-N-(1-methylpiperidin-4-yl)benzamide.
¹H NMR (CDCl₃) δ (ppm): 8.66 (d, 1 H); 8.61 (s, 1 H); 7.97 (s, 1 H); 7.50 (d, 1 H); 7.32 (dd, 1 H); 5.98 (d, 1 H); 4.10 (s, 3 H); 4.04 (s, 3 H); 3.96 - 4.14 (m, 2 H); 3.75 (s, 3 H); 2.76 - 3.03 (m, 2 H); 2.37 (s, 3 H); 2.18 - 2.30 (m, 2 H); 1.99 - 2.16 (m, 2 H); 1.65 - 1.77 (m, 2 H); 1.48 (d, 6 H).
MS (ESI Pos, 3.2 kV, 25 V, 350°C): 495,35 m/z (MH⁺).

### Example 25: N-(1-ethylpiperidin-4-yl)-3-methoxy-4-({7-methoxy-5-methyl-6-oxo-8-[(1E)-prop-1-en-1-yl]-5,6-dihydropyrido[3,2-d]pyrimidin-2-yl}amino)benzamide

The title compound was prepared analogously to example 20 using 1-ethylpiperidin-4-amine instead of 1-(1-methyl-1H-imidazol-5-yl)methanamine in step 2.
¹H NMR (CDCl₃) δ (ppm): 8.68 (d, 1 H); 8.61 (s, 1 H); 7.97 (s, 1 H); 7.48 (d, 1 H); 7.31 (dd, 1 H); 7.14 (dq, 1 H); 6.99 (dq, 1 H); 6.00 (d, 1 H); 4.11 (s, 3 H); 4.06 - 4.16 (m, 1 H); 4.03 (s, 3 H); 3.75 (s, 3 H); 2.92 - 3.17 (m, 2 H); 2.56 (q, 2 H); 2.20 - 2.34 (m, 2 H); 2.11 (dd, 3 H); 1.97 - 2.20 (m, 2 H); 1.60 - 1.85 (m, 2 H); 1.19 (t, 3 H).
¹³C NMR (CDCl₃) δ (ppm): 166.63 (s, 1 C); 157.21 (s, 1 C); 154.69 (s, 1 C); 151.50 (s, 1 C); 147.27 (s, 1 C); 144.37 (s, 1 C); 144.32 (s, 1 C); 137.92 (s, 1 C); 132.53 (s, 1 C); 129.79 (s, 1 C); 127.25 (s, 1 C); 125.01 (s, 1 C); 120.08 (s, 1 C); 119.21 (s, 1 C); 116.01 (s, 1 C); 109.21 (s, 1 C); 59.72 (s, 1 C); 55.99 (s, 1 C); 52.26 (s, 1 C); 51.95 (s, 2 C); 46.84 (s, 1 C); 31.83 (s, 2 C); 28.76 (s, 1 C); 20.52 (s, 1 C); 11.79 (s, 1 C).
MS (ESI Pos, 3.2 kV, 25 V, 350°C): 507.33 m/z (MH⁺).

### Example 26: 3-methoxy-4-({7-methoxy-5-methyl-6-oxo-8-[(1E)-prop-1-en-1-yl]-5,6-dihydropyrido[3,2-d]pyrimidin-2-yl}amino)-N-[(1-methylpiperidin-4-yl)methyl]benzamide

The title compound was prepared analogously to example 20 using 1-(1-methylpiperidin-4-yl)methanamine instead of 1-(1-methyl-1H-imidazol-5-yl)methanamine in step 2.
¹H NMR (CDCl₃) δ (ppm): 8.67 (d, 1 H); 8.60 (s, 1 H); 7.97 (s, 1 H); 7.50 (d, 1 H); 7.33 (dd, 1 H); 7.13 (dq, 1 H); 6.98 (dq, 1 H); 6.33 (t, 1 H); 4.10 (s, 3 H); 4.03 (s, 3 H); 3.74 (s, 3 H); 3.41 (dd, 2 H); 2.91 - 3.11 (m, 2 H); 2.38 (s, 3 H); 2.10 (dd, 3 H); 2.04 - 2.18 (m, 2 H); 1.78 - 1.90 (m, 2 H); 1.66 - 1.84 (m, 1 H); 1.41 1.61 (m, 2 H).
MS (ESI Pos, 3.2 kV, 25 V, 350°C): 507.33 m/z (MH⁺).

### Example 27: 4-({7-(cyclopropylmethoxy)-5-methyl-6-oxo-8-[(1E)-prop-1-en-1-yl]-5,6-dihydropyrido[3,2-d]pyrimidin-2-yl}amino)-3-methoxy-N-(1-methylpiperidin-4-yl)benzamide

The title compound was prepared analogously to example 2 using (bromomethyl)cyclopropane instead of iodomethane in step 3.
¹H NMR (CDCl₃) δ (ppm): 8.68 (d, 1 H); 8.60 (s, 1 H); 7.96 (s, 1 H); 7.48 (d, 1 H); 7.31 (dd, 1 H); 7.28 (dq, 1 H); 7.04 (dq, 1 H); 5.95 (d, 1 H); 4.23 (d, 2 H); 4.03 (s, 3 H); 3.88 - 4.13 (m, 1 H); 3.74 (s, 3 H); 2.67 - 2.97 (m, 2 H); 2.33 (s, 3 H); 2.20 (td, 2 H); 2.11 (dd, 3 H); 2.02 - 2.14 (m, 2 H); 1.54 - 1.69 (m, 2 H); 1.21 - 1.39 (m, 1 H); 0.53 - 0.69 (m, 2 H); 0.29 - 0.46 (m, 2 H).
¹³C NMR (CDCl₃) δ (ppm): 166.63 (s, 1 C); 157.42 (s, 1 C); 154.71 (s, 1 C); 150.89 (s, 1 C); 147.39 (s, 1 C); 144.51 (s, 1 C); 144.27 (s, 1 C); 137.91 (s, 1 C); 132.62 (s, 1 C); 130.11 (s, 1 C); 127.31 (s, 1 C); 125.03 (s, 1 C); 120.32 (s, 1 C); 118.89 (s, 1 C); 116.06 (s, 1 C); 109.22 (s, 1 C); 77.19 (s, 1 C); 56.00 (s, 1 C); 54.58 (s, 2 C); 46.64 (s, 1 C); 46.23 (s, 1 C); 32.47 (s, 2 C); 28.78 (s, 1 C); 20.54 (s, 1 C); 11.29 (s, 1 C); 3.39 (s, 2 C).
MS (ESI Pos, 3.2 kV, 25 V, 350°C): m/z 533.47 (MH⁺).

### Example 28: 3-methoxy-4-({7-methoxy-5-methyl-6-oxo-8-[(1E)-prop-1-en-1-yl]-5,6-dihydropyrido[3,2-d]pyrimidin-2-yl}amino)-N-pyridin-3-ylbenzamide

Bromo-tris-pyrrolidino phosphoniumhexafluorophosphate (PyBrOP) (0.120 g), N,N-dimethylpyridin-4-amine (0.096 g) and pyridin-3-amine (0.026 g) were added to a mixture of 3-methoxy-4-({7-methoxy-5-methyl-6-oxo-8-[(1E)-prop-1-en-1-yl]-5,6-dihydro-pyrido[3,2-d]pyrimidin-2-yl}amino)benzoic acid (example 20, step 1) (0.103 g) in dry dichloromethane, at 0°C, under nitrogen atmosphere. The mixture was stirred at room temperature for 1 hour, then diluted with dichloromethane and washed with water and a saturated solution of NaHCO3 in water. The organic phase was dried over Na₂SO₄ and concentrated. The crude was purified on silica gel (eluent: dichloromethane/methanol/32% NH₄OH 92/8/1 (v/v)). The title compound precipitated from a mixture of methanol/diethyl ether by continuous addition of diethyl ether.
¹H NMR (CDCl₃) δ (ppm): 9.02 (s, 1 H); 8.83 (d, 1 H); 8.72 (d, 1 H); 8.59 (s, 1 H); 8.27 - 8.39 (m, 2 H); 7.99 (s, 1 H); 7.53 - 7.67 (m, 2 H); 7.28 - 7.35 (m, 1 H); 7.02 - 7.19 (m, 1 H); 6.86 - 7.02 (m, 1 H); 4.06 (s, 3 H); 4.02 (s, 3 H); 3.71 (s, 3 H); 2.09 (d, 3 H).
MS (ESI Pos, 3.2 kV, 25 V, 350°C): 473.35 m/z (MH⁺).

### Example 29: N-[(1S)-2-(1H-imidazol-4-yl)-1-methylethyl]-3-methoxy-4-({7-methoxy-5-methyl-6-oxo-8-[(1E)-prop-1-en-1-yl]-5,6-dihydropyrido[3,2-d]pyrimidin-2-yl}amino)benzamide

The title compound was prepared analogously to example 20 using (2S)-1-(1H-imidazol-4-yl)propan-2-amine instead of 1-(1-methyl-1H-imidazol-5-yl)methanamine in step 2. ¹H NMR (CDCl₃) δ (ppm): 8.67 (d, 1 H); 8.59 (s, 1 H); 7.95 (s, 1 H); 7.62 (s, 1 H); 7.54 (d, 1 H); 7.46 (d, 1 H); 7.14 (dq, 1 H); 6.97 (dd, 1 H); 6.91 (s, 1 H); 4.37 - 4.59 (m, 1 H); 4.10 (s, 3 H); 4.03 (s, 3 H); 3.74 (s, 3 H); 3.02 (dd, 1 H); 2.85 (dd, 1 H); 2.12 (dd, 3 H); 1.27 (d, 3 H).
MS (ESI Pos, 3.2 kV, 25 V, 350°C): 504.37 m/z (MH⁺).

### Example 30: 4-{[7-ethoxy-5-methyl-6-oxo-8-(3-thienyl)-5,6-dihydropyrido[3,2-d]pyri-midin-2-yl]amino}-3-methoxy-N-(1-methylpiperidin-4-yl)benzamide

The title compound was prepared analogously to example 3 using iodoethane instead of iodomethane in step 1 and 3-thienylboronic acid instead of phenylboronic acid in step 3. ¹H NMR (CDCl₃) δ (ppm): 8.64 (s, 1 H); 8.39 (d, 1 H); 7.97 (s, 1 H); 7.79 (dd, 1 H); 7.51 (dd, 1 H); 7.48 (dd, 1 H); 7.42 (d, 1 H); 7.09 (dd, 1 H); 5.91 (d, 1 H); 4.27 (q, 2 H); 3.93 - 4.11 (m, 1 H); 3.99 (s, 3 H); 3.81 (s, 3 H); 2.86 (dt, 2 H); 2.33 (s, 3 H); 2.19 (td, 2 H); 2.03 - 2.13 (m, 2 H); 1.63 (ddd, 2 H); 1.28 (t, 3 H).
¹³C NMR (75 MHz, CDCl₃) δ (ppm): 166.64 (s, 1 C); 157.53 (s, 1 C); 154.89 (s, 1 C); 151.72 (s, 1 C); 147.15 (s, 1 C); 144.59 (s, 1 C); 144.51 (s, 1 C); 132.35 (s, 1 C); 130.64 (s, 1 C); 130.26 (s, 1 C); 130.23 (s, 1 C); 127.82 (s, 1 C); 127.23 (s, 1 C); 125.14 (s, 1 C); 123.83 (s, 1 C); 118.88 (s, 1 C); 116.28 (s, 1 C); 108.99 (s, 1 C); 68.95 (s, 1 C); 55.96 (s, 1 C); 54.58 (s, 2 C); 46.62 (s, 1 C); 46.24 (s, 1 C); 32.46 (s, 2 C); 29.00 (s, 1 C); 15.60 (s, 1 C).
MS (ESI Pos, 3.2 kV, 25 V, 350°C): 549.36 m/z (MH⁺).

### Example 31: 4-{[7-isopropoxy-5-methyl-6-oxo-8-(3-thienyl)-5,6-dihydropyrido[3,2-d]pyrimidin-2-yl]amino}-3-methoxy-N-(1-methylpiperidin-4-yl)benzamide

### Step 1: 2-chloro-7-isopropoxy-5-methyl-8-(3-thienyl)pyrido[3,2-d]pyrimidin-6(5H)-one

8-bromo-2-chloro-7-isopropoxy-5-methylpyrido[3,2-d]pyrimidin-6(5H)-one (prepared analogously to the product of example 3, step 2 using 2-iodo-propane instead of iodomethane in step 1) (0.265 g), 3-thienylboronic acid (0.307 g), KF (0.418 g) were mixed in degassed isopropanol (7 ml) and the mixture was refluxed under nitrogen atmosphere. Palladium(II) acetate was added in 0.08 mmol portion every 0.5 hours. After 5 additions the mixture was diluted with ethyl acetate and washed with 10% K₂CO₃. The organic layer was separated, dried over Na₂SO₄ and concentrated. The residue was purified on silica gel (eluent: ethyl acetate/toluene 1/9 (v/v)) to give the title compound.
¹H NMR (DMSO-d₆) δ (ppm): 9.08 (s, 1 H); 7.82 (dd, 1 H); 7.63 (dd, 1 H); 7.37 (dd, 1 H); 4.93 (spt, 1 H); 3.74 (s, 3 H); 1.08 (d, 6 H).

### Step 2: 4-{[7-isopropoxy-5-methyl-6-oxo-8-(3-thienyl)-5,6-dihydropyrido[3,2-d]pyrimidin-2-yl]amino}-3-methoxy-N-(1-methylpiperidin-4-yl)benzamide

The product of step 1 (0.130 g), 4-amino-3-methoxy-N-(1-methylpiperidin-4-yl)benzamide (example 1, step 6) (0.112 g), Cs₂CO₃ (0.508 g), (+/-) 2,2'-bis-(diphenylphosphino)-1,1'-binaphthyl (0.029 g) and Tris(dibenzylideneacetone)dipalladium (0) (0.043 g) were mixed in degassed toluene (5 ml) and the mixture was stirred at reflux for 3.5 hours. The mixture was diluted with water and partitioned with dichloromethane. The organic layer was dried over Na₂SO₄ and concentrated. The residue was purified on silica gel (eluent: dichloromethane/methanol/32% NH₄OH, elution gradient 100/0/0 to 30/1/0.15 (v/v)) to give the title compound.
¹H NMR (CDCl₃) δ (ppm): 8.64 (s, 1 H); 8.40 (d, 1 H); 7.97 (s, 1 H); 7.78 (dd, 1 H); 7.45 - 7.53 (m, 2 H); 7.42 (d, 1 H); 7.08 (dd, 1 H); 5.90 (d, 1 H); 4.95 (spt, 1 H); 3.99 (s, 3 H); 3.89 - 4.14 (m, 1 H); 3.81 (s, 3 H); 2.86 (d, 2 H); 2.33 (s, 3 H); 2.19 (td, 2 H); 2.09 (dd, 2 H); 1.53 - 1.72 (m, 2 H); 1.17 (d, 6 H).
¹³C NMR (75 MHz, CDCl₃) δ (ppm): 166.64 (s, 1 C); 157.79 (s, 1 C); 154.89 (s, 1 C); 150.97 (s, 1 C); 147.15 (s, 1 C); 144.54 (s, 1 C); 132.40 (s, 1 C); 131.15 (s, 1 C); 130.97 (s, 1 C); 130.45 (s, 2 C); 127.88 (s, 1 C); 127.21 (s, 1 C); 125.16 (s, 1 C); 123.52 (s, 1 C); 118.87 (s, 1 C); 116.29 (s, 1 C); 108.99 (s, 1 C); 75.72 (s, 1 C); 55.95 (s, 1 C); 54.59 (s, 2 C); 46.62 (s, 1 C); 46.26 (s, 1 C); 32.47 (s, 2 C); 29.00 (s, 1 C); 22.59 (s, 2 C).
MS (ESI Pos, 3.2 kV, 25 V, 350°C): 563.44 m/z (MH⁺).

### Example 32: 4-{[7-(cyclopropylmethoxy)-5-methyl-6-oxo-8-(3-thienyl)-5,6-dihydro-pyrido[3,2-d]pyrimidin-2-yl]amino}-3-methoxy-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methyl-pyrido[3,2-d]pyrimidin-6(5H)-one.
¹H NMR (CDCl₃) δ (ppm): 8.64 (s, 1 H); 8.40 (d, 1 H); 7.97 (s, 1 H); 7.82 (dd, 1 H); 7.53 (dd, 1 H); 7.49 (dd, 1 H); 7.42 (d, 1 H); 7.09 (dd, 1 H); 5.92 (d, 1 H); 4.08 (d, 2 H); 3.99 (s, 3 H); 3.93 - 4.05 (m, 1 H); 3.80 (s, 3 H); 2.85 (d, 2 H); 2.33 (s, 3 H); 2.20 (td, 2 H); 2.09 (dd, 2 H); 1.51 - 1.74 (m, 2 H); 0.93 - 1.24 (m, 1 H); 0.33 - 0.62 (m, 2 H); 0.05 - 0.30 (m, 2 H).
¹³C NMR (75 MHz, CDCl₃) δ (ppm): 166.65 (s, 1 C); 157.56 (s, 1 C); 154.90 (s, 1 C); 151.64 (s, 1 C); 147.16 (s, 1 C); 144.59 (s, 1 C); 144.50 (s, 1 C); 132.36 (s, 1 C); 130.68 (s, 1 C); 130.41 (s, 1 C); 127.96 (s, 1 C); 127.25 (s, 1 C); 125.19 (s, 1 C); 123.65 (s, 1 C); 118.89 (s, 1 C); 116.30 (s, 1 C); 109.00 (s, 1 C); 77.72 (s, 2 C); 55.96 (s, 1 C); 54.59 (s, 2 C); 46.62 (s, 1 C); 46.23 (s, 1 C); 32.44 (s, 2 C); 28.97 (s, 1 C); 10.98 (s, 1 C); 3.16 (s, 2 C).
MS (ESI Pos, 3.2kV, 25V, 350°C): 575.36 m/z (MH⁺).

### Example 33: 3-methoxy-4-{[7-methoxy-5-methyl-8-(4-methyl-3-thienyl)-6-oxo-5,6-dihydropyrido[3,2-d]pyrimidin-2-yl]amino}-N-(1-methylpiperidin-4-yl)benzamide

The title compound was prepared analogously to example 3 using 4-methylthiophen-3-ylboronic acid instead of phenylboronic acid in step 3.
¹H NMR (CDCl₃) δ (ppm): 8.62 (s, 1 H); 8.09 (d, 1 H); 7.97 (s, 1 H); 7.39 (d, 1 H); 7.32 (d, 1 H); 7.21 (dq, 1 H); 7.01 (dd, 1 H); 5.88 (d, 1 H); 3.97 (s, 3 H); 3.95 - 4.14 (m, 1 H); 3.91 (s, 3 H); 3.83 (s, 3 H); 2.67 - 3.02 (m, 2 H); 2.34 (s, 3 H); 2.20 (td, 2 H); 2.10 (s, 3 H); 1.98 - 2.12 (m, 2 H); 1.48 - 1.80 (m, 2 H).
¹³C NMR (CDCl₃) δ (ppm): 166.65 (s, 1 C); 157.18 (s, 1 C); 155.01 (s, 1 C); 153.36 (s, 1 C); 147.05 (s, 1 C); 145.09 (s, 1 C); 144.35 (s, 1 C); 137.90 (s, 1 C); 132.67 (s, 1 C); 132.48 (s, 1 C); 128.64 (s, 1 C); 127.05 (s, 1 C); 125.38 (s, 1 C); 124.51 (s, 1 C); 121.18 (s, 1 C); 118.94 (s, 1 C); 115.99 (s, 1 C); 108.83 (s, 1 C); 60.64 (s, 1 C); 55.93 (s, 1 C); 54.59 (s, 2 C); 46.61 (s, 1 C); 46.24 (s, 1 C); 32.44 (s, 2 C); 29.08 (s, 1 C); 14.97 (s, 1 C).
MS (ESI Pos, 3.2 kV, 25 V, 350°C): 549.36 m/z (MH⁺).

### Example 34: 3-methoxy-4-[(7-methoxy-5-methyl-6-oxo-8-pyrimidin-5-yl-5,6-dihydro-pyrido[3,2-d]pyrimidin-2-yl)amino]-N-(1-methylpiperidin-4-yl)benzamide

### Step 1: 2-chloro-7-methoxy-5-methyl-8-pyrimidin-5-ylpyrido[3,2-d]pyrimidin-6(5H)-one

A mixture of 8-bromo-2-chloro-7-methoxy-5-methylpyrido[3,2-d]pyrimidin-6(5H)-one (0.150 g), pyrimidin-5-ylboronic acid (0.096 g), KF (0.043 g) and tetrakis(triphenylphosphine)palladium(0) (0.057 g) in acetonitrile (3 ml) and water (0.3 ml) in a closed vessel was heated to 135°C for 40 minutes, under microwave irradiation. After cooling to room temperature, the mixture was partitioned between dichloromethane and 1 N K₂CO₃. The organic phase was dried over Na₂SO₄ and concentrated. The residue was purified on silica gel (eluent: dichloro-methane/methanol 20/0.5 (v/v)) and further triturated in ethyl acetate to give the title compound as yellow solid.
¹H NMR (DMSO-d₆) δ (ppm): 9.27 (s, 1 H); 9.15 (s, 1 H); 8.91 (s, 2 H); 4.03 (s, 3 H); 3.78 (s, 3 H).

### Step 2: 3-methoxy-4-[(7-methoxy-5-methyl-6-oxo-8-pyrimidin-5-yl-5,6-dihydropyrido[3,2-d]pyrimidin-2-yl)amino]-N-(1-methylpiperidin-4-yl)benzamide

Tris(dibenzylideneacetone)dipalladium (0) (0.013 g) was added to a stirred mixture of the product of step 1 (0.033 g), 4-amino-3-methoxy-N-(1-methylpiperidin-4-yl)benzamide (example 1, step 6) (0.028 g), (+/-) 2,2'-bis-(diphenylphosphino)-1,1'-binaphthyl (0.009 g) and Cs₂CO₃ (0.141 g) in dry toluene (1.5 ml), at room temperature, under nitrogen atmosphere. The mixture was heated to reflux for 5 hours, then cooled to room temperature and partitioned between dichloromethane and water. The aqueous phase was separated and extracted with dichloromethane. The combined organic layers were dried over Na₂SO₄ and concentrated. The residue was purified on silica gel (eluent: dichloro-methane/methanol/32% NH₄OH 20/2/0.2 (v/v)) and further through a C18 cartridge (water to acetonitrile) to give the title compound as yellow solid.
¹H NMR (CDCl₃) δ (ppm): 9.36 (s, 1 H); 8.97 (s, 2 H); 8.69 (s, 1 H); 8.09 (d, 1 H); 7.96 (s, 1 H); 7.47 (d, 1 H); 7.07 (dd, 1 H); 6.02 (d, 1 H); 4.13 (s, 3 H); 3.99 (s, 3 H); 3.92 - 4.10 (m, 1 H); 3.84 (s, 3 H); 2.82 - 2.99 (m, 2 H); 2.36 (s, 3 H); 2.22 (td, 2 H); 1.96 - 2.15 (m, 2 H); 1.54 - 1.77 (m, 2 H).
MS (ESI Pos, 3.2 kV, 25 V, 350°C): 531.38 m/z (MH⁺).

### Example 35: 3-methoxy-4-{[7-methoxy-5-methyl-8-(1-methyl-1H-pyrazol-4-yl)-6-oxo-5,6-dihydropyrido[3,2-d]pyrimidin-2-yl]amino}-N-(1-methylpiperidin-4-yl)benzamide

The title compound was prepared analogously to example 22 using 1-methyl-4-(4,4,5,5-tetramethyl-1,3,2-dioxaborolan-2-yl)-1H-pyrazole instead of 2-isopropenyl-4,4,5,5-tetramethyl-1,3,2-dioxaborolane in step 1.
¹H NMR (CDCl₃) δ (ppm): 8.66 (s, 1 H); 8.50 (d, 1 H); 8.38 (d, 1 H); 8.26 (s, 1 H); 7.90 (s, 1 H); 7.50 (d, 1 H); 7.25 (dd, 1 H); 6.04 (d, 1 H); 4.08 (s, 3 H); 4.04 (s, 3 H); 4.02 (s, 3 H); 3.91 - 4.05 (m, 1 H); 3.79 (s, 3 H); 2.67 - 3.00 (m, 2 H); 2.33 (s, 3 H); 2.19 (td, 2 H); 1.93 - 2.13 (m, 2 H); 1.54 - 1.72 (m, 2 H).
MS (ESI Pos, 3.2 kV, 25 V, 350°C): 533,40 m/z (MH⁺).

### Example 36: 4-{[7-sec-butoxy-5-methyl-6-oxo-8-(3-thienyl)-5,6-dihydropyrido[3,2-d]pyrimidin-2-yl]amino}-3-methoxy-N-(1-methylpiperidin-4-yl)benzamide

The title compound was prepared analogously to example 31 starting from 8-bromo-7-sec-butoxy-2-chloro-5-methylpyrido[3,2-d]pyrimidin-6(5H)-one (prepared analogously to the product of example 3, step 2 using 2-iodo-butane instead of iodomethane in step 1) instead of 8-bromo-2-chloro-7-isopropoxy-5-methylpyrido[3,2-d]pyrimidin-6(5H)-one.
¹H NMR (CDCl₃) δ (ppm): 8.64 (s, 1 H); 8.40 (d, 1 H); 7.97 (s, 1 H); 7.75 (dd, 1 H); 7.45 - 7.54 (m, 2 H); 7.42 (d, 1 H); 7.09 (dd, 1 H); 5.96 (d, 1 H); 4.71 - 4.94 (m, 1 H); 3.99 (s, 3 H); 3.97 - 4.12 (m, 1 H); 3.81 (s, 3 H); 2.88 - 3.02 (m, 2 H); 2.40 (s, 3 H); 2.20 - 2.37 (m, 2 H); 2.03 - 2.20 (m, 2 H); 1.69 - 1.82 (m, 2 H); 1.57 - 1.68 (m, 1 H); 1.38 - 1.54 (m, 1 H); 1.11 (d, 3 H); 0.68 - 0.99 (m, 3 H).
MS (ESI Pos, 3.2 kV, 25 V, 350°C): 577.45 m/z (MH⁺).

### Example 37: 4-[(8-cyclopropyl-7-ethoxy-5-methyl-6-oxo-5,6-dihydropyrido[3,2-d]pyrimidin-2-yl)amino]-3-methoxy-N-(1-methylpiperidin-4-yl)benzamide

The title compound was prepared analogously to example 19 starting from 8-bromo-2-chloro-7-ethoxy-5-methylpyrido[3,2-d]pyrimidin-6(5H)-one (prepared analogously to the product of example 3, step 2 using iodoethane instead of iodomethane in step 1) instead of 8-bromo-2-chloro-7-methoxy-5-methylpyrido[3,2-d]pyrimidin-6(5H)-one.
¹H NMR (CDCl₃) δ (ppm): 8.71 (d, 1 H); 8.58 (s, 1 H); 7.95 (s, 1 H); 7.49 (d, 1 H); 7.29 (dd, 1 H); 5.96 (d, 1 H); 4.34 (q, 2 H); 4.02 (s, 3 H); 3.87 - 4.10 (m, 3 H); 3.74 (s, 3 H); 2.43 - 2.57 (m, 1 H); 2.33 (s, 3 H); 2.19 (td, 2 H); 2.08 (dd, 2 H); 1.53 - 1.70 (m, 2 H); 1.46 (t, 3 H); 1.38 - 1.48 (m, 2 H); 1.01 - 1.25 (m, 2 H).
¹³C NMR (CDCl₃) δ (ppm): 166.57 (s, 1 C); 157.10 (s, 1 C); 154.67 (s, 1 C); 152.17 (s, 1 C); 147.33 (s, 1 C); 145.72 (s, 1 C); 144.29 (s, 1 C); 136.50 (s, 1 C); 132.65 (s, 1 C); 127.16 (s, 1 C); 124.75 (s, 1 C); 118.82 (s, 1 C); 115.88 (s, 1 C); 109.25 (s, 1 C); 68.80 (s, 1 C); 55.99 (s, 1 C); 54.60 (s, 2 C); 46.66 (s, 1 C); 46.24 (s, 1 C); 32.49 (s, 2 C); 28.75 (s, 1 C); 15.45 (s, 1 C); 8.68 (s, 1 C); 7.79 (s, 2 C).
MS (ESI Pos, 3.2 kV, 25 V, 350°C): 507.32 m/z (MH⁺).

### Example 38: 4-{[8-cyclopropyl-7-(cyclopropylmethoxy)-5-methyl-6-oxo-5,6-dihydro-pyrido[3,2-d]pyrimidin-2-yl]amino}-3-methoxy-N-(1-methylpiperidin-4-yl)benzamide

The title compound was prepared analogously to example 19 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-methoxy-5-methyl-pyrido[3,2-d]pyrimidin-6(5H)-one.
¹H NMR (CDCl₃) δ (ppm): 8.72 (d, 1 H); 8.59 (s, 1 H); 7.95 (s, 1 H); 7.49 (d, 1 H); 7.30 (dd, 1 H); 5.99 (d, 1 H); 4.15 (d, 2 H); 4.03 (s, 3 H); 3.98 - 4.11 (m, 1 H); 3.73 (s, 3 H); 2.91 (d, 2 H); 2.53 (tt, 1 H); 2.37 (s, 3 H); 2.17 - 2.31 (m, 2 H); 2.01 - 2.17 (m, 2 H); 1.61 - 1.72 (m, 2 H); 1.47 - 1.57 (m, 2 H); 1.26 - 1.41 (m, 1 H); 1.06 - 1.23 (m, 2 H); 0.54 - 0.75 (m, 2 H); 0.29 - 0.47 (m, 2 H).
¹³C NMR (CDCl₃) δ (ppm): 166.57 (s, 1 C); 157.05 (s, 1 C); 154.66 (s, 1 C); 152.15 (s, 1 C); 147.33 (s, 1 C); 145.73 (s, 1 C); 144.27 (s, 1 C); 136.41 (s, 1 C); 132.70 (s, 1 C); 127.05 (s, 1 C); 124.73 (s, 1 C); 118.90 (s, 1 C); 115.91 (s, 1 C); 109.22 (s, 1 C); 77.75 (s, 1 C); 56.00 (s, 1 C); 54.56 (s, 2 C); 46.45 (s, 1 C); 46.03 (s, 1 C); 32.22 (s, 2 C); 28.73 (s, 1 C); 10.94 (s, 1 C); 8.84 (s, 1 C); 7.77 (s, 2 C); 3.27 (s, 2 C).
MS (ESI Pos, 3.2 kV, 25 V, 350°C): 533.39 m/z (MH⁺).

### Example 39: 4-{[7-(cyclopropylmethoxy)-8-(3-furyl)-5-methyl-6-oxo-5,6-dihydro-pyrido[3,2-d]pyrimidin-2-yl]amino}-3-methoxy-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methyl-pyrido[3,2-d]pyrimidin-6(5H)-one and using furan-3-ylboronic acid instead of 3-thienylboronic acid in step 1.
¹H NMR (CDCl₃) δ (ppm): 8.65 (s, 1 H); 8.56 (d, 1 H); 8.34 (dd, 1 H); 7.94 (s, 1 H); 7.62 (t, 1 H); 7.48 (d, 1 H); 7.20 - 7.24 (m, 1 H); 7.20 (dd, 1 H); 5.95 (d, 1 H); 4.18 (d, 2 H); 4.02 (s, 3 H); 3.88 - 4.10 (m, 1 H); 3.78 (s, 3 H); 2.86 (d, 2 H); 2.33 (s, 3 H); 2.19 (td, 2 H); 1.96 - 2.13 (m, 2 H); 1.51 - 1.70 (m, 2 H); 1.11 - 1.40 (m, 1 H); 0.46 - 0.67 (m, 2 H); 0.20 - 0.37 (m, 2 H).
¹³C NMR (CDCl₃) δ (ppm): 166.59 (s, 1 C); 157.29 (s, 1 C); 154.87 (s, 1 C); 151.10 (s, 1 C); 147.33 (s, 1 C); 145.64 (s, 1 C); 144.56 (s, 1 C); 144.18 (s, 1 C); 141.57 (s, 1 C); 132.30 (s, 1 C); 127.43 (s, 1 C); 126.85 (s, 1 C); 125.19 (s, 1 C); 118.82 (s, 1 C); 116.23 (s, 1 C); 115.20 (s, 1 C); 112.88 (s, 1 C); 109.26 (s, 1 C); 77.70 (s, 1 C); 55.98 (s, 1 C); 54.58 (s, 2 C); 46.65 (s, 1 C); 46.21 (s, 1 C); 32.41 (s, 2 C); 28.92 (s, 1 C); 11.14 (s, 1 C); 3.36 (s, 2 C).
MS (ESI Pos, 3.2 kV, 25 V, 350°C): 559.32 m/z (MH⁺).

### Example 40: 4-({7-sec-butoxy-5-methyl-6-oxo-8-[(1E)-prop-1-en-1-yl]-5,6-dihydro-pyrido[3,2-d]pyrimidin-2-yl}amino)-3-methoxy-N-(1-methylpiperidin-4-yl)benzamide

The title compound was prepared analogously to example 2 using 2-iodobutane instead of iodomethane in step 3.
¹H NMR (CDCl₃) δ (ppm): 8.69 (d, 1 H); 8.59 (s, 1 H); 7.96 (s, 1 H); 7.47 (d, 1 H); 7.32 (dd, 1 H); 7.21 (dq, 1 H); 6.98 (dq, 1 H); 6.03 (d, 1 H); 5.08 (sxt, 1 H); 4.03 (s, 3 H); 3.94 - 4.22 (m, 1 H); 3.74 (s, 3 H); 2.96 (d, 2 H); 2.40 (s, 3 H); 2.30 (td, 2 H); 2.03 - 2.18 (m, 2 H); 2.09 (dd, 3 H); 1.55 - 1.90 (m, 4 H); 1.31 (d, 3 H); 1.02 (t, 3 H).
¹³C NMR (CDCl₃) δ (ppm): 166.68 (s, 1 C); 157.42 (s, 1 C); 154.65 (s, 1 C); 150.29 (s, 1 C); 147.33 (s, 1 C); 144.56 (s, 1 C); 144.18 (s, 1 C); 137.79 (s, 1 C); 132.72 (s, 1 C); 130.41 (s, 1 C); 127.03 (s, 1 C); 124.90 (s, 1 C); 120.56 (s, 1 C); 119.05 (s, 1 C); 116.04 (s, 1 C); 109.15 (s, 1 C); 80.09 (s, 1 C); 56.00 (s, 1 C); 54.51 (s, 2 C); 46.26 (s, 1 C); 45.82 (s, 1 C); 31.92 (s, 2 C); 30.00 (s, 1 C); 28.80 (s, 1 C); 20.47 (s, 1 C); 19.96 (s, 1 C); 9.74 (s, 1 C).
MS (ESI Pos, 3.2 kV, 25 V, 350°C): m/z 535.29 (MH⁺).

### Example 41: 4-({7-isobutoxy-5-methyl-6-oxo-8-[(1E)-prop-1-en-1-yl]-5,6-dihydro-pyrido[3,2-d]pyrimidin-2-yl}amino)-3-methoxy-N-(1-methylpiperidin-4-yl)benzamide

The title compound was prepared analogously to example 2 using 1-bromo-2-methylpropane instead of iodomethane in step 3.
¹H NMR (CDCl₃) δ (ppm): 8.68 (d, 1 H); 8.59 (s, 1 H); 7.96 (s, 1 H); 7.48 (d, 1 H); 7.30 (dd, 1 H); 7.13 (dq, 1 H); 6.96 (dq, 1 H); 5.95 (d, 1 H); 4.13 (d, 2 H); 4.03 (s, 3 H); 3.94 - 4.09 (m, 1 H); 3.74 (s, 3 H); 2.67 - 3.08 (m, 2 H); 2.33 (s, 3 H); 2.15 - 2.26 (m, 2 H); 2.10 (dd, 3 H); 2.01 - 2.16 (m, 3 H); 1.47 - 1.75 (m, 2 H); 1.07 (d, 6 H).
¹³C NMR (CDCl₃) δ (ppm): 166.67 (s, 1 C); 157.32 (s, 1 C); 154.66 (s, 1 C); 151.26 (s, 1 C); 147.31 (s, 1 C); 144.54 (s, 1 C); 144.18 (s, 1 C); 137.74 (s, 1 C); 132.59 (s, 1 C); 129.70 (s, 1 C); 127.26 (s, 1 C); 124.89 (s, 1 C); 120.18 (s, 1 C); 118.96 (s, 1 C); 115.99 (s, 1 C); 109.16 (s, 1 C); 79.04 (s, 1 C); 55.98 (s, 1 C); 54.57 (s, 2 C); 46.60 (s, 1 C); 46.16 (s, 1 C); 32.36 (s, 2 C); 29.35 (s, 1 C); 28.76 (s, 1 C); 20.39 (s, 1 C); 19.20 (s, 2 C).
MS (ESI Pos, 3.2 kV, 25 V, 350°C): 535.35 m/z (MH⁺).

### Example 42: 4-({7-(cyclopentylmethoxy)-5-methyl-6-oxo-8-[(1E)-prop-1-en-1-yl]-5,6-dihydropyrido[3,2-d]pyrimidin-2-yl}amino)-3-methoxy-N-(1-methylpiperidin-4-yl)benzamide

### Step 1: 8-allyl-2-chloro-7-(cyclopentylmethoxy)-5-methylpyrido[3,2-d]pyrimidin-6(5H)-one

8-allyl-2-chloro-7-hydroxy-5-methylpyrido[3,2-d]pyrimidin-6(5H)-one (0.225 g), iodo-methylcyclopentane (0.567 g) and K₂CO₃ (0.373 g) were mixed in dimethylformamide (4 ml) and heated to 100°C under microwave irradiation for 4 hours. The reaction mixture was partitioned between water and ethyl acetate. The organic layer was dried over Na₂SO₄ and concentrated. The residue was purified on silica gel (eluent: dichloro-methane/ethyl acetate 20/1 (v/v)) to give the title compound as pale yellow solid.
¹H NMR (CDCl₃) δ (ppm): 8.64 (s, 1 H); 5.82 - 6.17 (m, 1 H); 5.17 (dq, 1 H); 5.03 - 5.11 (m, 1 H); 4.31 (d, 2 H); 3.75 (s, 3 H); 3.77 (dt, 2 H); 2.27 - 2.55 (m, 1 H); 1.77 - 1.96 (m, 2 H); 1.57 - 1.76 (m, 4 H); 1.33 - 1.51 (m, 2 H).

### Step 2: 2-chloro-7-(cyclopentylmethoxy)-5-methyl-8-[(1E)-prop-1-en-1-yl]pyrido[3,2-d]pyrimidin-6(5H)-one

The product of step 1 (0.165 g) and 1,8-diazabicyclo [5.4.0] undec-7-ene (0.072 ml) were mixed in dioxane (5 ml) and the mixture was heated to 60°C for 6 hours. The reaction mixture was partitioned between water and ethyl acetate. The organic layer was separated, dried over Na₂SO₄ and concentrated. The residue was purified on silica gel (eluent: dichloromethane/ ethyl acetate 20/1 (v/v)) to give the title compound as off-white solid.
¹H NMR (CDCl₃) δ (ppm): 8.65 (s, 1 H); 7.28 (dq, 1 H); 6.96 (dq, 1 H); 4.28 (d, 2 H); 3.75 (s, 3 H); 2.25 - 2.54 (m, 1 H); 2.06 (dd, 3 H); 1.76 - 1.94 (m, 2 H); 1.56 - 1.73 (m, 4 H); 1.31 - 1.49 (m, 2 H).

### Step 3: 4-({7-(cyclopentylmethoxy)-5-methyl-6-oxo-8-[(1E)-prop-1-en-1-yl]-5,6-dihydro-pyrido[3,2-d]pyrimidin-2-yl}amino)-3-methoxy-N-(1-methylpiperidin-4-yl)benzamide

The product of step 2 (0.134 g), Cs₂CO₃ (0.521 g), (+/-) 2,2'-bis-(diphenylphosphino)-1,1'-binaphthyl (0.030 g), tris(dibenzylideneacetone)dipalladium (0) (0.044 g) and 4-amino-3-methoxy-N-(1-methylpiperidin-4-yl)benzamide (example 1, step 6) (0.117 g) were mixed in dry toluene (5 ml, degassed), and the mixture was refluxed for 3 hours. The mixture was then stirred at room temperature for 40 hours; Tris(dibenzylideneacetone)dipalladium (0) (0.044 g) and (+/-) 2,2'-bis-(diphenylphosphino)-1,1'-binaphthyl (0.030 g) were added and the mixture was heated to reflux for an additional 3 hours. The mixture was partitioned between water and dichloromethane, the organic layer was concentrated and the residue was purified on silica gel (eluent: dichlormethane/methanol/32% NH₄OH 20/1/0.15 (v/v)) and further triturated in a mixture of methanol/diethyl ether 1/8 (v/v) to give the title compound as yellow solid.
¹H NMR (CDCl₃) δ (ppm): 8.68 (d, 1 H); 8.59 (s, 1 H); 7.96 (s, 1 H); 7.47 (d, 1 H); 7.30 (dd, 1 H); 7.16 (dq, 1 H); 6.97 (dq, 1 H); 5.96 (d, 1 H); 4.24 (d, 2 H); 4.03 (s, 3 H); 4.01 - 4.05 (m, 1 H); 3.74 (s, 3 H); 2.86 (d, 2 H); 2.33 (s, 3 H); 2.30 - 2.52 (m, 1 H); 2.20 (td, 2 H); 2.10 (dd, 3 H); 2.01 - 2.14 (m, 2 H); 1.77 - 1.94 (m, 2 H); 1.53 - 1.74 (m, 6 H); 1.32 - 1.51 (m, 2 H).
¹³C NMR (CDCl₃) δ (ppm): 166.66 (s, 1 C); 157.35 (s, 1 C); 154.68 (s, 1 C); 151.18 (s, 1 C); 147.33 (s, 1 C); 144.54 (s, 1 C); 144.19 (s, 1 C); 137.83 (s, 1 C); 132.62 (s, 1 C); 129.69 (s, 1 C); 127.24 (s, 1 C); 124.92 (s, 1 C); 120.22 (s, 1 C); 118.96 (s, 1 C); 116.01 (s, 1 C); 109.18 (s, 1 C); 76.87 (s, 1 C); 55.98 (s, 1 C); 54.56 (s, 2 C); 46.57 (s, 1 C); 46.14 (s, 1 C); 40.17 (s, 1 C); 32.34 (s, 2 C); 29.48 (s, 2 C); 28.77 (s, 1 C); 25.54 (s, 2 C); 20.46 (s, 1 C).
MS (ESI Pos, 3.2 kV, 25 V, 350°C): 561.42 m/z (MH⁺).

### Example 43: 4-({7-(cyclobutyloxy)-5-methyl-6-oxo-8-[(1E)-prop-1-en-1-yl]-5,6-dihydropyrido[3,2-d]pyrimidin-2-yl}amino)-3-methoxy-N-(1-methylpiperidin-4-yl)benzamide

### Step 1: 8-allyl-2-chloro-7-(cyclobutyloxy)-5-methylpyrido[3,2-d]pyrimidin-6(5H)-one

8-allyl-2-chloro-7-hydroxy-5-methylpyrido[3,2-d]pyrimidin-6(5H)-one (0.050 g), triphenylphosphine (0.079 g) and cyclobutanol (0.024 ml) were dissolved in dry tetrahydrofuran (4 ml) under nitrogen atmosphere and cooled at 0°C. Diethyl azodicarboxylate (0.047 ml) was added dropwise and the mixture was stirred for 40 minutes at room temperature. The reaction mixture was concentrated and the residue was purified on silica gel (eluent: dichloromethane/ ethyl acetate 20/1 (v/v)) to give the title compound as white solid.
¹H NMR (CDCl₃) δ (ppm): 8.63 (s, 1 H); 5.78 - 6.19 (m, 1 H); 5.18 - 5.32 (m, 1 H); 5.18 (ddd, 1 H); 5.04 - 5.12 (m, 1 H); 3.77 (dt, 2 H); 3.74 (s, 3 H); 2.33 - 2.49 (m, 2 H); 2.16 - 2.33 (m, 2 H); 1.70 - 1.89 (m, 1 H); 1.45 - 1.66 (m, 1 H).

### Step 2: 2-chloro-7-(cyclobutyloxy)-5-methyl-8-[(1E)-prop-1-en-1-yl]pyrido[3,2-d]pyrimidin-6(5H)-one

The product of step 1 (0.200 g) and 1,8-diazabicyclo [5.4.0] undec-7-ene (0.096 ml) were mixed in dioxane (7 ml) and the mixture was heated to 60°C for 6 hours. After keeping the mixture at room temperature for 12 h, the mixture was heated again to 60°C for 5 hours, then it was partitioned between water and ethyl acetate. The organic layer was dried over Na₂SO₄ and concentrated. The residue was purified on silica gel (eluent: dichloromethane/ethyl acetate 20/1 (v/v)) to give the title compound as white solid.
¹H NMR (CDCl₃) δ (ppm): 8.63 (s, 1 H); 7.28 - 7.43 (dq, 1 H); 6.97 (dq, 1 H); 5.11 - 5.30 (m, 1 H); 3.73 (s, 3 H); 2.17 - 2.49 (m, 4 H); 2.06 (dd, 3 H); 1.69 - 1.88 (m, 1 H); 1.44 - 1.65 (m, 1 H).

### Step 3: 4-({7-(cyclobutyloxy)-5-methyl-6-oxo-8-[(1E)-prop-1-en-1-yl]-5,6-dihydro-pyrido[3,2-d]pyrimidin-2-yl}amino)-3-methoxy-N-(l-methylpiperidin-4-yl)benzamide

The product of step 3 (0.148 g), Cs₂CO₃ (0.645 g), (+/-) 2,2'-bis (diphenylphosphino)-1,1'-binaphthyl (0.037 g), tris(dibenzylideneacetone)dipalladium (0) (0.055 g) and 4-amino-3-methoxy-N-(1-methylpiperidin-4-yl)benzamide (example 1, step 6) (0.142 g) were mixed in dry toluene (6 ml, degassed), and the mixture was refluxed for 4 hours under nitrogen atmosphere. The mixture was then cooled to room temperature and partitioned between dichloromethane and water. The organic phase was separated and the aqueous phase was extracted with dichloromethane. The combined organic layers were dried over Na₂SO₄ and concentrated. The residue was purified on silica gel (eluent: dichloromethane/methanol/32% NH₄OH 20/1.6/0.16 (v/v)). The title compound precipitated from a mixture of methanol/water by continuous addition of water.
¹H NMR (CDCl₃) δ (ppm): 8.67 (d, 1 H); 8.58 (s, 1 H); 7.95 (s, 1 H); 7.47 (d, 1 H); 7.30 (dd, 1 H); 7.23 (dq, 1 H); 6.99 (dq, 1 H); 5.96 (d, 1 H); 5.03 - 5.33 (m, 1 H); 4.03 (s, 3 H); 3.91 - 4.13 (m, 1 H); 3.73 (s, 3 H); 2.69 - 2.98 (m, 2 H); 2.33 (s, 3 H); 2.24 - 2.47 (m, 4 H); 2.14 - 2.23 (m, 2 H); 2.11 (dd, 3 H); 2.01 - 2.14 (m, 2 H); 1.73 - 1.90 (m, 1 H); 1.60 - 1.72 (m, 2 H); 1.43 - 1.70 (m, 1 H).
¹³C NMR (CDCl₃) δ (ppm): 166.63 (s, 1 C); 157.26 (s, 1 C); 154.70 (s, 1 C); 149.89 (s, 1 C); 147.37 (s, 1 C); 144.52 (s, 1 C); 144.19 (s, 1 C); 137.77 (s, 1 C); 132.63 (s, 1 C); 129.47 (s, 1 C); 127.29 (s, 1 C); 124.90 (s, 1 C); 120.45 (s, 1 C); 118.89 (s, 1 C); 116.04 (s, 1 C); 109.20 (s, 1 C); 76.26 (s, 1 C); 55.99 (s, 1 C); 54.58 (s, 2 C); 46.65 (s, 1 C); 46.24 (s, 1 C); 32.47 (s, 2 C); 31.77 (s, 2 C); 28.84 (s, 1 C); 20.49 (s, 1 C); 12.55 (s, 1 C).
MS (ESI Pos, 3.2kV, 25V, 350°C): 533.39 m/z (MH⁺).

### Example 44: 4-{[7-ethoxy-5-methyl-8-(1,3-oxazol-2-yl)-6-oxo-5,6-dihydropyrido[3,2-d]pyrimidin-2-yl]amino}-3-methoxy-N-(1-methylpiperidin-4-yl)benzamide

### Step 1: 2-chloro-7-ethoxy-5-methyl-8-(1,3-oxazol-2-yl)pyrido[3,2-d]pyrimidin-6(5H)-one

8-bromo-2-chloro-7-ethoxy-5-methylpyrido[3,2-d]pyrimidin-6(5H)-one (prepared analogously to the product of example 3, step 2 using iodoethane instead of iodomethane in step 1) (0.180 g) was dissolved in toluene (3 ml) under nitrogen atmosphere, and 2-(tributylstannyl)-1,3-oxazole (0.238 ml), triphenylphosphine (0.037 g) and tris(dibenzylideneacetone)dipalladium (0) (0.026 g) were added. The solution was stirred at reflux for 2 hours and then 2-(tributyl-stannyl)-1,3-oxazole (0.120 ml), triphenylphosphine (0.037 g) and tris(dibenzylidene-acetone)dipalladium (0) (0.026 g) were added. The solution was stirred at reflux for 2 hours and then 2-(tributylstannyl)-1,3-oxazole (0.120 ml), triphenylphosphine (0.037 g) and tris(dibenzylideneacetone)dipalladium (0) (0.026 g) were added. After stirring for 3 hours at reflux, the mixture was cooled to room temperature, diluted with dichloromethane and washed with water and a saturated solution of NaCl. The organic layer was dried over Na₂SO₄ and concentrated. The residue was purified on silica gel (eluent: dichloromethane/ ethyl acetate 9/1 (v/v)) to give the title compound as white solid.
¹H NMR (CDCl₃) δ (ppm): 8.69 (s, 1 H); 7.92 (d, 1 H); 7.42 (d, 1 H); 4.40 (q, 2 H); 3.81 (s, 3 H); 1.34 (t, 3 H).

### Step 2: 4-{[7-ethoxy-5-methyl-8-(1,3-oxazol-2-yl)-6-oxo-5,6-dihydropyrido[3,2-d]pyrimi-din-2-yl]amino}-3-methoxy-N-(1-methylpiperidin-4-yl)benzamide

The product of step 1 (0.102 g) was dissolved under nitrogen atmosphere in dry toluene (5 ml). Cs₂CO₃ (0.430 g), (+/-) 2,2'-bis-(diphenylphosphino)-1,1'-binaphthyl (0.031 g), tris(dibenzylideneacetone)dipalladium (0) (0.046 g) and 4-amino-3-methoxy-N-(1-methyl-piperidin-4-yl)benzamide (example 1, step 6) (0.114 g) were added and the mixture was stirred at reflux for 8 hours. The mixture was cooled to room temperature, diluted with dichloromethane and washed with water and a saturated solution of NaCl. The organic phase was dried over Na₂SO₄ and concentrated. The residue was purified on silica gel (eluent: dichloromethane/methanol/32% NH₄OH 95/5/0.5 (v/v)) to give a solid that was further washed with water to give the title compound as yellow solid.
¹H NMR (CDCl₃) δ (ppm): 8.63 (s, 1 H); 8.29 (d, 1 H); 8.01 (d, 1 H); 7.98 (s, 1 H); 7.53 (d, 1 H); 7.38 (d, 1 H); 7.12 (dd, 1 H); 5.89 (d, 1 H); 4.41 (q, 2 H); 3.98 (s, 3 H); 3.93 - 4.10 (m, 1 H); 3.81 (s, 3 H); 2.65 - 2.99 (m, 2 H); 2.33 (s, 3 H); 2.19 (td, 2 H); 1.88 - 2.13 (m, 2 H); 1.59 - 1.74 (m, 2 H); 1.35 (t, 3 H).
MS (ESI Pos, 3.2kV, 25V, 350°C): 534.52 m/z (MH⁺).

### Example 45: 4-({7-(1-cyclopropylethoxy)-5-methyl-6-oxo-8-[(1E)-prop-1-en-1-yl]-5,6-dihydropyrido[3,2-d]pyrimidin-2-yl}amino)-3-methoxy-N-(1-methylpiperidin-4-yl)benzamide

The title compound was prepared analogously to example 43 using 1-cyclopropylethanol instead of cyclobutanol in step 1.
¹H NMR (CDCl₃) δ (ppm): 8.69 (d, 1 H); 8.60 (s, 1 H); 7.96 (s, 1 H); 7.48 (d, 1 H); 7.28 - 7.35 (m, 2 H); 6.98 - 7.08 (m, 1 H); 5.95 (d, 1 H); 4.39 - 4.52 (m, 1 H); 4.03 (s, 3 H); 3.93 - 4.09 (m, 1 H); 3.73 (s, 3 H); 2.79 - 2.91 (m, 2 H); 2.33 (s, 3 H); 2.19 (td, 2 H); 2.03 - 2.14 (m, 5 H); 1.62 (s, 2 H); 1.47 (d, 3 H); 1.04 - 1.17 (m, 1 H); 0.41 - 0.56 (m, 2 H); 0.29 - 0.41 (m, 1 H); 0.16 - 0.26 (m, 1 H).
¹³C NMR (CDCl₃) δ (ppm): 166.64 (s, 1 C); 157.51 (s, 1 C); 154.70 (s, 1 C); 150.37 (s, 1 C); 147.39 (s, 1 C); 144.55 (s, 1 C); 144.24 (s, 1 C); 137.70 (s, 1 C); 132.65 (s, 1 C); 130.73 (s, 1 C); 127.27 (s, 1 C); 124.98 (s, 1 C); 120.67 (s, 1 C); 118.89 (s, 1 C); 116.09 (s, 1 C); 109.21 (s, 1 C); 83.40 (s, 1 C); 55.99 (s, 1 C); 54.57 (s, 2 C); 46.62 (s, 1 C); 46.22 (s, 1 C); 32.45 (s, 2 C); 28.77 (s, 1 C); 20.75 (s, 1 C); 20.49 (s, 1 C); 17.37 (s, 1 C); 4.33 (s, 1 C); 1.79 (s, 1 C).
MS (ESI Pos, 3.2 kV, 25 V, 350°C): 547.54 m/z (MH⁺).

### Example 46: 4-[(8-ethynyl-7-methoxy-5-methyl-6-oxo-5,6-dihydropyrido[3,2-d]pyri-midin-2-yl)amino]-3-methoxy-N-(1-methylpiperidin-4-yl)benzamide

### Step 1: 2-chloro-7-methoxy-5-methyl-8-[(trimethylsilyl)ethynyl]pyrido[3,2-d]pyrimidin-6(5H)-one

Dimethylformamide (6 ml) and N-ethyldiisopropylamine (EDIPA) (0.124 ml) were added to a mixture of 8-bromo-2-chloro-7-methoxy-5-methylpyrido[3,2-d]pyrimidin-6(5H)-one (0.200 g), dichlorobis(triphenylphosphine)-palladium(II) (0.024 g) and Cul (0.006 g). The solution was degassed bubbling nitrogen for 10 minutes and then ethynyl(trimethyl)silane (0.140 ml) was added. The mixture was heated to 80°C for 6 hours under nitrogen atmosphere and then cooled to room temperature. The mixture was concentrated to dryness, re-dissolved in dichloromethane and washed with water. The organic phase was dried over Na₂SO₄ and concentrated. The crude was purified on silica gel (eluent: dichloromethane/ethyl acetate 85/15 (v/v)) to give the title compound as white solid.
¹H NMR (CDCl₃) δ (ppm): 8.62 (s, 1 H); 4.44 (s, 3 H); 3.76 (s, 3 H), 0.36 (s, 9 H).

### Step 2: 4-[(8-ethynyl-7-methoxy-5-methyl-6-oxo-5,6-dihydropyrido[3,2-d]pyrimidin-2-yl)amino]-3-methoxy-N-(1-methylpiperidin-4-yl)benzamide

The product of step 1 (0.170 g), 4-amino-3-methoxy-N-(1-methylpiperidin-4-yl)benzamide (example 1, step 6) (0.181 g), Cs₂CO₃ (0.690 g), (+/-) 2,2'-bis-(diphenylphosphino)-1,1'-binaphthyl (0.049 g) and tris(dibenzylideneacetone)dipalladium (0) (0.072 g) were mixed in dry toluene (6 ml) under nitrogen atmosphere. The reaction mixture was stirred at reflux for 3 hours and then (+/-) 2,2'-bis-(diphenylphosphino)-1,1'-binaphthyl (0.049 g) and tris(dibenzy-lideneacetone)dipalladium (0) (0.072 g) were added. After stirring at reflux for 2 hours, additional (+/-) 2,2'-bis-(diphenylphosphino)-1,1'-binaphthyl (0.049 g) and tris(dibenzylidene-acetone)dipalladium (0) (0.072 g) were added. After stirring at reflux for 1 hour, the mixture was stirred at room temperature overnight and then diluted with dichloromethane and washed with water and a saturated solution of NaCl. The organic phase was dried over Na₂SO₄ and concentrated. The crude product was sequentially purified on silica gel (dichloromethane/ methanol/32%NH₄OH 96/4/0.4 (v/v)) and preparative HPLC (acetonitrile/water/trifluoroacetic acid 200/1800/1 (v/v) / acetonitrile/water/trifluoroacetic acid 1800/200/1 (v/v), elution gradient 95/5 to 0/100 to 95/5 (v/v) to give the title compound as yellow solid.
¹H NMR (CDCl₃) δ (ppm): 8.99 (d, 1 H); 8.59 (s, 1 H); 8.10 (s, 1 H); 7.48 (d, 1 H); 7.29 - 7.34 (m, 1 H); 5.89 - 6.05 (m, 1 H); 4.41 (s, 3 H); 4.02 (s, 3 H); 3.97 - 4.09 (m, 1 H); 3.96 (s, 1 H); 3.76 (s, 3 H); 2.86 (dt, 2 H); 2.33 (s, 3 H); 2.01 - 2.25 (m, 4 H); 1.62 - 1.71 (m, 2 H).
MS (ESI Pos, 3.2 kV, 25 V, 350°C): 477.34 m/z (MH⁺).

### Example 47: 4-{[7-(cyclopropylmethoxy)-5-methyl-6-oxo-8-(3-thienyl)-5,6-dihydropyrido[3,2-d]pyrimidin-2-yl]amino}-3-methoxy-N-[2-(1-methylpyrrolidin-2-yl)ethyl]benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methyl-pyrido[3,2-d]pyrimidin-6(5H)-one and using 4-amino-3-methoxy-N-[2-(1-methylpyrrolidin-2-yl)ethyl]benzamide instead of 4-amino-3-methoxy-N-(1-methylpiperidin-4-yl)benzamide (example 1, step 6) in step 2. 4-amino-3-methoxy-N-[2-(1-methylpyrrolidin-2-yl)ethyl]benzamide was prepared analogously to example 1 step 6 using 2-(1-methylpyrrolidin-2-yl)ethaneamine instead of 1-methylpiperidin-4-amine.
¹H NMR (CDCl₃) δ (ppm): 8.64 (s, 1 H); 8.38 (d, 1 H); 8.31 (br. s., 1 H); 7.98 (s, 1 H); 7.82 (dd, 1 H); 7.41 - 7.56 (m, 3 H); 7.01 - 7.15 (m, 1 H); 4.07 (d, 2 H); 3.98 (s, 3 H); 3.80 (s, 3 H); 3.67 - 3.86 (m, 1 H); 3.36 - 3.55 (m, 1 H); 3.12 - 3.26 (m, 1 H); 2.43 - 2.58 (m, 1 H); 2.45 (s, 3 H); 2.17 - 2.38 (m, 1 H); 1.70 - 2.02 (m, 6 H); 0.98 - 1.15 (m, 1 H); 0.42 - 0.53 (m, 2 H); 0.10 - 0.25 (m, 2 H)
¹³C NMR (CDCl₃) δ (ppm): 166.73 (s, 1 C); 157.52 (s, 1 C); 154.90 (s, 1 C); 151.57 (s, 1 C); 147.00 (s, 1 C); 144.65 (s, 1 C); 144.40 (s, 1 C); 132.07 (s, 1 C); 130.72 (s, 1 C); 130.60 (s, 1 C); 130.49 (s, 1 C); 127.86 (s, 1 C); 127.13 (s, 1 C); 125.08 (s, 1 C); 123.52 (s, 1 C); 119.08 (s, 1 C); 116.17 (s, 1 C); 109.00 (s, 1 C); 77.69 (s, 1 C); 65.40 (s, 1 C); 57.00 (s, 1 C); 55.91 (s, 1 C); 40.49 (s, 1 C); 37.05 (s, 1 C); 29.16 (s, 1 C); 28.96 (s, 1 C); 28.40 (s, 1 C); 22.51 (s, 1 C); 10.97 (s, 1 C); 3.16 (s, 2 C).
MS (ESI Pos, 3.2 kV, 25 V, 350°C): 589.24 m/z (MH⁺).

### Example 48: 4-{[7-(cyclopropylmethoxy)-5-methyl-6-oxo-8-(3-thienyl)-5,6-dihydro-pyrido[3,2-d]pyrimidin-2-yl]amino}-3-methoxy-N-[(1-methylpiperidin-4-yl)methyl] benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methyl-pyrido[3,2-d]pyrimidin-6(5H)-one and using 4-amino-3-methoxy-N-[(1-methylpiperidin-4-yl)methyl]benzamide instead of 4-amino-3-methoxy-N-(1-methylpiperidin-4-yl)benzamide (example 1, step 6) in step 2. 4-amino-3-methoxy-N-(1-methyl-piperidin-4-ylmethyl)-benzamide was prepared analogously to example 1 step 6 using 1-(1-methylpiperidin-4-yl)methanamine instead of 1-methylpiperidin-4-amine.
¹H NMR (CDCl₃) δ (ppm): 8.64 (s, 1 H); 8.39 (d, 1 H); 7.97 (s, 1 H); 7.81 (dd, 1 H); 7.46 - 7.55 (m, 2 H); 7.44 (d, 1 H); 7.09 (dd, 1 H); 6.19 (t, 1 H); 4.07 (d, 2 H); 3.98 (s, 3 H); 3.79 (s, 3 H); 3.39 (t, 2 H); 2.91 (dt, 2 H); 2.30 (s, 3 H); 1.90 - 2.07 (m, 2 H); 1.59 - 1.86 (m, 3 H); 1.33 - 1.51 (m, 2 H); 0.97 - 1.15 (m, 1 H); 0.41 - 0.52 (m, 2 H); 0.12 - 0.23 (m, 2 H). ¹³C NMR (CDCl₃) δ (ppm): 167.31 (s, 1 C); 157.55 (s, 1 C); 154.88 (s, 1 C); 151.63 (s, 1 C); 147.14 (s, 1 C); 144.62 (s, 1 C); 144.47 (s, 1 C); 132.37 (s, 1 C); 130.69 (s, 1 C); 130.57 (s, 1 C); 130.42 (s, 1 C); 127.93 (s, 1 C); 127.02 (s, 1 C); 125.17 (s, 1 C); 123.66 (s, 1 C); 118.85 (s, 1 C); 116.24 (s, 1 C); 109.06 (s, 1 C); 77.72 (s, 1 C); 55.95 (s, 1 C); 55.39 (s, 2 C); 46.19 (s, 1 C); 45.46 (s, 1 C); 35.49 (s, 1 C); 29.91 (s, 2 C); 28.97 (s, 1 C); 10.99 (s, 1 C); 3.17 (s, 2 C).
MS (ESI Pos, 3.2 kV, 25 V, 350°C): 589.2m/z (MH⁺).

### Example 49: 4-{[7-(cyclopropylmethoxy)-5-methyl-6-oxo-8-(3-thienyl)-5,6-dihydropyrido[3,2-d]pyrimidin-2-yl]amino}-N-(1-isopropylpiperidin-4-yl)-3-methoxybenzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methyl-pyrido[3,2-d]pyrimidin-6(5H)-one and using 4-amino-N-(1-isopropylpiperidin-4-yl)-3-methoxy-benzamide instead of 4-amino-3-methoxy-N-(1-methylpiperidin-4-yl)benzamide (example 1, step 6) in step 2. 4-Amino-N-(1-isopropyl-piperidin-4-yl)-3-methoxy-benzamide was prepared analogously to example 1 step 6 using 1-isopropylpiperidin-4-amine instead of 1-methylpiperidin-4-amine.
¹H NMR (CDCl₃) δ (ppm): 8.63 (s, 1 H); 8.39 (d, 1 H); 7.96 (s, 1 H); 7.81 (dd, 1 H); 7.44 - 7.55 (m, 2 H); 7.42 (d, 1 H); 7.06 (dd, 1 H); 5.93 (d, 1 H); 4.06 (d, 2 H); 3.93 - 4.03 (m, 1 H); 3.98 (s, 3 H); 3.79 (s, 3 H); 2.84 - 2.98 (m, 2 H); 2.69 - 2.84 (m, 1 H); 2.35 (td, 2 H); 2.00 - 2.16 (m, 2 H); 1.48 - 1.62 (m, 2 H); 1.08 (d, 6 H); 0.98 - 1.16 (m, 1 H); 0.40 - 0.53 (m, 2 H); 0.10 - 0.23 (m, 2 H).
¹³C NMR (CDCl₃) δ (ppm): 166.54 (s, 1 C); 157.57 (s, 1 C); 154.91 (s, 1 C); 151.64 (s, 1 C); 147.17 (s, 1 C); 144.61 (s, 1 C); 144.50 (s, 1 C); 132.32 (s, 1 C); 130.67 (s, 1 C); 130.60 (s, 1 C); 130.40 (s, 1 C); 127.96 (s, 1 C); 127.29 (s, 1 C); 125.17 (s, 1 C); 123.67 (s, 1 C); 118.84 (s, 1 C); 116.34 (s, 1 C); 109.01 (s, 1 C); 77.73 (s, 1 C); 55.95 (s, 1 C); 54.50 (s, 1 C); 47.56 (s, 2 C); 47.26 (s, 1 C); 32.74 (s, 2 C); 28.98 (s, 1 C); 18.41 (s, 2 C); 10.98 (s, 1 C); 3.17 (s, 2 C).
MS (ESI Pos, 3.2 kV, 25 V, 350°C): 603.20 m/z (MH⁺).

### Example 50: 4-{[7-ethoxy-5-methyl-6-oxo-8-(1,3-thiazol-2-yl)-5,6-dihydropyrido[3,2-d]pyrimidin-2-yl]amino}-3-methoxy-N-(1-methylpiperidin-4-yl)benzamide

The title compound was prepared analogously to example 44 using 2-(tributylstannyl)-1,3-thiazole instead of 2-(tributylstannyl)-1,3-oxazole in step 1.
¹H NMR (CDCl₃) δ (ppm): 8.64 (s, 1 H); 8.32 (d, 1 H); 8.18 (d, 1 H); 7.97 (s, 1 H); 7.72 (d, 1 H); 7.36 (d, 1 H); 7.07 (dd, 1 H); 5.87 (d, 1 H); 4.48 (q, 2 H); 3.97 (s, 2 H); 3.91 - 4.12 (m, 2 H); 3.81 (s, 3 H); 2.85 (dt, 2 H); 2.32 (s, 3 H); 1.99 - 2.25 (m, 4 H); 1.49 - 1.70 (m, 2 H); 1.33 (t, 3 H).
¹³C NMR (CDCl₃) δ (ppm): 166.74 (s, 1 C); 157.45 (s, 1 C); 156.85 (s, 1 C); 154.96 (s, 1 C); 153.44 (s, 1 C); 147.07 (s, 1 C); 144.53 (s, 1 C); 143.86 (s, 1 C); 143.06 (s, 1 C); 132.22 (s, 1 C); 127.26 (s, 1 C); 126.56 (s, 1 C); 124.62 (s, 1 C); 121.57 (s, 1 C); 119.05 (s, 1 C); 116.34 (s, 1 C); 108.75 (s, 1 C); 69.88 (s, 1 C); 55.93 (s, 1 C); 54.61 (s, 2 C); 46.66 (s, 1 C); 46.22 (s, 1 C); 32.43 (s, 2 C); 29.18 (s, 1 C); 15.60 (s, 1 C).
MS (ESI Pos, 3.2 kV, 25 V, 350°C): 550.22 m/z (MH⁺).

### Example 51: 4-{[7-(cyclopropylmethoxy)-5-methyl-6-oxo-8-phenyl-5,6-dihydro-pyrido[3,2-d]pyrimidin-2-yl]amino}-3-methoxy-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methyl-pyrido[3,2-d]pyrimidin-6(5H)-one and using phenylboronic acid instead of 3-thienyl-boronic acid in step 1.
¹H NMR (CDCl₃) δ (ppm): 8.63 (s, 1 H); 8.11 (d, 1 H); 7.94 (s, 1 H); 7.47 - 7.62 (m, 5 H); 7.35 (d, 1 H); 6.89 (dd, 1 H); 5.84 (d, 1 H); 4.05 (d, 2 H); 3.95 (s, 3 H); 3.90 - 4.02 (m, 1 H); 3.81 (s, 3 H); 2.72 - 2.93 (m, 2 H); 2.33 (s, 3 H); 2.18 (td, 2 H); 1.97 - 2.13 (m, 2 H); 1.52 - 1.73 (m, 2 H); 0.95 - 1.12 (m, 1 H); 0.36 - 0.49 (m, 2 H); 0.04 - 0.18 (m, 2 H).
¹³C NMR (CDCl₃) δ (ppm):166.68 (s, 1 C); 157.67 (s, 1 C); 154.85 (s, 1 C); 151.66 (s, 1 C); 147.02 (s, 1 C); 144.83 (s, 1 C); 144.46 (s, 1 C); 135.60 (s, 1 C); 132.40 (s, 1 C); 131.99 (s, 1 C); 130.64 (s, 2 C); 128.05 (s, 1 C); 127.77 (s, 2 C); 127.02 (s, 1 C); 125.17 (s, 1 C); 118.90 (s, 1 C); 116.18 (s, 1 C); 108.72 (s, 1 C); 77.65 (s, 1 C); 55.92 (s, 1 C); 54.57 (s, 2 C); 46.51 (s, 1 C); 46.24 (s, 1 C); 32.44 (s, 2 C); 28.99 (s, 1 C); 10.89 (s, 1 C); 3.09 (s, 2 C).
MS (ESI Pos, 3.2 kV, 25 V, 350°C): 569.26 m/z (MH⁺).

### Example 52: 4-{[7-(cyclopropylmethoxy)-8-(4-fluorophenyl)-5-methyl-6-oxo-5,6-dihydropyrido[3,2-d]pyrimidin-2-yl]amino}-3-methoxy-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methylpyrido[3,2-d]pyrimidin-6(5H)-one and using 4-fluorophenylboronic acid instead of 3-thienylboronic acid in step 1.
¹H NMR (CDCl₃) δ (ppm): 8.64 (s, 1 H); 8.11 (d, 1 H); 7.94 (s, 1 H); 7.52 (m, 2 H); 7.41 (d, 1 H); 7.24 (m, 2 H); 6.93 (dd, 1 H); 5.90 (d, 1 H); 4.09 (d, 2 H); 3.99 - 4.06 (m, 1 H); 3.97 (s, 3 H); 3.81 (s, 3 H); 2.84 (dt, 2 H); 2.34 (s, 3 H); 2.14 - 2.28 (m, 2 H); 2.01 - 2.14 (m, 2 H); 1.50 - 1.74 (m, 2 H); 0.95 - 1.11 (m, 1 H); 0.40 - 0.50 (m, 2 H); 0.10 - 0.19 (m, 2 H).
¹³C NMR (CDCl₃) δ (ppm): 166.54 (s, 1 C); 162.62 (d, 1 C); 157.51 (s, 1 C); 154.85 (s, 1 C); 151.70 (s, 1 C); 147.13 (s, 1 C); 144.55 (s, 1 C); 144.58 (s, 1 C); 134.43 (s, 1 C); 132.66 (d, 2 C); 132.24 (s, 1 C); 127.78 (d, 1 C); 127.23 (s, 1 C); 125.15 (s, 1 C); 118.57 (s, 1 C); 116.12 (s, 1 C); 114.79 (d, 2 C); 108.99 (s, 1 C); 77.68 (s, 1 C); 55.92 (s, 1 C); 54.46 (s, 2 C); 46.45 (s, 1 C); 46.22 (s, 1 C); 32.33 (s, 2 C); 29.00 (s, 1 C); 10.92 (s, 1 C); 3.12 (s, 2 C).
MS (ESI Pos, 3.2 kV, 25 V, 350°C): 587.17 m/z (MH⁺).

### Example 53: 4-{[7-(cyclopropylmethoxy)-5-methyl-6-oxo-8-(2-thienyl)-5,6-dihydro-pyrido[3,2-d]pyrimidin-2-yl]amino}-3-methoxy-N-(1-methylpiperidin-4-yl)benzamide

### Step 1: 2-chloro-7-(cyclopropylmethoxy)-5-methyl-8-(2-thienyl)pyrido[3,2-d]pyrimidin-6(5H)-one

8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) (0.100 g), 2-thienylboronic acid (0.078 g) and triethylamine (0.242 ml) were dissolved in dry dioxane (2 ml). Nitrogen was bubbled through the mixture for 20 min., then [1-1'-bis(diphenylphosphino)-ferrocene]dichloro-palladium(II), complex with dichloromethane (0.059 g), was added. The mixture was heated to 120°C for 2 hours in a closed vessel, under microwave irradiation. After cooling, the solution was diluted with dichloro-methane and washed with water and saturated solution of NaHCO₃ in water. The organic phase was dried over Na₂SO₄ and the solvent was evaporated. The crude was purified on silica gel (eluent: petroleum ether/dichloro-methane/methanol 85/18/3 (v/v)) to give the title compound.
¹H NMR (CDCl₃) δ (ppm): 8.73 (s, 1 H); 8.48 (dd, 1 H); 7.64 (dd, 1 H); 7.25 (dd, 1 H); 4.33 (d, 2 H); 3.80 (s, 3 H); 1.21 - 1.44 (m, 1 H); 0.52 - 0.65 (m, 2 H); 0.26 - 0.39 (m, 2 H).

### Step 2: 4-{[7-(cyclopropylmethoxy)-5-methyl-6-oxo-8-(2-thienyl)-5,6-dihydropyrido[3,2-d]pyrimidin-2-yl]amino}-3-methoxy-N-(1-methylpiperidin-4-yl)benzamide

4-amino-3-methoxy-N-(1-methylpiperidin-4-yl)benzamide (example 1, step 6) (0.048 g) was added to a solution of the product of step 1 (0.064 g), (+/-) 2,2'-bis-(diphenylphos-phino)-1,1'-binaphthyl (0.028 g), tris(dibenzylideneacetone)dipalladium (0) (0.042 g) and Cs₂CO₃ (0.235 g) in dry toluene (3 ml), under nitrogen atmosphere. The mixture was heated to reflux for 3 hours and then cooled to room temperature. Dichloromethane was added and the mixture was washed with water and a saturated solution of NaCl. The organic phase was dried over Na₂SO₄ and the solvent was evaporated. The residue was purified on silica gel (eluent: dichloromethane/methanol/32% NH₄OH 95/5/0.5 (v/v)) and further triturated in diethyl ether to give the title compound as yellow powder.
¹H NMR (CDCl₃) δ (ppm): 8.66 (s, 1 H); 8.50 (d, 1 H); 8.05 (dd, 1 H); 7.96 (s, 1 H); 7.64 (dd, 1 H); 7.45 (d, 1 H); 7.27 (dd, 1 H); 7.14 (dd, 1 H); 5.92 (d, 1 H); 4.23 (d, 2 H); 4.01 (s, 3 H); 3.88 - 4.14 (m, 1 H); 3.80 (s, 3 H); 2.81 - 2.91 (m, 2 H); 2.34 (s, 3 H); 2.14 - 2.27 (m, 2 H); 2.02 - 2.14 (m, 2 H); 1.52 - 1.72 (m, 2 H); 1.13 - 1.39 (m, 1 H); 0.42 - 0.67 (m, 2 H); 0.19 - 0.40 (m, 2 H).
MS (ESI Pos, 3.2 kV, 25 V, 350°C): 575.25 m/z (MH⁺).

### Example 54: 4-{[7-(cyclopropylmethoxy)-8-(3-fluorophenyl)-5-methyl-6-oxo-5,6-dihydropyrido[3,2-d]pyrimidin-2-yl]amino}-3-methoxy-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methyl-pyrido[3,2-d]pyrimidin-6(5H)-one and using 3-fluorophenylboronic acid instead of 3-thienylboronic acid in step 1.
¹H NMR (CDCl₃) δ (ppm): 8.64 (s, 1 H), 8.15 (d, 1 H), 7.95 (s, 1 H), 7.46 - 7.59 (m, 1 H), 7.38 (d, 1 H), 7.29 - 7.34 (m, 2 H), 7.18 - 7.27 (m, 1 H), 6.96 (dd, 1 H), 5.89 (d, 1 H), 4.12 (d, 2 H), 3.98 - 4.08 (m, 1 H), 3.97 (s, 3 H), 3.81 (s, 3 H), 2.75 - 2.93 (m, 2 H), 2.34 (s, 3 H), 2.20 (td, 2 H), 2.00 - 2.14 (m, 2 H), 1.52 - 1.62 (m, 2 H), 0.95 - 1.12 (m, 1 H), 0.39 - 0.52 (m, 2 H), 0.10 - 0.21 (m, 2 H).
¹³C NMR (CDCl₃) δ (ppm): 166.69 (s, 1 C); 162.36 (d, 1 C); 157.42 (s, 1 C); 154.85 (s, 1 C); 151.72 (s, 1 C); 147.06 (s, 1 C); 144.59 (s, 1 C); 144.31 (s, 1 C); 134.01 (d, 1 C); 134.02 (d, 1 C); 132.20 (s, 1 C); 129.24 (d, 1 C); 127.29 (s, 1 C); 126.61 (d, 1 C); 125.05 (s, 1 C); 118.82 (s, 1 C); 117.90 (d, 1 C); 116.15 (s, 1 C); 114.95 (d, 1 C); 108.84 (s, 1 C); 77.77 (s, 1 C); 55.92 (s, 1 C); 54.53 (s, 2 C); 46.49 (s, 1 C); 46.20 (s, 1 C); 32.34 (s, 2 C); 29.01 (s, 1 C); 10.91 (s, 1 C); 3.14 (s, 2 C).
MS (ESI Pos, 3.2 kV, 25 V, 350°C): 587.30 m/z (MH⁺).

### Example 55: 4-{[7-(cyclopropylmethoxy)-5-methyl-8-(4-methyl-2-thienyl)-6-oxo-5,6-dihydropyrido[3,2-d]pyrimidin-2-yl]amino}-3-methoxy-N-(1-methylpiperidin-4-yl)benzamide

The title compound was prepared analogously to example 53 using 4-methylthiophen-2-ylboronic acid instead of 2-thienylboronic acid in step 1.
¹H NMR (CDCl₃) δ (ppm): 8.64 (s, 1 H); 8.53 (d, 1 H); 7.94 (s, 1 H); 7.88 (d, 1 H); 7.44 (d, 1 H); 7.20 (s, 1 H); 7.16 (dd, 1 H); 5.89 (d, 1 H); 4.21 (d, 2 H); 4.00 (s, 3 H); 3.87 - 4.11 (m, 1 H); 3.78 (s, 3 H); 2.73 - 2.95 (m, 2 H); 2.38 (s, 3 H); 2.33 (s, 3 H); 2.13 - 2.27 (m, 2 H); 1.98 - 2.13 (m, 2 H); 1.48 - 1.71 (m, 2 H); 1.23 - 1.37 (m, 1 H); 0.50 - 0.62 (m, 2 H); 0.20 - 0.35 (m, 2 H).
¹³C NMR (CDCl₃) δ (ppm): 166.62 (s, 1 C); 157.22 (s, 1 C); 154.74 (s, 1 C); 150.63 (s, 1 C); 147.36 (s, 1 C); 144.54 (s, 1 C); 144.05 (s, 1 C); 136.67 (s, 1 C); 134.60 (s, 1 C); 132.30 (s, 1 C); 130.60 (s, 1 C); 128.09 (s, 1 C); 127.41 (s, 1 C); 125.05 (s, 1 C); 124.31 (s, 1 C); 118.92 (s, 1 C); 116.42 (s, 1 C); 109.07 (s, 1 C); 77.72 (s, 1 C); 55.98 (s, 1 C); 54.53 (s, 2 C); 46.49 (s, 1 C); 46.25 (s, 1 C); 32.45 (s, 2 C); 28.93 (s, 1 C); 15.78 (s, 1 C); 11.07 (s, 1 C); 3.44 (s, 2 C).
MS (ESI Pos, 3.2 kV, 25 V, 350°C): 589.26 m/z (MH⁺).

### Example 56: 4-({7-(cyclopropyloxy)-5-methyl-6-oxo-8-[(1E)-prop-1-en-1-yl]-5,6-dihydropyrido[3,2-d]pyrimidin-2-yl}amino)-3-methoxy-N-(1-methylpiperidin-4-yl)benzamide

### Step 1: 8-allyl-2-chloro-7-(cyclopropyloxy)-5-methylpyrido[3,2-d]pyrimidin-6(5H)-one

To a solution of cyclopropylamine (0.116 ml) and K₂CO₃ (0.344 g) in dimethylformamide (1 ml) at 0°C, a solution of 4-bromobenzenediazonium tetrafluoroborate (0.494 g) in dimethyl-formamide (3 ml) was added and the mixture was stirred at 0°C for 2 hours under nitrogen atmosphere. The reaction mixture was poured into ice and extracted with petroleum ether. The organic phase was dried over Na₂SO₄ and concentrated to dryness at 20°C. The solid obtained was dissolved in dichloromethane (10 ml) and reacted with 8-allyl-2-chloro-7-hydroxy-5-methylpyrido[3,2-d]pyrimidin-6(5H)-one (example 2, step 2) (0.055 g). The reaction mixture was stirred overnight at room temperature. The reaction mixture was washed with water and the organic phase was separated and concentrated. The residue was filtered through a plug of silica gel (eluent: dichloromethane/ethyl acetate 20/1 (v/v)) to give the title compound as light brown solid which was used for the next step without further purification.

### Step 2: 2-chloro-7-(cyclopropyloxy)-5-methyl-8-[(1E)-prop-1-en-1-yl]pyrido[3,2-d]pyri-midin-6(5H)-one

The crude product of step 1 (0.200 g) and 1,8-diazabicyclo [5.4.0] undec-7-ene (0.103 g) were mixed in dioxane (10 ml) and the mixture was heated to 60°C for 6 hours. The reaction mixture was partitioned between ethyl acetate and water; the organic phase was separated and concentrated to dryness. The residue was filtered through a plug of silica gel (eluent: dichloromethane/ethyl acetate 20/1.5 (v/v)) to give the title compound as light brown solid which was used for the next step without further purification.

### Step 3: 4-({7-(cyclopropyloxy)-5-methyl-6-oxo-8-[(1E)-prop-1-en-1-yl]-5,6-dihydropyrido[3,2-d]pyrimidin-2-yl}amino)-3-methoxy-N-(1-methylpiperidin-4-yl)benzamide

Tris(dibenzylideneacetone)dipalladium (0) (0.068 g), the crude product of step 2, 4-amino-3-methoxy-N-(1-methylpiperidin-4-yl)benzamide (example 1, step 6) (0.180 g), Cs₂CO₃ (0.808 g) and (+/-) 2,2'-bis-(diphenylphosphino)-1,1'-binaphthyl (0.046 g) were mixed in dry toluene (7 ml, degassed) and the mixture was stirred for 3 hours at reflux, under nitrogen atmosphere. The reaction mixture was partitioned between water and dichloromethane; the organic layer was separated, dried over Na₂SO₄ and concentrated. The residue was purified on silica gel (eluent: dichloromethane/methanol/32% NH₄OH 20/1/1.5 (v/v)) and further triturated in a mixture of methanol/diethyl ether 2/8 (v/v) to give the title compound.
¹H NMR (CDCl₃) δ (ppm): 8.66 (d, 1 H); 8.61 (s, 1 H); 7.96 (s, 1 H); 7.47 (d, 1 H); 7.29 - 7.34 (m, 1 H); 6.96 - 7.11 (m, 1 H); 6.77 - 6.92 (m, 1 H); 5.96 (d, 1 H); 4.81 - 4.93 (m, 1 H); 4.03 (s, 3 H); 3.93 - 4.13 (m, 1 H); 3.76 (s, 3 H); 2.85 (dt, 2 H); 2.33 (s, 3 H); 2.19 (td, 2 H); 2.06 - 2.13 (m, 2 H); 2.06 (dd, 3 H); 1.52 - 1.69 (m, 2 H); 0.77 - 0.92 (m, 2 H); 0.53 - 0.66 (m, 2 H).
¹³C NMR (CDCl₃) δ (ppm): 166.63 (s, 1 C); 157.24 (s, 1 C); 154.71 (s, 1 C); 150.78 (s, 1 C); 147.37 (s, 1 C); 144.40 (s, 1 C); 144.24 (s, 1 C); 138.01 (s, 1 C); 132.57 (s, 1 C); 130.38 (s, 1 C); 127.31 (s, 1 C); 125.18 (s, 1 C); 120.17 (s, 1 C); 118.93 (s, 1 C); 116.04 (s, 1 C); 109.21 (s, 1 C); 56.00 (s, 1 C); 55.89 (s, 1 C); 54.54 (s, 2 C); 46.55 (s, 1 C); 46.11 (s, 1 C); 32.31 (s, 2 C); 28.87 (s, 1 C); 20.39 (s, 1 C); 6.07 (s, 2 C).
MS (ESI Pos, 3.2 kV, 25 V, 350°C): 519.22 m/z (MH⁺).

### Example 57: 4-{[8-cyclopent-1-en-1-yl-7-(cyclopropylmethoxy)-5-methyl-6-oxo-5,6-dihydropyrido[3,2-d]pyrimidin-2-yl]amino}-3-methoxy-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methyl-pyrido[3,2-d]pyrimidin-6(5H)-one and using cyclopentenylboronic acid instead of 3-thienylboronic acid in step 1.
¹H NMR (CDCl₃) δ (ppm): 8.72 (d, 1 H); 8.58 (s, 1 H); 7.97 (s, 1 H); 7.43 (d, 1 H); 7.23 (dd, 1 H); 6.06 - 6.14 (m, 1 H); 5.91 (d, 1 H); 4.15 (d, 2 H); 4.00 (s, 3 H); 3.93 - 4.10 (m, 1 H); 3.75 (s, 3 H); 2.78 - 2.94 (m, 4 H); 2.64 - 2.78 (m, 2 H); 2.32 (s, 3 H); 1.98 - 2.26 (m, 6 H); 1.62 - 1.71 (m, 2 H); 1.16 - 1.35 (m, 1 H); 0.54 - 0.65 (m, 2 H); 0.26 - 0.37 (m, 2 H). ¹³C NMR (CDCl₃) δ (ppm): 166.68 (s, 1 C); 157.37 (s, 1 C); 154.86 (s, 1 C); 151.36 (s, 1 C); 147.17 (s, 1 C); 144.84 (s, 1 C); 144.32 (s, 1 C); 134.41 (s, 1 C); 134.20 (s, 1 C); 133.61 (s, 1 C); 132.72 (s, 1 C); 127.12 (s, 1 C); 125.00 (s, 1 C); 119.00 (s, 1 C); 116.03 (s, 1 C); 108.98 (s, 1 C); 77.63 (s, 1 C); 55.98 (s, 1 C); 54.58 (s, 2 C); 46.60 (s, 1 C); 46.16 (s, 1 C); 36.44 (s, 1 C); 33.85 (s, 1 C); 32.36 (s, 2 C); 28.83 (s, 1 C); 24.17 (s, 1 C); 11.08 (s, 1 C); 3.22 (s, 2 C).
MS (ESI Pos, 3.2 kV, 25 V, 350°C): 559.29 m/z (MH⁺).

### Example 58: 4-{[7-(cyclopropyloxy)-5-methyl-6-oxo-8-(3-thienyl)-5,6-dihydropyri-do[3,2-d]pyrimidin-2-yl]amino}-3-methoxy-N-(1-methylpiperidin-4-yl)benzamide

### Step 1: 2-chloro-7-hydroxy-5-methyl-8-(3-thienyl)pyrido[3,2-d]pyrimidin-6(5H)-one

Palladium(II) acetate (0.030 g) was added to a stirred mixture of 8-bromo-2-chloro-7-methoxy-5-methylpyrido[3,2-d]pyrimidin-6(5H)-one (0.200 g), 3-thienylboronic acid (0.084 g) and KF (0.114 g) in degassed methanol (20 ml), at room temperature, under nitrogen atmosphere. The mixture was heated to reflux. After 30 minutes 3-thienylboronic acid (0.084 g), KF (0.114 g) and palladium(II) acetate (0.030 g) were added. After 30 minutes, additional 3-thienyl-boronic acid (0.084 g), KF (0.114 g) and palladium(II) acetate (0.030 g) were added. After 30 minutes the mixture was cooled to room temperature, insoluble parts were filtered off and the solution was diluted with dichloro-methane and washed with 1 N K₂CO₃. The organic phase was dried over Na₂SO₄ and concentrated. The residue was purified on silica gel (eluent: ethyl acetate/petroleum ether 2/3 (v/v)) to give 0.134 g of a white solid.
The solid was heated to reflux in a mixture of dioxane (6 ml) and 37% HCI (6 ml) for 2 hours, kept at room temperature for 20 hours and then heated to reflux for another 2 hours. After cooling to room temperature, the mixture was diluted with dichloromethane and washed with water. The aqueous phase was extracted with dichloromethane, the combined organic layers were dried over Na₂SO₄ and concentrated to give the title compound.
¹H NMR (DMSO-d6) δ (ppm): 11.12 (br. s., 1 H); 8.99 (s, 1 H); 7.92 (dd, 1 H); 7.60 (dd, 1 H); 7.53 (dd, 1 H); 3.79 (s, 3 H).

### Step 2: 2-chloro-7-(cyclopropyloxy)-5-methyl-8-(3-thienyl)pyrido[3,2-d]pyrimidin-6(5H)-one

To a solution of cyclopropylamine (0.580 ml) and K₂CO₃ (1.72 g) in dimethylformamide (5 ml) at 0°C, a solution of 4-bromobenzenediazonium tetrafluoroborate (2.47 g) was added and the mixture was stirred at 0°C for 2 hours under nitrogen atmosphere. The reaction mixture was poured into ice and extracted with petroleum ether. The organic phase was separated, dried over Na₂SO₄ and concentrated to dryness at 20°C. The solid obtained was dissolved in dichloromethane (50 ml) and reacted with 2-chloro-7-hydroxy-5-methyl-8-(3-thienyl)pyri-do[3,2-d]pyrimidin-6(5H)-one (product of step 1) (0.250 g). The reaction mixture was stirred at room temperature for 2 days. The reaction mixture was washed with water, the organic phase was separated and concentrated. The residue was filtered through a plug of silica gel (eluent: dichloromethane/ethyl acetate 20/1 (v/v)) and afterwards purified by preparative HPLC (acetonitrile/water/trifluoroacetic acid 200/1800/1 (v/v) / acetonitrile/water/ trifluoroacetic acid 1800/200/1 (v/v), elution gradient 95/5 to 0/100 to 95/5 (v/v) to give the title compound together with about 25% of the corresponding allyloxy derivative as indicated by ¹H NMR shifts.
¹H NMR (CDCl₃) δ (ppm): 8.74 (s, 1 H); 7.84 (dd, 1 H); 7.45 - 7.49 (m, 1 H); 7.38 - 7.44 (m, 1 H); 4.59 - 4.69 (m, 1 H); 3.83 (s, 3 H); 0.37 - 0.67 (m, 4 H).
NMR shifts refering to the corresponding allyloxy derivative: ¹H NMR (CDCl₃) δ (ppm): 8.72 (s, 1 H); 7.93 (dd, 1 H); 7.56 (dd, 1 H); 7.42 (dd, 1 H); 5.87 (dddd, 1 H); 5.25 (dq, 1 H); 5.17 (dq, 1 H); 4.80 (ddd, 2 H); 3.81 (s, 3 H).

### Step 3: 4-{[7-(cyclopropyloxy)-5-methyl-6-oxo-8-(3-thienyl)-5,6-dihydropyrido[3,2-d]pyrimidin-2-yl]amino}-3-methoxy-N-(1-methylpiperidin-4-yl)benzamide

Tris(dibenzylideneacetone)dipalladium (0) (0.049 g), the crude product of step 1 (0.150 g), 4-amino-3-methoxy-N-(1-methylpiperidin-4-yl)benzamide (example 1, step 6) (0.130 g), Cs₂CO₃ (0.586 g) and (+/-) 2,2'-bis-(diphenylphosphino)-1,1'-binaphthyl (0.034 g) were mixed in dry toluene (6 ml, degassed) and the mixture was stirred for 3 hours at reflux, under nitrogen atmosphere. Tris(dibenzylideneacetone)dipalladium (0) (0.049 g) and (+/-) 2,2'-bis-(diphenyl-phosphino)-1,1'-binaphthyl (0.034 g) were added and the mixture was heated to reflux for 2 hours. The reaction mixture was partitioned between water and dichloromethane; the organic layer was separated, dried over Na₂SO₄ and concentrated. The residue was purified on silica gel (eluent: dichloromethane/methanol/32% NH₄OH 20/1/0.15 (v/v)) and further triturated in a mixture methanol/diethyl ether 2/8 (v/v) to give the title compound.
¹H NMR (CDCl₃) δ (ppm): 8.65 (s, 1 H), 8.35 (d, 1 H), 7.97 (s, 1 H), 7.69 (dd, 1 H), 7.47 (dd, 1 H), 7.41 (d, 1 H), 7.39 (dd, 1 H), 7.06 (dd, 1 H), 5.89 (d, 1 H), 4.53 - 4.67 (m, 1 H), 3.98 (s, 3 H), 3.89 - 4.11 (m, 1 H), 3.82 (s, 3 H), 2.69 - 2.98 (m, 2 H), 2.32 (s, 3 H), 2.18 (ddd, 2 H), 2.00 - 2.13 (m, 2 H), 1.48 - 1.73 (m, 2 H), 0.49 - 0.60 (m, 2 H), 0.39 - 0.49 (m, 2 H).
¹³C NMR (CDCl₃) δ (ppm): 166.65 (s, 1 C), 157.37 (s, 1 C), 154.90 (s, 1 C), 151.61 (s, 1 C), 147.12 (s, 1 C), 144.67 (s, 1 C), 144.33 (s, 1 C), 132.31 (s, 1 C), 130.60 (s, 1 C), 130.57 (s, 1 C), 130.18 (s, 1 C), 127.75 (s, 1 C), 127.23 (s, 1 C), 125.27 (s, 1 C), 123.92 (s, 1 C), 118.93 (s, 1 C), 116.24 (s, 1 C), 108.98 (s, 1 C), 55.99 (s, 1 C), 55.96 (s, 1 C), 54.56 (s, 2 C), 46.54 (s, 1 C), 46.13 (s, 1 C), 32.31 (s, 2 C), 29.05 (s, 1 C), 5.94 (s, 2 C). MS (ESI Pos, 3.2 kV, 25 V, 350°C): 561.06 m/z (MH⁺).

### Example 59: 4-{[7-(cyclopropylmethoxy)-8-(2-fluorophenyl)-5-methyl-6-oxo-5,6-dihydropyrido[3,2-d]pyrimidin-2-yl]amino}-3-methoxy-N-(1-methylpiperidin-4-yl)benzamide

The title compound was prepared analogously to example 53 using (2-fluorophenyl)boronic acid instead of 2-thienylboronic acid in step 1.
¹H NMR (CDCl₃) δ (ppm): 8.65 (s, 1 H); 8.06 (d, 1 H); 7.96 (s, 1 H); 7.51 - 7.63 (m, 1 H); 7.41 - 7.49 (m, 1 H); 7.31 - 7.40 (m, 2 H); 7.25 - 7.29 (m, 1 H); 6.91 (dd, 1 H); 5.85 (d, 1 H); 4.21 - 4.31 (m, 1 H); 4.09 - 4.19 (m, 1 H); 3.99 - 4.09 (m, 1 H); 3.97 (s, 3 H); 3.83 (s, 3 H); 2.87 (dt, 2 H); 2.35 (s, 3 H); 2.20 (td, 2 H); 2.03 - 2.14 (m, 2 H); 1.52 - 1.63 (m, 2 H); 0.98 - 1.17 (m, 1 H); 0.41 - 0.51 (m, 2 H); 0.13 - 0.21 (m, 2 H).
¹³C NMR (CDCl₃) δ (ppm): 166.67 (s, 1 C); 160.01 (d, 1 C); 157.33 (s, 1 C); 154.97 (s, 1 C); 152.63 (s, 1 C); 147.06 (s, 1 C); 144.50 (s, 1 C); 144.40 (s, 1 C); 132.45 (s, 1 C); 132.23 (d, 1 C); 130.15 (d, 1 C); 129.97 (s, 1 C); 127.02 (s, 1 C); 124.91 (s, 1 C); 123.73 (d, 1 C); 120.35 (d, 1 C); 118.90 (s, 1 C); 116.01 (s, 1 C); 115.44 (d, 1 C); 108.67 (s, 1 C); 77.70 (s, 1 C); 55.93 (s, 1 C); 54.58 (s, 2 C); 46.54 (s, 1 C); 46.24 (s, 1 C); 32.46 (s, 2 C); 29.03 (s, 1 C); 10.88 (s, 1 C); 3.06 (s, 1 C); 3.00 (s, 1 C).
MS (ESI Pos, 3.2 kV, 25 V, 350°C): 587.30 m/z (MH⁺).

### Example 60: 4-{[7-(cyclopropylmethoxy)-5-methyl-8-(5-methyl-3-thienyl)-6-oxo-5,6-dihydropyrido[3,2-d]pyrimidin-2-yl]amino}-3-methoxy-N-(1-methylpiperidin-4-yl)benzamide

### Step 1: 4,4,5,5-tetramethyl-2-(5-methyl-3-thienyl)-1,3,2-dioxaborolane

Dichlorobis(triphenylphosphine)-palladium(II) (0.089 g) was dissolved in dry dioxane (8 ml) under nitrogen atmosphere; 4-bromo-2-methylthiophene (0.158 ml), 4,4,5,5-tetramethyl-1,3,2 dioxaborolane (0.511 ml) and triethylamine (0.588 ml) were added and the mixture was heated to reflux for 3 hours. After cooling to room temperature dichloro-methane was added and the mixture was washed with water and a saturated solution of NaHCO₃ in water. The organic phase was dried over Na₂SO₄ and the solvent was evaporated. The residue was purified on silica gel (eluent: ethyl acetate/petroleum ether 2/8 (v/v)) to give the title compound as pale brown oil.
¹H NMR (CDCl₃) δ (ppm): 7.68 (d, 1 H); 7.05 (quin., 1 H); 2.50 (d, 3 H); 1.33 (s, 12 H).

### Step 2: 2-chloro-7-(cyclopropylmethoxy)-5-methyl-8-(5-methyl-3-thienyl)pyrido[3,2-d]pyri-midin-6(5H)-one

Tetrakis(triphenylphosphine)palladium(0) (0.007 g) was added to a mixture of 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) (0.042 g), 4,4,5,5-tetramethyl-2-(5-methyl-3-thienyl)-1,3,2-dioxa-borolane (product of step 1) (0.083 g) and KF (0.023 g) in dry toluene (1 ml). The mixture was stirred for 1 hour at reflux and then for 1 hour at 160°C, under microwave irradiation. Tetrakis(triphenylphosphine)palladium(0) (0.007 g) and KF (0.023 g) were added and the mixture was heated to 160°C for 3 hours. Additional tetrakis(triphenylphosphine)palla-dium(0) (0.007 g) and KF (0.023 g) were added and the mixture was heated to 160°C for 2 hours. After cooling, the mixture was diluted with dichloro-methane and washed with water. The organic phase was dried over Na₂SO₄ and the solvent was evaporated. The residue was purified on silica gel (eluent: ethyl acetate/petroleum ether 3/7) to give the title compound.
¹H NMR (CDCl₃) δ (ppm): 8.71 (s, 1 H); 7.73 (d, 1 H); 7.21 - 7.27 (m, 1 H); 4.10 (d, 2 H); 3.80 (s, 3 H); 2.58 (d, 3 H); 1.14 (tt, 1 H); 0.38 - 0.63 (m, 2 H); 0.14 - 0.28 (m, 2 H).

### Step 3: 4-{[7-(cyclopropylmethoxy)-5-methyl-8-(5-methyl-3-thienyl)-6-oxo-5,6-dihydropyrido[3,2-d]pyrimidin-2-yl]amino}-3-methoxy-N-(1-methylpiperidin-4-yl)benzamide

4-amino-3-methoxy-N-(1-methylpiperidin-4-yl)benzamide (example 1, step 6) (0.038 g) was added to a mixture of the product of step 2 (0.053 g), tris(dibenzylideneacetone)dipalladium (0) (0.034 g), (+/-) 2,2'-bis-(diphenylphosphino)-1,1'-binaphthyl (0.022 g) and Cs₂CO₃ (0.190 g) in dry toluene (5 ml). The mixture was stirred at reflux for 3 hour. After cooling, the mixture was diluted with dichloromethane and washed with water and a saturated solution of NaCl. The organic phase was dried over Na₂SO₄ and the solvent was evaporated. The residue was purified on silica gel (dichloromethane/methanol/32% NH₄OH 95/5/0.5 (v/v)) and further triturated in water and then in diethyl ether to give the title compound as yellow powder.
¹H NMR (CDCl₃) δ (ppm): 8.64 (s, 1 H); 8.49 (d, 1 H); 7.97 (s, 1 H); 7.57 (d, 1 H); 7.42 (d, 1 H); 7.16 - 7.20 (m, 1 H); 7.13 (dd, 1 H); 5.89 (d, 1 H); 4.07 (d, 2 H); 4.00 (s, 3 H); 4.05 (br. s., 1 H); 3.80 (s, 3 H); 2.76 - 2.90 (m, 2 H); 2.62 (d, 3 H); 2.34 (s, 3 H); 2.14 - 2.30 (m, 2 H); 1.96 - 2.14 (m, 2 H); 1.58 - 1.76 (m, 2 H); 1.00 - 1.21 (m, 1 H); 0.45 - 0.58 (m, 2 H); 0.14 - 0.29 (m, 2 H).
MS (ESI Pos, 3.2 kV, 25 V, 350°C): 589.06 m/z (MH⁺).

### Example 61: 4-{[7-(cyclopropylmethoxy)-5-methyl-8-(3-methylphenyl)-6-oxo-5,6-dihydropyrido[3,2-d]pyrimidin-2-yl]amino}-3-methoxy-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methyl-pyrido[3,2-d]pyrimidin-6(5H)-one and using (3-methylphenyl)boronic acid instead of 3-thienylboronic acid in step 1.
¹H NMR (CDCl₃) δ (ppm): 8.62 (s, 1 H), 8.15 (d, 1 H), 7.94 (s, 1 H), 7.39 - 7.51 (m, 1 H), 7.29 - 7.38 (m, 4 H), 6.91 (dd, 1 H), 5.82 (d, 1 H), 4.05 (d, 2 H), 3.97 - 4.03 (m, 1 H), 3.96 (s, 3 H), 3.81 (s, 3 H), 2.84 (dt, 2 H), 2.45 (s, 3 H), 2.33 (s, 3 H), 2.20 (td, 2 H), 2.08 (s, 2 H), 1.60 - 1.70 (m, 2 H), 0.93 - 1.20 (m, 1 H), 0.37 - 0.51 (m, 2 H), 0.02 - 0.19 (m, 2 H). ¹³C NMR (75 MHz, CDCl₃) δ (ppm): 166.69 (s, 1 C), 157.67 (s, 1 C), 154.83 (s, 1 C), 151.60 (s, 1 C), 147.04 (s, 1 C), 144.89 (s, 1 C), 144.42 (s, 1 C), 137.25 (s, 1 C), 135.68 (s, 1 C), 132.45 (s, 1 C), 131.81 (s, 1 C), 131.22 (s, 1 C), 128.78 (s, 1 C), 127.70 (s, 1 C), 127.69 (s, 1 C), 127.01 (s, 1 C), 125.15 (s, 1 C), 119.00 (s, 1 C), 116.19 (s, 1 C), 108.67 (s, 1 C), 77.59 (s, 1 C), 55.91 (s, 1 C), 54.52 (s, 2 C), 46.41 (s, 1 C), 46.21 (s, 1 C), 32.39 (s, 2 C), 28.96 (s, 1 C), 21.55 (s, 1 C), 10.89 (s, 1 C), 3.11 (s, 2 C). MS (ESI Pos, 3.2 kV, 25 V, 350°C): 583.2 m/z (MH⁺).

### Example 62: 4-{[7-(cyclopropylmethoxy)-5-methyl-8-(4-methylphenyl)-6-oxo-5,6-dihydropyrido[3,2-d]pyrimidin-2-yl]amino}-3-methoxy-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methyl-pyrido[3,2-d]pyrimidin-6(5H)-one and using (4-methylphenyl)boronic acid instead of 3-thienylboronic acid in step 1.
¹H NMR (CDCl₃) δ (ppm): 8.61 (s, 1 H); 8.14 (d, 1 H); 7.92 (s, 1 H); 7.32 - 7.45 (m, 5 H); 6.87 (dd, 1 H); 5.84 (d, 1 H); 4.05 (d, 2 H); 3.97 - 4.03 (m, 1 H); 3.95 (s, 3 H); 3.80 (s, 3 H); 2.83 (dt, 2 H); 2.53 (s, 3 H); 2.33 (s, 3 H); 2.20 (td, 2 H); 1.99 - 2.12 (m, 2 H); 1.49 - 1.71 (m, 2 H); 0.96 - 1.15 (m, 1 H); 0.39 - 0.49 (m, 2 H); 0.10 - 0.19 (m, 2 H).
¹³C NMR (75 MHz, CDCl₃) δ (ppm): 166.62 (s, 1 C); 157.71 (s, 1 C); 154.81 (s, 1 C); 151.58 (s, 1 C); 147.10 (s, 1 C); 145.02 (s, 1 C); 144.37 (s, 1 C); 137.65 (s, 1 C); 135.56 (s, 1 C); 132.48 (s, 1 C); 130.55 (s, 2 C); 129.02 (s, 1 C); 128.50 (s, 1 C); 126.91 (s, 1 C); 125.19 (s, 1 C); 118.80 (s, 1 C); 116.40 (s, 2 C); 108.74 (s, 1 C); 77.57 (s, 1 C); 55.91 (s, 1 C); 54.47 (s, 2 C); 46.27 (s, 1 C); 46.15 (s, 1 C); 32.34 (s, 2 C); 28.95 (s, 1 C); 21.52 (s, 1 C); 10.92 (s, 1 C); 3.12 (s, 2 C). MS (ESI Pos, 3.2 kV, 25 V, 350°C): 583.26 m/z (MH⁺).

### Example 63: 4-{[7-(cyclopropylmethoxy)-5-methyl-6-oxo-8-(1,3-thiazol-4-yl)-5,6-dihydropyrido[3,2-d]pyrimidin-2-yl]amino}-3-methoxy-N-(1-methylpiperidin-4-yl)benzamide

The title compound was prepared analogously to example 44 starting from 8-bromo-2-chloro-7-(cyclopropylmethoxy)-5-methylpyrido[3,2-d]pyrimidin-6(5H)-one (prepared analogously to example 3 using bromomethyl-cyclopropane instead of iodomethane in step 1) instead of 8-bromo-2-chloro-7-ethoxy-5-methylpyrido[3,2-d]pyrimidin-6(5H)-one and using 4-(tributyl-stannyl)-1,3-thiazole instead of2-(tributylstannyl)-1,3-oxazole in step 1.
¹H NMR (CDCl₃) δ (ppm): 9.08 (d, 1 H), 8.63 (s, 1 H), 8.26 (d, 1 H), 7.95 (s, 1 H), 7.73 (d, 1 H), 7.36 (d, 1 H), 7.06 (dd, 1 H), 5.87 (d, 1 H), 4.17 (d, 2 H), 3.96 (s, 3 H), 3.90 - 4.08 (m, 1 H), 3.81 (s, 3 H), 2.78 - 2.94 (m, 2 H), 2.33 (s, 3 H), 2.19 (td, 2 H), 2.00 - 2.13 (m, 2 H), 1.53 - 1.64 (m, 2 H), 1.00 - 1.18 (m, 1 H), 0.41 - 0.52 (m, 2 H), 0.13 - 0.23 (m, 2 H). ¹³C NMR (75 MHz, CDCl₃) δ (ppm): 166.66 (s, 1 C), 157.37 (s, 1 C), 154.98 (s, 1 C), 153.13 (s, 1 C), 151.59 (s, 1 C), 147.13 (s, 1 C), 146.63 (s, 1 C), 144.51 (s, 1 C), 144.46 (s, 1 C), 132.45 (s, 1 C), 129.09 (s, 1 C), 127.13 (s, 1 C), 125.05 (s, 1 C), 120.78 (s, 1 C), 118.94 (s, 1 C), 116.23 (s, 1 C), 108.80 (s, 1 C), 78.13 (s, 1 C), 55.95 (s, 1 C), 54.60 (s, 2 C), 46.60 (s, 1 C), 46.18 (s, 1 C), 32.41 (s, 2 C), 29.07 (s, 1 C), 10.93 (s, 1 C), 3.10 (s, 2 C).
MS (ESI Pos, 3.2 kV, 25 V, 350°C): 576.13 m/z (MH⁺).

### Example 64: 4-({7-(cyclopropylmethoxy)-5-methyl-6-oxo-8-[(1E)-prop-1-en-1-yl]-5,6-dihydropyrido[3,2-d]pyrimidin-2-yl}amino)-3-methyl-N-(1-methylpiperidin-4-yl)benzamide

The title compound was prepared analogously to example 2 using bromomethyl)cyclopropane instead of iodomethane in step 3 and using 4-amino-3-methyl-N-(1-methyl-piperidin-4-yl)benz-amide instead of 4-amino-3-methoxy-N-(1-methylpiperidin-4-yl)benzamide in step 4. 4-amino-3-methyl-N-(1-methylpiperidin-4-yl)benzamide was prepared analogously to 4-amino-3-methoxy-N-(1-methylpiperidin-4-yl)benzamide (example 1, step 6) using 4-amino-3-methyl-benzoic acid instead of 4-amino-3-methoxybenzoic acid . ¹H NMR (CDCl₃) δ (ppm): 8.58 (s, 1 H), 8.45 (d, 1 H), 7.60 - 7.71 (m, 2 H), 7.29 - 7.36 (m, 1 H), 7.06 (s, 1 H), 6.93 - 7.04 (m, 1 H), 5.90 (d, 1 H), 4.23 (d, 2 H), 3.90 - 4.13 (m, 1 H), 3.74 (s, 3 H), 2.73 - 2.94 (m, 2 H), 2.43 (s, 3 H), 2.33 (s, 3 H), 2.13 - 2.26 (m, 2 H), 2.00 - 2.13 (m, 5 H), 1.61 - 1.69 (m, 2 H), 1.18 - 1.40 (m, 1 H), 0.56 - 0.66 (m, 2 H), 0.29 - 0.40 (m, 2 H).
¹³C NMR (75 MHz, CDCl₃) δ (ppm): 166.58 (s, 1 C), 157.41 (s, 1 C), 154.97 (s, 1 C), 150.95 (s, 1 C), 144.59 (s, 1 C), 144.30 (s, 1 C), 140.94 (s, 1 C), 138.12 (s, 1 C), 129.95 (s, 1 C), 129.23 (s, 1 C), 128.21 (s, 1 C), 126.03 (s, 1 C), 125.37 (s, 1 C), 125.19 (s, 1 C), 120.19 (s, 1 C), 118.51 (s, 1 C), 77.53 (s, 1 C), 54.56 (s, 1 C), 46.50 (s, 1 C), 46.22 (s, 2 C), 32.47 (s, 2 C), 28.78 (s, 1 C), 20.48 (s, 1 C), 18.05 (s, 1 C), 11.27 (s, 1 C), 3.38 (s, 2 C).
MS (ESI Pos, 3.2 kV, 25 V, 350°C): 517.12 m/z (MH⁺).

### Example 65: 4-({7-(cyclopropylmethoxy)-5-methyl-6-oxo-8-[(1E)-prop-1-en-1-yl]-5,6-dihydropyrido[3,2-d]pyrimidin-2-yl}amino)-3-ethyl-N-(1-methylpiperidin-4-yl)benzamide

The title compound was prepared analogously to example 2 using (bromomethyl)cyclopropane instead of iodomethane in step 3 and using 4-amino-3-ethyl-N-(1-methylpiperi-din-4-yl)benzamide instead of 4-amino-3-methoxy-N-(1-methylpiperidin-4-yl)benzamide step 4. 4-amino-3-ethyl-N-(1-methylpiperidin-4-yl)benzamide was prepared analogously to 4-amino-3-methoxy-N-(1-methylpiperidin-4-yl)benzamide (example 1, step 6) using 4-amino-3-ethyl-benzoic acid instead of 4-amino-3-methoxybenzoic acid .
¹H NMR (CDCl₃) δ (ppm): 8.57 (s, 1 H), 8.42 (d, 1 H), 7.70 (d, 1 H), 7.64 (dd, 1 H), 7.29 (dq, 1 H), 7.10 (s, 1 H), 7.00 (dq, 1 H), 5.91 (d, 1 H), 4.23 (d, 2 H), 3.86 - 4.13 (m, 1 H), 3.73 (s, 3 H), 2.76 - 2.93 (m, 2 H), 2.78 (q, 2 H), 2.33 (s, 3 H), 2.20 (td, 2 H), 2.05 (dd, 3 H), 1.96 - 2.14 (m, 2 H), 1.48 - 1.73 (m, 2 H), 1.36 (t, 3 H), 1.23 - 1.41 (m, 1 H), 0.50 - 0.76 (m, 2 H), 0.17 - 0.45 (m, 2 H).
¹³C NMR (75 MHz, CDCl₃) δ (ppm): 166.70 (s, 1 C), 157.40 (s, 1 C), 155.15 (s, 1 C), 150.94 (s, 1 C), 144.60 (s, 1 C), 144.34 (s, 1 C), 140.27 (s, 1 C), 138.12 (s, 1 C), 132.20 (s, 1 C), 129.95 (s, 1 C), 128.65 (s, 1 C), 127.58 (s, 1 C), 125.17 (s, 1 C), 125.01 (s, 1 C), 120.21 (s, 1 C), 119.32 (s, 1 C), 77.52 (s, 1 C), 54.57 (s, 2 C), 46.53 (s, 1 C), 46.24 (s, 1 C), 32.49 (s, 2 C), 28.78 (s, 1 C), 24.51 (s, 1 C), 20.49 (s, 1 C), 13.63 (s, 1 C), 11.27 (s, 1 C), 3.38 (s, 2 C).
MS (ESI Pos, 3.2 kV, 25 V, 350°C): 531.14 m/z (MH⁺).

### Example 66: 4-{[7-(cyclopropylmethoxy)-5-methyl-6-oxo-8-(3-thienyl)-5,6-dihydropyrido[3,2-d]pyrimidin-2-yl]amino}-3-methyl-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methylpyrido[3,2-d]pyrimidin-6(5H)-one and using 4-amino-3-methyl-N-(1-methyl-piperidin-4-yl)benzamide instead of 4-amino-3-methoxy-N-(1-methylpiperidin-4-yl)benzamide in step 2.
¹H NMR (CDCl₃) δ (ppm): 8.62 (s, 1 H), 8.31 (d, 1 H), 7.81 (dd, 1 H), 7.57 - 7.63 (m, 1 H), 7.41 - 7.54 (m, 3 H), 7.08 (s, 1 H), 5.86 (d, 1 H), 4.07 (d, 2 H), 3.92 - 4.04 (m, 1 H), 3.79 (s, 3 H), 2.76 - 2.91 (m, 2 H), 2.38 (s, 3 H), 2.32 (s, 3 H), 2.13 - 2.26 (m, 2 H), 1.99 - 2.12 (m, 2 H), 1.50 - 1.69 (m, 2 H), 0.99 - 1.19 (m, 1 H), 0.41 - 0.52 (m, 2 H), 0.12 - 0.22 (m, 2 H).
MS (ESI Pos, 3.2 kV, 25 V, 350°C): 559.10 m/z (MH⁺).

### Example 67: 3-chloro-4-({7-(cyclopropylmethoxy)-5-methyl-6-oxo-8-[(1E)-prop-1-en-1-yl]-5,6-dihydropyrido[3,2-d]pyrimidin-2-yl}amino)-N-(1-methylpiperidin-4-yl)benzamide

The title compound was prepared analogously to example 2 using (bromomethyl)cyclopropane instead of iodomethane in step 3 and using 4-amino-3-chloro-N-(1-methyl-piperidin-4-yl)benzamide instead of 4-amino-3-methoxy-N-(1-methylpiperidin-4-yl)benzamide step 4. 4-Amino-3-chloro-N-(1-methylpiperidin-4-yl)benzamide was prepared analogously to 4-amino-3-methoxy-N-(1-methylpiperidin-4-yl)benzamide (example 1, step 6) using 4-amino-3-chloro-benzoic acid instead of 4-amino-3-methoxybenzoic acid.
¹H NMR (CDCl₃) δ (ppm): 8.81 (d, 1 H); 8.62 (s, 1 H); 7.86 (d, 1 H); 7.84 (br. s., 1 H); 7.71 (dd, 1 H); 7.28 (dq, 1 H); 7.00 (dq, 1 H); 5.89 (d, 1 H); 4.24 (d, 2 H); 3.90 - 4.12 (m, 1 H); 3.75 (s, 3 H); 2.66 - 2.93 (m, 2 H); 2.33 (s, 3 H); 2.19 (td, 2 H); 2.09 (dd, 3 H); 2.08 (br. s., 2 H); 1.46 - 1.73 (m, 2 H); 1.10 - 1.40 (m, 1 H); 0.48 - 0.74 (m, 2 H); 0.16 - 0.50 (m, 2 H).
¹³C NMR (75 MHz, CDCl₃) δ (ppm): 165.31 (s, 1 C); 157.42 (s, 1 C); 154.13 (s, 1 C); 151.07 (s, 1 C); 144.50 (s, 1 C); 144.19 (s, 1 C); 139.13 (s, 1 C); 138.12 (s, 1 C); 129.87 (s, 1 C); 128.15 (s, 1 C); 128.00 (s, 1 C); 126.16 (s, 1 C); 125.58 (s, 1 C); 121.41 (s, 1 C); 120.13 (s, 1 C); 118.03 (s, 1 C); 77.57 (s, 1 C); 54.53 (s, 2 C); 46.75 (s, 1 C); 46.20 (s, 1 C); 32.41 (s, 2 C); 28.82 (s, 1 C); 20.49 (s, 1 C); 11.29 (s, 1 C); 3.40 (s, 2 C).
MS (ESI Pos, 3.2 KV, 25 V, 350°C): 537.07 m/z (M⁺).

### Example 68: 4-{[7-(cyclopropylmethoxy)-5-methyl-6-oxo-8-pyridin-4-yl-5,6-dihydro-pyrido[3,2-d]pyrimidin-2-yl]amino}-3-methoxy-N-(1-methylpiperidin-4-yl)benzamide

The title compound was prepared analogously to example 34 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-methoxy-5-methylpyrido[3,2-d]pyrimidin-6(5H)-one and using pyridin-4-ylboronic acid instead of pyrimidin-5-ylboronic acid in step 1.
¹H NMR (CDCl₃) δ (ppm): 8.77 - 8.86 (m, 2 H); 8.65 (s, 1 H); 7.90 - 8.05 (m, 2 H); 7.41 - 7.51 (m, 3 H); 6.92 (dd, 1 H); 5.96 (d, 1 H); 4.18 (d, 2 H); 3.89 - 4.07 (m, 4 H); 3.81 (s, 3 H); 2.86 (dt, 2 H); 2.32 (s, 3 H); 2.17 (td, 2 H); 1.98 - 2.11 (m, 2 H); 1.54 - 1.63 (m, 2 H); 0.93 - 1.10 (m, 1 H); 0.40 - 0.50 (m, 2 H); 0.11 - 0.20 (m, 2 H).
¹³C NMR (75 MHz, CDCl₃) δ (ppm): 166.39 (s, 1 C); 157.17 (s, 1 C); 154.96 (s, 1 C); 151.73 (s, 1 C); 149.33 (s, 2 C); 147.19 (s, 1 C); 144.74 (s, 1 C); 143.79 (s, 1 C); 140.64 (s, 1 C); 132.36 (s, 1 C); 132.05 (s, 1 C); 127.42 (s, 1 C); 125.59 (s, 2 C); 125.03 (s, 1 C); 118.27 (s, 1 C); 115.72 (s, 1 C); 109.44 (s, 1 C); 77.97 (s, 1 C); 55.93 (s, 1 C); 54.69 (s, 2 C); 46.92 (s, 1 C); 46.24 (s, 1 C); 32.39 (s, 2 C); 29.10 (s, 1 C); 10.94 (s, 1 C); 3.18 (s, 2 C).
MS (ESI Pos, 3.2 kV, 25 V, 350°C): 570.20 m/z (MH⁺).

### Example 69: 4-{[7-(cyclopropylmethoxy)-5-methyl-6-oxo-8-pyridin-3-yl-5,6-dihydro-pyrido[3,2-d]pyrimidin-2-yl]amino}-3-methoxy-N-(1-methylpiperidin-4-yl)benzamide

The title compound was prepared analogously to example 34 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-methoxy-5-methylpyrido[3,2-d]pyrimidin-6(5H)-one and using pyridin-3-ylboronic acid instead of pyrimidin-5-ylboronic acid in step 1.
¹H NMR (CDCl₃) δ (ppm): 8.79 - 8.86 (m, 1 H); 8.72 - 8.79 (m, 1 H); 8.66 (s, 1 H); 8.09 (d, 1 H); 7.95 (s, 1 H); 7.84 - 7.93 (m, 1 H); 7.45 - 7.54 (m, 1 H); 7.41 (d, 1 H); 6.97 (dd, 1 H); 5.81 - 6.01 (m, 1 H); 4.17 (d, 2 H); 3.97 (s, 3 H); 3.94 - 4.06 (m, 1 H); 3.82 (s, 3 H); 2.73 - 2.93 (m, 2 H); 2.33 (s, 3 H); 2.02 - 2.24 (m, 4 H); 1.62 - 1.74 (m, 2 H); 0.89 - 1.12 (m, 1 H); 0.36 - 0.51 (m, 2 H); 0.06 - 0.20 (m, 2 H).
MS (ESI Pos, 3.2 kV, 25 V, 350°C): 570.20 m/z (MH⁺).

### Example 70: 3-chloro-4-{[7-(cyclopropylmethoxy)-5-methyl-6-oxo-8-(3-thienyl)-5,6-dihydropyrido[3,2-d]pyrimidin-2-yl]amino}-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methylpyrido[3,2-d]pyrimidin-6(5H)-one and using 4-amino-3-chloro-N-(1-methylpiperidin-4-yl)benzamide instead of 4-amino-3-methoxy-N-(1-methylpiperidin-4-yl)benzamide in step 2. 4-Amino-3-chloro-N-(1-methylpiperi-din-4-yl)benzamide was prepared analogously to 4-amino-3-methoxy-N-(1-methylpiperidin-4-yl)benzamide (example 1, step 6) using 4-amino-3-chlorobenzoic acid instead of 4-amino-3-methoxybenzoic acid.
¹H NMR (CDCl₃); δ (ppm): 8.66 (s, 1 H); 8.56 (d, 1 H); 7.86 (s, 1 H); 7.75 - 7.82 (m, 2 H); 7.42 - 7.51 (m, 3 H); 5.84 (d, 1 H); 4.09 (d, 2 H); 3.90 - 4.06 (m, 1 H); 3.81 (s, 3 H); 2.77 - 2.92 (m, 2 H); 2.33 (s, 3 H); 2.19 (td, 2 H); 1.99 - 2.13 (m, 2 H); 1.62 - 1.71 (m, 2 H); 1.01 - 1.17 (m, 1 H); 0.42 - 0.52 (m, 2 H); 0.12 - 0.23 (m, 2 H).
MS (ESI Pos, 3.2 kV, 25 V, 350°C): 579.09 m/z (M⁺).

### Example 71: 4-{[7-(cyclopropylmethoxy)-5-methyl-8-(2-methylphenyl)-6-oxo-5,6-dihydropyrido[3,2-d]pyrimidin-2-yl]amino}-3-methoxy-N-(1-methylpiperidin-4-yl)benzamide

The title compound was prepared analogously to example 34 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-methoxy-5-methylpyrido[3,2-d]pyrimidin-6(5H)-one and using (2-methylphenyl)boronic acid instead of pyrimidin-5-ylboronic acid in step 1.
¹H NMR (CDCl₃) δ (ppm): 8.62 (s, 1 H) 7.93 (s, 1 H) 7.88 (d, 1 H) 7.30 - 7.50 (m, 4 H) 7.19 - 7.25 (m, 1 H) 6.81 (dd, 1 H) 5.81 (d, 1 H) 3.96 - 4.14 (m, 3 H) 3.94 (s, 3 H) 3.82 (s, 3 H) 2.77 - 2.91 (m, 2 H) 2.33 (s, 3 H) 2.15 - 2.26 (m, 2 H) 2.14 (s, 3 H) 1.99 - 2.12 (m, 2 H) 1.61 - 1.71 (m, 2 H) 0.94 - 1.11 (m, 1 H) 0.37 - 0.48 (m, 2 H) 0.04 - 0.17 (m, 2 H).
¹³C NMR (75 MHz, CDCl₃) δ (ppm): 166.64 (s, 1 C), 157.67 (s, 1 C), 154.95 (s, 1 C), 151.81 (s, 1 C), 146.98 (s, 1 C), 144.89 (s, 1 C), 144.33 (s, 1 C), 136.84 (s, 1 C), 135.22 (s, 1 C), 132.49 (s, 1 C), 132.45 (s, 1 C), 129.92 (s, 1 C), 129.82 (s, 1 C), 128.09 (s, 1 C), 126.91 (s, 1 C), 125.40 (s, 1 C), 124.89 (s, 1 C), 118.94 (s, 1 C), 115.96 (s, 1 C), 108.67 (s, 1 C), 77.43 (s, 1 C), 55.90 (s, 1 C), 54.55 (s, 2 C), 46.46 (s, 1 C), 46.22 (s, 1 C), 32.42 (s, 2 C), 28.97 (s, 1 C), 20.13 (s, 1 C), 10.94 (s, 1 C), 3.11 (s, 1 C), 2.98 (s, 1 C).
MS (ESI Pos, 3.2 kV, 25 V, 350°C): 583.21 m/z (MH⁺).

### Example 72: 4-{[7-(cyclopropylmethoxy)-5-methyl-6-oxo-8-(3-thienyl)-5,6-dihydro-pyrido[3,2-d]pyrimidin-2-yl]amino}-3-ethyl-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methylpyrido[3,2-d]pyrimidin-6(5H)-one and using 4-amino-3-ethyl-N-(1-methylpiperidin-4-yl)benzamide instead of 4-amino-3-methoxy-N-(1-methylpiperidin-4-yl)benzamide in step 2. 4-Amino-3-ethyl-N-(1-methylpiperidin-4-yl)benzamide was prepared analogously to 4-amino-3-methoxy-N-(1-methylpiperidin-4-yl)benzamide (example 1, step 6) using 4-amino-3-ethylbenzoic acid instead of 4-amino-3-methoxybenzoic acid.
¹H NMR (CDCl₃) δ (ppm): 8.62 (s, 1 H); 8.30 (d, 1 H); 7.81 (dd, 1 H); 7.63 (d, 1 H); 7.51 (dd, 1 H); 7.40 - 7.48 (m, 2 H); 7.14 (s, 1 H); 5.87 (d, 1 H); 4.06 (d, 2 H); 3.93 - 4.04 (m, 1 H); 3.79 (s, 3 H); 2.78 - 2.91 (m, 2 H); 2.73 (q, 2 H); 2.33 (s, 3 H); 2.13 - 2.25 (m, 2 H); 2.00 - 2.13 (m, 2 H); 1.61 - 1.72 (m, 2 H); 1.33 (t, 3 H); 1.01 - 1.17 (m, 1 H); 0.41 - 0.52 (m, 2 H); 0.09 - 0.23 (m, 2 H).
MS (ESI Pos, 3.2 kV, 25 V, 350°C): 573.18 m/z (MH⁺).

### Example 73: 4-{[7-(cyclopropylmethoxy)-5-methyl-6-oxo-8-(1H-pyrrol-2-yl)-5,6-dihydro-pyrido[3,2-d]pyrimidin-2-yl]amino}-3-methoxy-N-(1-methylpiperidin-4-yl)benzamide

### Step 1: 2-chloro-7-(cyclopropylmethoxy)-5-methyl-8-(1H-pyrrol-2-yl)pyrido[3,2-d]pyrimi-din-6(5H)-one

8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) (0.150 g), [1-(tert-butoxycarbonyl)-1H-pyrrol-2-yl]boronic acid (0.101 g), KF (0.038 g) and tetrakis(triphenylphosphine)palladium(0) (0.051 g) were dissolved in a mixture of acetonitrile (4 ml) and water (0.4 ml). The mixture was heated at 135°C for 1 hour under microwave irradiation. After cooling, the mixture was diluted with dichloromethane and washed with water. The organic phase was dried over Na₂SO₄ and concentrated. The crude was purified on silica gel (eluent: dichloromethane/ethyl acetate/methanol 95/4.5/0.5 (v/v)) to give the title compound as yellow solid.
¹H NMR (CDCl₃) δ (ppm): 11.81 (br. s., 1 H); 8.73 (s, 1 H); 7.73 (ddd, 1 H); 7.14 (dddd, 1 H); 6.44 (dt, 1 H); 4.34 (d, 2 H); 3.78 (s, 3 H); 1.31 - 1.49 (m, 1 H); 0.59 - 0.72 (m, 2 H); 0.35 - 0.45 (m, 2 H).

### Step2: 4-{[7-(cyclopropylmethoxy)-5-methyl-6-oxo-8-(1H-pyrrol-2-yl)-5,6-dihydropyrido [3,2-d]pyrimidin-2-yl]amino}-3-methoxy-N-(1-methylpiperidin-4-yl)benzamide

4-amino-3-methoxy-N-(1-methylpiperidin-4-yl)benzamide (example 1, step 6) (0.048 g) was added to a mixture of 2-chloro-7-(cyclopropylmethoxy)-5-methyl-8-(1H-pyrrol-2-yl)pyrido[3,2-d]pyrimidin-6(5H)-one (0.056 g),, Cs₂CO₃ (0.222 g), (+/-) 2,2'-bis-(diphenylphosphino)-1,1'-binaphthyl (0.025 g) and tris-(dibenzylidenacetone)-dipalladium (0.039 g) in dry toluene (3 ml), under nitrogen atmosphere. The mixture was heated at reflux for 3 hours. After cooling, the mixture was diluted with dichloromethane and washed with water and a saturated solution of NaCl. The organic phase was dried over Na₂SO₄ and the solvent was evaporated. The crude was purified on silica gel (eluent: dichloromethane/methanol/32% NH₄OH 96/4/0.04 (v/v)) and by preparative HPLC (acetonitrile/water/trifluoroacetic acid 200/1800/1 (v/v) / acetonitrile/water/ trifluoroacetic acid 1800/200/1 (v/v), elution gradient 95/5 to 0/100 to 95/5 (v/v)) to give the title compound as yellow powder.
¹H NMR (CDCl₃) δ (ppm): 11.88 (br. s., 1 H); 8.68 (s, 1 H); 8.29 (d, 1 H); 7.74 (s, 1 H); 7.60 - 7.66 (m, 1 H); 7.53 (d, 1 H); 7.29 - 7.34 (m, 1 H); 6.97 (td, 1 H); 6.37 - 6.45 (m, 1 H); 5.95 (d, 1 H); 4.27 (d, 2 H); 4.03 (s, 3 H); 3.93 - 4.13 (m, 1 H); 3.77 (s, 3 H); 2.77 - 2.94 (m, 2 H); 2.34 (s, 3 H); 2.15 - 2.28 (m, 2 H); 2.00 - 2.15 (m, 2 H); 1.63 - 1.73 (m, 2 H); 1.31 - 1.46 (m, 1 H); 0.58 - 0.67 (m, 2 H); 0.33 - 0.42 (m, 2 H).
MS (ESI Pos, 3.2 kV, 25 V, 350°C): 558.17 m/z (MH⁺).

### Example 74: 4-{[7-(cyclopropylmethoxy)-5-methyl-6-oxo-8-pyridin-2-yl-5,6-dihydro-pyrido[3,2-d]pyrimidin-2-yl]amino}-3-methoxy-N-(1-methylpiperidin-4-yl)benzamide

The title compound was prepared analogously to example 44 starting from 8-bromo-2-chloro-7-(cyclopropylmethoxy)-5-methylpyrido[3,2-d]pyrimidin-6(5H)-one (prepared analogously to example 3 using bromomethyl-cyclopropane instead of iodomethane in step 1) instead of 8-bromo-2-chloro-7-ethoxy-5-methylpyrido[3,2-d]pyrimidin-6(5H)-one and using 2-(tributylstannyl)pyridine instead of 2-(tributylstannyl)-1,3-oxazole in step 1.
¹H NMR (CDCl₃) δ (ppm): 8.82 - 8.95 (m, 1 H), 8.64 (s, 1 H), 8.00 (d, 1 H), 7.85 - 7.96 (m, 2 H), 7.50 - 7.57 (m, 1 H), 7.41 - 7.50 (m, 1 H), 7.31 (d, 1 H), 6.92 (dd, 1 H), 5.74 - 5.90 (m, 1 H), 4.15 (d, 2 H), 3.97 - 4.08 (m, 1 H), 3.95 (s, 3 H), 3.82 (s, 3 H), 2.75 - 3.00 (m, 2 H), 2.35 (s, 3 H), 2.14 - 2.28 (m, 2 H), 1.99 - 2.14 (m, 2 H), 0.96 - 1.19 (m, 2 H), 0.78 - 0.96 (m, 1 H), 0.34 - 0.52 (m, 2 H), 0.10 - 0.21 (m, 2 H).
MS (ESI Pos, 3.2 kV, 25 V, 350°C): 570.1 m/z (MH⁺).

### Example 75: 4-{[7-(cyclopropylmethoxy)-5-methyl-6-oxo-8-(3-thienyl)-5,6-dihydro-pyrido[3,2-d]pyrimidin-2-yl]amino}-3-ethoxy-N-(1-methylpiperidin-4-yl)benzamide

### Step 1: Methyl 3-ethoxy-4-nitrobenzoate

Methyl 3-hydroxy-4-nitrobenzoate (0.200 g), K₂CO₃ (0.418 g) and iodoethane (0.245 ml) were mixed in DMF (4 mL) and heated under microwave irradiation in a closed vessel at 40°C for 1 hour. The reaction mixture was partitioned between water and dichloro-methane. The organic layer was separated, dried over Na₂SO₄ and concentrated. The crude was purified on silica gel (eluent: petroleum ether/ethyl acetate 9/1 (v/v)) to give the title compound as off-white solid.
¹H NMR (CDCl₃) δ (ppm): 7.77 - 7.86 (m, 1 H), 7.75 (d, 1 H), 7.63 - 7.72 (m, 1 H), 4.27 (q, 2 H), 3.97 (s, 3 H), 1.50 (t, 3 H).

### Step 2: Methyl 4-amino-3-ethoxybenzoate

Methyl 3-ethoxy-4-nitrobenzoate (0.751 g) was dissolved in a mixture of ethanol (15 ml) and ethyl acetate (15 ml), 10% Pd/C (0.080 g) was added and the mixture was hydrogenated for 2 hours under a pressure of 20 psi of hydrogen. The reaction mixture was filtered and the filtrate was concentrated to dryness to give the title compound.
1 H NMR (DMSO-d6) δ (ppm): 7.37 (dd, 1 H) 7.28 (d, 1 H) 6.65 (d, 1 H) 5.43 - 5.60 (m, 2 H) 4.04 (q, 2 H) 3.74 (s, 3 H) 1.36 (t, 3 H).

### Step 3: 4-amino-3-ethoxybenzoic acid hydrochloride

Methyl 4-amino-3-ethoxybenzoate (0.603 g) was suspended in 37% HCl (20 ml) and the mixture was heated at 70°C for 4 hours. The reaction mixture was concentrated to dryness to give the title compound.
¹H NMR (DMSO-d₆) δ (ppm): 7.43 (dd, 1 H), 7.38 (d, 1 H), 6.92 (d, 1 H), 4.09 (q, 2 H), 1.37 (t, 3 H).

### Step 4: 4-amino-3-ethoxy-N-(1-methylpiperidin-4-yl)benzamide

4-Amino-3-ethoxybenzoic acid hydrochloride (0.156 g), 1-methylpiperidin-4-amine (0.098 g), HOBT (0.146 g), polymer-supported carbodiimide (1.2 mmol/g loading, 0.720 g) and triethylamine (0.218 g) were mixed in dichloromethane (8 ml) and the mixture was stirred under nitrogen overnight. The mixture was filtered; the filtrate was diluted with dichloro-methane and washed with a 10% aqueous solution of Na₂CO₃. The organic layer was separated, dried over Na₂SO₄ and concentrated. The crude was purified on silica gel (eluent: dichloromethane/methanol/32% NH₄OH 10/1/0.1 (v/v)) to give the title compound.
¹H NMR (DMSO-d₆) δ (ppm): 7.73 (d, 1 H); 7.25 - 7.31 (m, 2 H); 6.43 - 6.75 (m, 1 H); 5.12 (d, 2 H); 4.04 (q, 2 H); 3.62 - 3.76 (m, 1 H); 2.70 - 2.88 (m, 2 H); 2.16 (s, 3 H); 1.93 (ddd, 2 H); 1.66 - 1.80 (m, 2 H); 1.55 (dddd, 2 H); 1.36 (t, 3 H).

### Step 5: 4-{[7-(cyclopropylmethoxy)-5-methyl-6-oxo-8-(3-thienyl)-5,6-dihydropyrido[3,2-d]pyrimidin-2-yl]amino}-3-ethoxy-N-(1-methylpiperidin-4-yl)benzamide

The title compound was prepared analogously to example 31 starting from 8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) instead of 8-bromo-2-chloro-7-isopropoxy-5-methylpyrido[3,2-d]pyrimidin-6(5H)-one and using 4-amino-3-ethoxy-N-(1-methylpiperidin-4-yl)benzamide instead of 4-amino-3-methoxy-N-(1-methylpiperidin-4-yl)benzamide in step 2.
¹H NMR (CDCl₃) δ (ppm): 8.64 (s, 1 H); 8.40 (d, 1 H); 7.98 (s, 1 H); 7.82 (dd, 1 H); 7.52 (dd, 1 H); 7.48 (dd, 1 H); 7.40 (d, 1 H); 7.06 (dd, 1 H); 5.88 (d, 1 H); 4.22 (q, 2 H); 4.07 (d, 2 H); 3.91 - 4.05 (m, 1 H); 3.80 (s, 3 H); 2.72 - 2.97 (m, 2 H); 2.33 (s, 3 H); 2.13 - 2.26 (m, 2 H); 2.01 - 2.13 (m, 2 H); 1.56 - 1.70 (m, 2 H); 1.51 (t, 3 H); 0.98 - 1.19 (m, 1 H); 0.38 - 0.56 (m, 2 H); 0.07 - 0.26 (m, 2 H).
MS (ESI Pos, 3.2 kV, 25 V, 350°C): 589.06 m/z (MH⁺).

### Example 76: 4-{[7-(cyclopropylmethoxy)-5-methyl-6-oxo-8-(1H-pyrrol-3-yl)-5,6-dihydro-pyrido[3,2-d]pyrimidin-2-yl]amino}-3-methoxy-N-(1-methylpiperidin-4-yl)benzamide

### Step 1: 2-chloro-7-(cyclopropylmethoxy)-5-methyl-8-{1-[(4-methylphenyl)sulfonyl]-1H-pyrrol-3-yl}pyrido[3,2-d]pyrimidin-6(5H)-one

8-bromo-2-chloro-7-(cyclopropylmethoxy)-5-methylpyrido[3,2-d]pyrimidin-6(5H)-one (prepared analogously to the product of example 3, step 2 using (bromomethyl)cyclopropane instead of iodomethane in step 1) (0.150 g), [1-(triisopropylsilyl)-1H-pyrrol-3-yl]boronic acid (0.129 g), KF (0.038 g) and tetrakis(triphenylphosphine)palladium(0) (0.051 g) were dissolved in a mixture of acetonitrile (2 ml) and water (0.2 ml). The mixture was heated at 135°C for 2 hours under microwave irradiation. After cooling, the mixture was diluted with dichloromethane and washed with water. The organic phase was dried over Na₂SO₄ and concentrated. The crude was purified on silica gel (eluent: dichloromethane/methanol 97/3 (v/v/)) to give the title compound as off-white solid (contained approximately 40% of triphenylphosphine oxide). NaH (60% dispersion, 0.013 g) was added to a solution of the solid just obtained in dry tetrahydro-furan (5 ml), under nitrogen atmosphere, at room temperature. After 10 minutes, 4-methyl-benzenesulfonyl chloride (0.072 g) was added and the reaction was stirred overnight at room temperature. A saturated solution of NH₄Cl was added and the mixture was extracted with dichloromethane. The organic phase was dried over Na₂SO₄ and concentrated. The crude was purified on silica gel (eluent: ethyl acetate/petroleum ether 1/1 (v/v)) to give the title compound which was directly used for the next step.

### Step 2: 4-{[7-(cyclopropylmethoxy)-5-methyl-6-oxo-8-(1H-pyrrol-3-yl)-5,6-dihydro-pyrido[3,2-d]pyrimidin-2-yl]amino}-3-methoxy-N-(1-methylpiperidin-4-yl)benzamide

2-chloro-7-(cyclopropylmethoxy)-5-methyl-8-{1-[(4-methylphenyl)sulfonyl]-1H-pyrrol-3-yl}pyrido[3,2-d]pyrimidin-6(5H)-one (0.047 g), 4-amino-3-methoxy-N-(1-methylpiperidin-4-yl)benzamide (0.026 g), Cs₂CO₃ (0.126 g), (+/-) 2,2'-bis-(diphenylphosphino)-1,1'-binaphthyl (0.015 g) and tris-(dibenzylidenacetone)-dipalladium (0.022 g) were dissolved in dry toluene (2 ml), under nitrogen atmosphere. The mixture was heated at reflux for 4 hours. After cooling, the mixture was diluted with dichloromethane and washed with water and a saturated solution of NaCl. The organic phase was dried over Na₂SO₄ and concentrated. The solid obtained was purified on silica gel (eluent: dichloro-methane/methanol/32% NH₄OH 99/2/0.02 (v/v)) to give 0.032 g of a yellow solid. Tetrabutylammonium fluoride (1 M in tetrahydrofuran, 0.126 ml) was added to a solution of the solid obtained in tetrahydrofuran (1 ml) and the mixture was heated at reflux for 10 minutes. After cooling, the solvent was evaporated and the residue was partitioned between dichloromethane and water. The organic phase was separated, washed with a saturated solution of NaCl, dried over Na₂SO₄ and the solvent was evaporated. The residue was purified on silica gel (dichloromethane/methanol/32% NH₄OH 98/2/0.2 (v/v)) and further triturated in methanol to give the title compound as yellow solid.
MS (ESI Pos, 3.2 kV, 25 V, 350°C): 558.14 m/z (MH⁺).

### Example 77: 4-({7-(cyclopropylmethoxy)-5-methyl-6-oxo-8-[(1E)-prop-1-en-1-yl]-5,6-dihydropyrido[3,2-d]pyrimidin-2-yl}amino)-3-ethoxy-N-(1-methylpiperidin-4-yl)benzamide

The title compound was prepared analogously to example 2 using (bromomethyl)cyclopro-pane instead of iodomethane in step 3 and using 4-amino-3-ethoxy-N-(1-methylpiperidin-4-yl)benzamide instead of 4-amino-3-methoxy-N-(1-methylpiperidin-4-yl)benzamide step 4. 4-amino-3-ethoxy-N-(1-methylpiperidin-4-yl)benzamide was prepared as described in example 75.
¹H NMR (CDCl₃) δ (ppm): 8.70 (d, 1 H); 8.60 (s, 1 H); 7.97 (s, 1 H); 7.46 (d, 1 H); 7.18 - 7.37 (m, 2 H); 7.04 (dq, 1 H); 5.93 (d, 1 H); 4.27 (q, 2 H); 4.23 (d, 2 H); 3.91 - 4.11 (m, 1 H); 3.74 (s, 3 H); 2.77 - 2.94 (m, 2 H); 2.33 (s, 3 H); 2.14 - 2.27 (m, 2 H); 2.11 (dd, 3 H); 2.03 - 2.09 (m, 2 H); 1.62 - 1.72 (m, 2 H); 1.55 (t, 3 H); 1.19 - 1.41 (m, 1 H); 0.56 - 0.68 (m, 2 H); 0.30 - 0.41 (m, 2 H).
MS (ESI Pos, 3.2 kV, 25 V, 350°C): 547.16 m/z (MH⁺).

### Commercial utility

The compounds, salts thereof, and the stereoisomers of the compounds and the salts thereof according to the invention are hereinafter referred to as the compounds of the invention. In particular, the compounds of the invention are pharmaceutically acceptable.

The compounds of the invention have valuable pharmaceutical properties which make them commercially utilizable. Thus, one aspect of the invention relates to compounds according to formula I for use in the treatment or prophylaxis of diseases. In particular, as PIk1 inhibitors, they are able to interfere with the cell cycle of various cells, particularly neoplastic cells. In particular, the PIk1 inhibiting compounds of the invention can disrupt mitosis and drive cancer cells into apoptosis. The compounds of the invention are distinguished by valuable and desirable properties, such as, for example, high selectivity and low toxicity. On cellular level the compounds exhibit a cytotoxic effect only or preferentially on proliferating cells. The compounds are able to induce mitotic arrest in proliferating cells. Other valuable properties of the compounds of the invention include superior bioavailability in general (e.g., good enteral absorption), superior therapeutic window, superior pharmacokinetics (e.g., half-life), improved tolerability as compared to other anti-neoplastic agents, and further beneficial effects related with their therapeutic and pharmaceutical suitability.

Accordingly, the invention further relates to the compounds of the invention for use in the treatment or prophylaxis of diseases. Furtheron, the invention relates to the compounds of the invention for use in the treatment or prophylaxis of diseases alleviated by inhibition of kinases involved in cell division, preferably Polo-like kinases, even more preferably PIk1. In particular, the invention relates to the compounds of the invention for use in the treatment or prophylaxis of diseases characterized by increased PIk1 activity. Increased activity means that the activity of PIk1 in a given cell, group of cells, tissue, or region within a tissue is higher by a certain factor than it is in the healthy state. The factor by which PIk1 activity is increased can be, e.g., at least 1.5, at least 3, at least 10, at least 30, or even at least 100. Without being meant as a limitation, the reason for said increased PIk1 activity may be increased expression ("overexpression") of the PIk1 gene or genes, which can be due to, among others, altered regulation of expression, mutations in the PIk1 promotor, gene duplication, gene amplification, genomic rearrangements, and so forth. Other processes leading to increased PIk1 activity might comprise reduced inhibition of PIk1 enzyme, enhanced PIk1 stability, reduced PIk1 inactivation or degradation, and altered availability of substrate(s) and/or cofactors.

Preferably, the invention further relates to the compounds of the invention for use in the treatment or prophylaxis of diseases comprising abnormal cell growth. In one embodiment, said abnormal cell growth is a cancer. In particular, the disease may be one of the following: Cancers of the colon including colorectal carcinoma, ovaries, breast, prostate, bladder, lung, gastrointestinal carcinomas, gastrointestinal stromal tumors, small cell lung cancer, head and neck cancer, cancers of cervix, pancreas, esophagus, kidney, larynx and hypopharynx, liver, endocrine glands, soft tissue, testis, retinoblastoma and Wilms tumor, endometrial tumors, malignant melanoma, non-Hodgekins lymphoma, Hodgekins lymphoma or other lymphomas, chronic and acute myeloic leukemia, acute lymphoblastic leukemia, T-cell lymphoma, plasma cell neoplasia, and leukemias, hematological malignancies including multiple myeloma, soft tissue sarcoma, osteosarcoma, fibrosarcoma, and other tumors of mesothelial origin, glioma, astrocytoma, cancers of unknown primary site, glioblastoma and other brain tumors and tumors of neuronal origin, germ cell cancers, myelodysplastic syndromes, myeloproliferative syndromes, disorders such as polycythemia vera, essential thrombocytopenia, myelofibrosis, hypereosinophilic syndrome, or other paraneoplastic syndromes. In another embodiment, said abnormal cell growth is a benign proliferative disease such as, e.g., benign prostatic hyperplasia, restenosis, fibrosis, or psoriasis, or any other kind of unwanted cell or tissue proliferation.

The invention also relates to the use of a compound of the invention in the manufacture of a pharmaceutical composition inhibiting PIk1, in particular a pharmaceutical composition for the treatment or prophylaxis of diseases alleviated by inhibition of PIk1, preferably, a pharmaceutical composition for the treatment or prophylaxis of the diseases exemplified above.

The invention further relates to a method of treating or preventing a disease comprising administering to a patient in need thereof a therapeutically effective amount of at least one of the compounds of the invention.

In particular, the invention relates to a method of treating or preventing one of the above mentioned diseases comprising administering to a patient in need thereof a therapeutically effective amount of at least one of the compounds of the invention.

Especially, the invention relates to a method of treating or preventing a disease which is alleviated by inhibition of Plk1 comprising administering to a patient in need thereof a therapeutically effective amount of at least one of the compounds of the invention.

Furthermore, the invention preferably relates to a method of treating or preventing cancers of the colon including colorectal carcinoma, ovaries, breast, prostate, bladder, lung, gastrointestinal carcinomas, gastrointestinal stromal tumors, small cell lung cancer, head and neck cancer, cancers of cervix, pancreas, esophagus, kidney, larynx and hypopharynx, liver, endocrine glands, soft tissue, testis, retinoblastoma and Wilms tumor, endometrial tumors, malignant melanoma, non-Hodgekins lymphoma, Hodgekins lymphoma or other lymphomas, chronic and acute myeloic leukemia, acute lymphoblastic leukemia, T-cell lymphoma, plasma cell neoplasia, and leukemias, hematological malignancies including multiple myeloma, soft tissue sarcoma, osteosarcoma, fibrosarcoma, and other tumors of mesothelial origin, glioma, astrocytoma, cancers of unknown primary site, glioblastoma and other brain tumors and tumors of neuronal origin, germ cell cancers, myelodysplastic syndromes, myeloproliferative syndromes, disorders such as polycythemia vera, essential thrombocytopenia, myelofibrosis, hypereosinophilic syndrome, or other paraneoplastic syndromes, or benign proliferative disease such as, e.g., benign prostatic hyperplasia, restenosis, fibrosis, or psoriasis, or any other kind of unwanted cell or tissue proliferation, comprising administering to a patient in need thereof a therapeutically effective amount of at least one of the compounds of the invention.

In the above methods, the patient is preferably a mammal, more preferably a human. Furthermore, in the above methods, at least one of the compounds of the invention can be used. Preferably, one or two of the compounds of the invention are used, more preferably, one of the compounds of the invention is used.

In a particularly preferred embodiment of the invention, the above methods of treating or preventing one of the above mentioned diseases comprise administering to a patient in need thereof a therapeutically effective amount of one compound of the examples according to the present invention.

The invention furthermore relates to a pharmaceutical composition which comprises at least one of the compounds of the invention together with at least one pharmaceutically acceptable carrier, diluent or excipient.

Preferably, the pharmaceutical composition comprises one or two of the compounds of the invention. More preferably, the pharmaceutical composition comprises one of the compounds of the invention.

In a particularly preferred embodiment of the invention, the pharmaceutical composition comprises a compound of the examples according to the present invention together with at least one pharmaceutically acceptable carrier, diluent or excipient.

The invention additionally relates to a pharmaceutical composition comprising at least one of the compounds of the invention, at least one pharmaceutically acceptable carrier, diluent or excipient, and at least one additional therapeutic agent with the proviso that the compound or compounds of the invention and the at least one addtional therapeutic agent are therapeutically compatible. By therapeutically compatible it is meant that (a) the toxicity of the compound or compounds of the invention and the toxicity of the at least one addtional therapeutic agent do not add up to an unacceptable toxicity, and (b) that the therapeutic effect of the compound or compounds of the invention is not modified or reduced in an unacceptable or undesired way by the at least one additional therapeutic agent, and vice versa. In a preferred embodiment, the therapeutic effect of the compound or compounds of the invention and the therapeutic effect of the at least one addtional therapeutic agent are additive. In another preferred embodiment, the therapeutic effect of the compound or compounds of the invention and the therapeutic effect of the at least one addtional therapeutic agent are synergistic, i.e., their combined effect is different from, preferably more beneficial than, the pure sum of the therapeutic effects when the compound or compounds and the agent(s) are administered alone, without any interaction of the effects the compound or compounds and the effects the agent(s) exert to the treated patient.

The invention also relates to a kit of parts, comprising at least one pharmaceutical composition comprising at least one of the compounds of the invention, and at least one pharmaceutical composition comprising at least one additional therapeutic agent.

Typical additional therapeutic agents useful in the present invention include, but are not limited to, anti-neoplastic agents in general, cytostatic agents, cytotoxic agents, antimetabolites, microtubule interfering agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies (including, but not limited to, murine, chimeric, and humanized monoclonal antibodies), proapoptotic agents, hormones, hormone analogues, antibiotic agents, anti-emetic agents, and analgetic agents.

In this respect, the therapeutic agent includes anti-neoplastic agents, cytostatic agents, cytotoxic agents, antimetabolites, microtubule interfering agents, platinum complexes, DNA alkylating agents, DNA intercalating agents, topoisomerase I inhibitors, topoisomerase II inhibitors, signal transduction inhibitors including kinase inhibitors, cell cycle signalling inhibitors, angiogenesis inhibitors including antibodies and receptor tyrosine kinase inhibitors, proapoptotic agents, hormones, hormone analogues, antibiotic agents, immunotherapeutic agents, monoclonal antibodies (including, but not limited to, murine, chimeric, and humanized monoclonal antibodies), anti-emetic agents, or analgetic agents in form of the free compounds, the pharmaceutically acceptable salts thereof, the pharmaceutically acceptable derivatives thereof (e.g., but not limited to, ester derivatives), the solvates thereof and the stereoisomers of the compounds, salts, derivatives and solvates.

Antimetabolites interfere with DNA synthesis. Purine analogues and pyrimidine analogues are incorporated, during the S phase of the cell cycle, into growing DNA strands and thereby suppress incorporation of the proper DNA building blocks, purines and pyrimidines. Examples for purine analogues and pyrimidine analogues include, but are not limited to, 5-fluorouracil, floxuridine, cytosine arabinose, mercaptopurine, thioguanine, fludarapine, pentostatin, and cladribine. Another class of antimetabolites prevents the synthesis of tetrahydrofolate, which is essential for purine and pyrimidine biosynthesis. Examples for antimetabolites suppressing synthesis of tetrahydrofolate include, but are not limited to, methotrexate and pemetrexed.

Microtubule interfering agents are chemotherapeutics that are directed against the formation of microtubules by tumor cells during M phase of the cell cycle. The group of anti-microtubule agents comprises vinca alkaloids and diterpenoids. Examples for vinca alkaloids include vinblastine, navelbine, vindesine, vinorelbine, and vincristine. Examples for diterpenoids include, but are not limited to, paclitaxel and docetaxel.

Platinum complexes are active against tumor cells by interacting with the cells' genomic DNA. More specifically, crosslinks are introduced into the DNA which introduces serious damage to the affected tumor cells. Examples for platinum complexes include, but are not limited to, cisplatin, carboplatin, and oxaliplatin.

Alkylating agents act by alkylating the genomic DNA of tumor cells, which interrupts normal function of the DNA and causes aptoptosis. Examples for alkylating agents include, but are not limited to, alkyl sulfonates (e.g., busulfan), ethyleneimines and methylmelamines (e.g., hexamethylmelamine, thiotepa), nitrogen mustards (e.g., melphalan, cyclophosphamide, mechlorethamine, uramustine, and chlorambucil), nitrosoureas (e.g., carmustine, streptozocin), and triazenes (e.g., dacarbazine, temozolomide).

Inhibitors of topoisomerase I or topoisomerase II inhibit these enzymes' essential function in DNA supercoiling during cell division. Examples for inhibitors of topoisomerase I include, but are not limited to, camptothecins (e.g., irinotecan, topotecan). Examples for inhibitors of topoisomerase II include, but are not limited to, amsacrine, etoposide, etoposide phosphate, and teniposide.

Signal transduction inhibitors are compounds that interfere with certain cellular processes such as, e.g., cell division. Compounds useful for treatment of neoplasms include, but are not limited to, inhibitors of receptor and non-receptor tyrosine kinases, inhibitors of serine/threonine kinases, inhibitors of phosphoinositide 3-kinases, inhibitors of Ras oncogenes, myoinositol signaling inhibitors, and SH2/SH3 domain blockers. Examples for inhibitors of receptor tyrosine kinases include, but are not limited to, erlotinib (Tarceva), gefitinib (Iressa), dasatinib (Sprycel), sorafenib (Nexavar), SU11248 (Sutent), and lapatinib (Tycerb).

Cell cycle signalling inhibitors are intended to interfere with cell cycle progression by inhibiting the biological effect of crucial signalling molecules such as, e.g., cyclins/cyclin-dependent kinases and other essential cell cycle regulators.

Angiogenesis inhibitors such as, e.g., antibodies or receptor tyrosine kinase inhibitors inhibit the neovascularization of tumors. Examples include, but are not limited to, anti-VEGF antibodies such as, e.g., bevacizumab.

Monoclonal antibodies (including, but not limited to, murine, chimeric, and humanized monoclonal antibodies) are capable of binding to surface antigens specific for tumor cells, thus rendering susceptible the cancer cells to attack by the immune system. Examples for monoclonal antibodies as antitumor agents include, but are not limited to, trastuzumab (Herceptin), rituximab (Rituxan), gemtuzumab ozogamicin (Mylotarg), alemtuzumab (Campath), ibritumomab tiuxetan (Zevalin), tositumomab (Bexxar), cetuximab (Erbitux), bevacizumab (Avastin), and panitumumab (Vectibix).

Proapoptotic agents are intended to unblock apoptosis in cancer cells by reducing Bcl-2 activity in malignant cells characterized by upregulation of Bcl-2 activity. A proapoptotic effect is mediated by, for example, without being limited thereto, bcl-2 antisense oligonucleotides.

Hormones and hormone analogues are useful to treat neoplasms in which cell growth is affected by the level at which a certain hormone or hormones (or analogues thereof) are present. Examples for classes of hormones and hormone analogues include, but are not limited to, adrenocorticosteroids, androgens, anti-androgens, aromatase inhibitors, estrogens, anti-estrogens, gonadotropin-releasing hormone and its analogues, and progestrins. Examples for individual hormones and hormone analogues include, but are not limited to, aminoglutethimide, anastrozole, bicalutamide, cyproterone acetate, dexamethasone, droloxifene, dutasteride, exemestane, finasteride, flutamide, gosereline, iodoxyfene, letrazole, luprolide, megrestrol acetate, nilutamide, prednisolone, prednisone, raloxifene, tamoxifen, toremifene, and vorazole.

Antibiotic chemotherapeutic agents (antineoplastics) bind to or intercalate with DNA, which leads to cell death. Examples of antibiotic agents effective against cancer cells include, but are not limited to, actinomycin D, bleomycin, plicamycin, mitomycin, and anthracyclins such as, e.g., doxorubicin, daunorubicin, epirubicin, and others.

Immunotherapeutic agents are activating the immune system in order to attack cancer cells. Examples for immunotherapeutic agents include, but are not limited to, monoclonal antibodies, radiolabeled murine antibodies, and agents used for topic immunotherapy such as, e.g., imiquimod.

Anti-emetic agents are agents effective against nausea and vomiting, which can arise as side effects of chemotherapy. Examples for anti-emetic agents include, but are not limited to, 5HT₃ receptor antagonists (e.g., dolasetron, granisetron, ondansetron, tropisetron, palonosetron, and others), dopamine antagonists (e.g., domperidone, metoclopramide, droperidol, haloperidol, chlorpromazine, promethazine, prochlorperazine, and others), antihistamines (H₁R antagonists; cyclizine, diphenhydramine, dimenhydrinate, meclizine, hydroxyzine, and others), benzodiazepines such as, e.g., midazolam and others, cannabinoids such as, e.g, cannabis, marinol and others, and other anti-emetic agents not falling into one of the above groups, such as, e.g., emetrol, propofol, trimethobenzamide, and ginger extracts, among others.

Analgetic agents are compounds effective in relieving pain. Examples for analgetics include, but are not limited to, NSAIDs (non-steroidal anti-inflammatory drugs such as, e.g., Aspirin, rofecoxib, celecoxib, and others), paracetamol, carbamazepine, gabapentin, pregabalin, opiates, morphinomimetics (e.g., morphine, codeine, oxycodone, hydrocodone, diamorphine, pethidine, tramadol, buprenorphine, and others), and tricyclic antidepressants such as, e.g., amitriptyline.

In a preferred embodiment, the pharmaceutical composition comprises a compound of the invention in combination with an alkylating agent. In a particularly preferred embodiment, the pharmaceutical composition comprises:
a compound of the invention and cyclophosphamid,
a compound of the invention and chlorambucil,
a compound of the invention and melphalan,
a compound of the invention and BCNU (carmustin),
a compound of the invention and CCNU (lomustin),
a compound of the invention and thiotepa,
a compound of the invention and busulfan,
a compound of the invention and dacarbazine, or
a compound of the invention and temozolomide.

In a further preferred embodiment, the pharmaceutical composition comprises a compound of the invention in combination with an antimetabolite. In a particularly preferred embodiment, the pharmaceutical composition comprises:
a compound of the invention and aminopterin,
a compound of the invention and 5-fluorouracil,
a compound of the invention and methotrexate,
a compound of the invention and cytarabin,
a compound of the invention and mercaptopurin,
a compound of the invention and azathioprin,
a compound of the invention and azacytidine,
a compound of the invention and gemcitabine, or
a compound of the invention and capecitabine.

In a further preferred embodiment, the pharmaceutical composition comprises a compound of the invention in combination with an alkaloid. In a particularly preferred embodiment, the pharmaceutical composition comprises:
a compound of the invention and vinblastin,
a compound of the invention and vincristin,
a compound of the invention and daunomycin,
a compound of the invention and adriamycin,
a compound of the invention and bleomycin,
a compound of the invention and bleomycin, or
a compound of the invention and procarbazin.

In a further preferred embodiment, the pharmaceutical composition comprises a compound of the invention in combination with a platinum derivative. In a particularly preferred embodiment, the pharmaceutical composition comprises:
a compound of the invention and cisplatin,
a compound of the invention and carboplatin,
a compound of the invention and oxaliplatin,
a compound of the invention and satraplatin.

In a further preferred embodiment, the pharmaceutical composition comprises a compound of the invention in combination with a tubulin inhibitor. In a particularly preferred embodiment, the pharmaceutical composition comprises:
a compound of the invention and paclitaxel, or
a compound of the invention and docetaxel.

In a further preferred embodiment, the pharmaceutical composition comprises a compound of the invention in combination with a topoisomerase inhibitor. In a particularly preferred embodiment, the pharmaceutical composition comprises:
a compound of the invention and irinotecan, or
a compound of the invention and topotecan.

In a further preferred embodiment, the pharmaceutical composition comprises a compound of the invention in combination with an inhibitor of the EGFR pathway. In a particularly preferred embodiment, the pharmaceutical composition comprises:
a compound of the invention and cetuximab,
a compound of the invention and trastuzumab,
a compound of the invention and gefitinib,
a compound of the invention and erlotinib,
a compound of the invention and lapatinib,
a compound of the invention and imatinib,
a compound of the invention and nilotinib, or
a compound of the invention and dasatinib.

In a further preferred embodiment, the pharmaceutical composition comprises a compound of the invention in combination with an inhibitor of farnesyltransferase. In a particularly preferred embodiment, the pharmaceutical composition comprises a compound of the invention and tipifarnib.

In a further preferred embodiment, the pharmaceutical composition comprises a compound of the invention in combination with an inhibitor of the VEGF-/ VEGF-R signaling pathway. In a particularly preferred embodiment, the pharmaceutical composition comprises:
a compound of the invention and a VEGFR2 Mab,
a compound of the invention and bevacizumab,
a compound of the invention and sorafenib,
a compound of the invention and sunitinib, or
a compound of the invention and valatinib.

In a further preferred embodiment, the pharmaceutical composition comprises a compound of the invention in combination with a specific monoclonal antibody. In a particularly preferred embodiment, the pharmaceutical composition comprises:
a compound of the invention and trastuzumab,
a compound of the invention and rituximab,
a compound of the invention and gemtuzumab,
a compound of the invention and alemtuzumab,
a compound of the invention and ibritumomab,
a compound of the invention and tositumomab,
a compound of the invention and cetuximab,
a compound of the invention and bevacizumab,
a compound of the invention and panitumumab,
a compound of the invention and Anti-CD20 Mab, or
a compound of the invention and Anti-CD52 Mab.

In a further preferred embodiment, the pharmaceutical composition comprises a compound of the invention in combination with an Eg5 inhibitor. In a particularly preferred embodiment, the pharmaceutical composition comprises a compound of the invention and ispinesib.

In a further preferred embodiment, the pharmaceutical composition comprises a compound of the invention in combination with a proteasome inhibitor. In a particularly preferred embodiment, the pharmaceutical composition comprises a compound of the invention and bortezomib.

In a further preferred embodiment, the pharmaceutical composition comprises a compound of the invention in combination with a cyclin-dependent kinase inhibitor. In a particularly preferred embodiment, the pharmaceutical composition comprises:
a compound of the invention and flavopyridol, or
a compound of the invention and R-roscovitine.

In a further preferred embodiment, the pharmaceutical composition comprises a compound of the invention in combination with a multitarget enzyme inhbitor. In a particularly preferred embodiment, the pharmaceutical composition comprises a compound of the invention and pemetrexed.

In a further preferred embodiment, the pharmaceutical composition comprises a compound of the invention in combination with a histon deacetylase inhbitor. In a particularly preferred embodiment, the pharmaceutical composition comprises:
a compound of the invention and vorinostat,
a compound of the invention and belinostat,
a compound of the invention and LBH589,
a compound of the invention and depsipeptide,
a compound of the invention and 2-propylpentanoic acid,
a compound of the invention and BYK397957,
a compound of the invention and BYK408740.

In a further preferred embodiment, the pharmaceutical composition comprises a compound of the invention in combination with a hypo- or demethylating agent. In a particularly preferred embodiment, the pharmaceutical composition comprises:
a compound of the invention and procaine,
a compound of the invention and azacitidine, or
a compound of the invention and decitabine.

The invention additionally relates to a pharmaceutical composition comprising at least one of the compounds of the invention, at least one pharmaceutically acceptable carrier, diluent or excipient, and two additional therapeutic agents with the proviso that the compound or compounds of the invention and the two addtional therapeutic agents are therapeutically compatible. By therapeutically compatible it is meant that (a) the toxicity of the compound or compounds of the invention and the toxicity of the two addtional therapeutic agents do not add up to an unacceptable toxicity, and (b) that the therapeutic effect of the compound or compounds of the invention is not modified or reduced in an unacceptable or undesired way by the two additional therapeutic agents, and vice versa. In a preferred embodiment, the therapeutic effect of the compound or compounds of the invention and the therapeutic effect of the two addtional therapeutic agents are additive. In another preferred embodiment, the therapeutic effect of the compound or compounds of the invention and the therapeutic effect of the two addtional therapeutic agents are synergistic, i.e., their combined effect is different from, preferably more beneficial than, the pure sum of all three therapeutic effects when the compound or compounds and the agents are administered alone, without any interaction of the effects the compound or compounds and the effects the agents exert to the treated patient.

In a further preferred embodiment, the first and the second additional therapeutic agents are independently selected from anti-neoplastic agents, cytostatic agents, cytotoxic agents, antimetabolites, microtubule interfering agents, platinum derivatives, DNA alkylating agents, DNA intercalating agents, topoisomerase I inhibitors, topoisomerase II inhibitors, signal transduction inhibitors including kinase inhibitors, cell cycle signalling inhibitors, angiogenesis inhibitors including antibodies and receptor tyrosine kinase inhibitors, proapoptotic agents, hormones, hormone analogues, antibiotic agents, immunotherapeutic agents, monoclonal antibodies (including, but not limited to, murine, chimeric, and humanized monoclonal antibodies), anti-emetic agents, and analgetic agents, in form of the free compounds, the pharmaceutically acceptable salts thereof, the pharmaceutically acceptable derivatives thereof (e.g., but not limited to, ester derivatives), the solvates thereof and the stereoisomers of the compounds, salts, derivatives and solvates.

In a particularly preferred embodiment, one of the two additional therapeutic agents is a taxane.

In a further particularly preferred embodiment, one of the two additional therapeutic agents is a platinum compound.

In a further particularly preferred embodiment, one of the two additional therapeutic agents is a hormone or hormone analogue.

In a further particularly preferred embodiment, one of the two additional therapeutic agents is a monoclonal antibody.

The invention also relates to a kit of parts, comprising at least one pharmaceutical composition comprising at least one of the compounds of the invention, and at least one pharmaceutical composition comprising two additional therapeutic agents.

The invention also relates to a kit of parts, comprising at least one pharmaceutical composition comprising at least one of the compounds of the invention and a first additional therapeutic agent, and at least one pharmaceutical composition comprising a second additional therapeutic agent.

The invention also relates to a kit of parts, comprising at least one pharmaceutical composition comprising at least one of the compounds of the invention, at least one pharmaceutical composition comprising a first additional therapeutic agent, and at least one pharmaceutical composition comprising a second additional therapeutic agent.

The above mentioned compound of the invention is preferably a compound according to the examples.

The invention furthermore relates to pharmaceutical compositions according to the invention, as defined above, inhibiting PIk1, especially for the treatment or prophylaxis of diseases alleviated by inhibition of PIk1, in particular for the treatment or prophylaxis of the diseases exemplified above.

The invention also encompasses pharmaceutical compositions according to the invention, as defined above, for the treatment or prophylaxis of the following diseases involving abnormal cell growth: Cancers of the colon including colorectal carcinoma, ovaries, breast, prostate, bladder, lung, gastrointestinal carcinomas, gastrointestinal stromal tumors, small cell lung cancer, head and neck cancer, cancers of cervix, pancreas, esophagus, kidney, larynx and hypopharynx, liver, endocrine glands, soft tissue, testis, retinoblastoma and Wilms tumor, endometrial tumors, malignant melanoma, non-Hodgekins lymphoma, Hodgekins lymphoma or other lymphomas, chronic and acute myeloic leukemia, acute lymphoblastic leukemia, T-cell lymphoma, plasma cell neoplasia, and leukemias, hematological malignancies including multiple myeloma, soft tissue sarcoma, osteosarcoma, fibrosarcoma, and other tumors of mesothelial origin, glioma, astrocytoma, cancers of unknown primary site, glioblastoma and other brain tumors and tumors of neuronal origin, germ cell cancers, myelodysplastic syndromes, myeloproliferative syndromes, disorders such as polycythemia vera, essential thrombocytopenia, myelofibrosis, hypereosinophilic syndrome, or other paraneoplastic syndromes. In another embodiment, said abnormal cell growth is a benign proliferative disease such as, e.g., benign prostatic hyperplasia, restenosis, fibrosis, or psoriasis, or any other kind of unwanted cell or tissue proliferation.

The pharmaceutical compositions according to the invention preferably contain the compound or compounds of the invention in a total amount of from 0.1 to 99.9 wt%, more preferably 5 to 95 wt%, in particular 20 to 80 wt%. In case at least one therapeutic agent selected from anti-neoplastic agents in general, cytostatic agents, cytotoxic agents, antimetabolites, microtubule interfering agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies (including, but not limited to, murine, chimeric, and humanized monoclonal antibodies), proapoptotic agents, hormones, hormone analogues, antibiotic agents, anti-emetic agents, and analgetic agents is present in the pharmaceutical compositions of the invention, the total amount of said therapeutic agent or therapeutic agents in the pharmaceutical compositions is preferably in the range of from 0.1 to 99.9 wt%, more preferably 5 to 95 wt%, in particular 20 to 80 wt%, with the proviso that the total amount of the compound or compounds of the invention and the therapeutic agent or therapeutic agents is less than 100 wt%. Preferably, the at least one compound of the invention and the at least one therapeutic agent are present in the pharmaceutical composition in a weight ratio of from 1000:1 to 1:1000.

As pharmaceutically acceptable auxiliaries, any auxiliaries known to be suitable for preparing pharmaceutical compositions can be used. Examples thereof include, but are not limited to, solvents, diluents, excipients, dispersants, emulsifiers, solubilizers, gel formers, ointment bases, antioxidants, preservatives, stabilizers, carriers, fillers, binders, thickeners, complexing agents, disintegrating agents, buffers, permeation promoters, polymers, lubricants, coating agents, propellants, tonicity adjusting agents, surfactants, colorants, flavorings, sweeteners and dyes. In particular, auxiliaries of a type appropriate to the desired formulation and the desired mode of administration are used.

The pharmaceutical compositions can be formulated, for example, into tablets, coated tablets (dragees), pills, cachets, capsules (caplets), granules, powders, suppositories, solutions (e.g., but not limited to, sterile solutions), emulsions, suspensions, ointments, creams, lotions, pastes, oils, gels, sprays and patches (for example, without being limited thereto, transdermal therapeutic systems). Additionally, the pharmaceutical compositions can be prepared as, e.g., liposome delivery systems, systems in which the compound of the invention is coupled to monoclonal antibodies, and systems in which the compound of the invention is coupled to polymers (for example, without being limited thereto, soluble or biodegradable polymers).

In case of pharmaceutical compositions comprising at least one of the compounds of the invention and at least one therapeutic agent selected from anti-neoplastic agents in general, cytostatic agents, cytotoxic agents, antimetabolites, anti-microtubule agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies (including, but not limited to, murine, chimeric, and humanized monoclonal antibodies), proapoptotic agents, hormones, hormone analogues, antibiotic agents, anti-emetic agents, and analgetic agents, the compound of the invention and the therapeutic agent may be formulated together into the same dosage form (for example, without being limited thereto, tablets or solutions for injection), separately into the same dosage form (for example, without being limited thereto, tablets or solutions for injection), or into different dosage forms (for example, without being limited thereto, the compound of the invention may be formulated as tablet and the therapeutic agent may be formulated as powder, solution or suspension).

The pharmaceutical compositions can be manufactured in a manner known to a person skilled in the art, e.g. by dissolving, mixing, granulating, dragee-making, levigating, emulsifying, encapsulating, entrapping or lyophilizing processes.

The selected formulation depends *inter alia* on the route of administering the pharmaceutical composition. The pharmaceutical compositions of the invention can be administered by any suitable route, for example, by the oral, sublingual, buccal, intravenous, intraarterial, intramuscular, subcutaneous, intracutaneous, topical, transdermal, intranasal, intraocular, intraperitoneal, intrasternal, intracoronary, transurethral, rectal or vaginal route, by inhalation or by insufflation. Oral administration is preferred.

In case of pharmaceutical compositions comprising at least one of the compounds of the invention and at least one therapeutic agent selected from anti-neoplastic agents in general, cytostatic agents, cytotoxic agents, antimetabolites, anti-microtubule agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies (including, but not limited to, murine, chimeric, and humanized monoclonal antibodies), proapoptotic agents, hormones, hormone analogues, antibiotic agents, anti-emetic agents, and analgetic agents, the compound of the invention and the therapeutic agent may be administered by the same route, e.g., without limitation, orally, or by different routes, e.g., without limitation, the compound of the invention can be administered orally and the therapeutic agent can be administered topically or by injection.

Tablets, coated tablets (dragees), pills, cachets, capsules (caplets), granules, solutions, emulsions and suspensions are, e.g., suitable for oral administration. In particular, said formulations can be adapted so as to represent, for example, an enteric form, an immediate release form, a delayed release form, a repeated dose release form, a prolonged release form or a sustained release form. Said forms can be obtained, for example, by coating tablets, by dividing tablets into several compartments separated by layers disintegrating under different conditions (e.g., under different pH conditions) or by coupling the compound of the invention to a biodegradable polymer.

Administration by inhalation is preferably made by using an aerosol. The aerosol is a liquid-gaseous dispersion, a solid-gaseous dispersion or a mixed liquid/solid-gaseous dispersion.

The aerosol may be generated by means of aerosol-producing devices such as dry powder inhalers (DPIs), pressurized metered dose inhalers (PMDIs) and nebulizers. Depending on the kind of the compound of the invention, and optionally the therapeutic agent, to be administered, the aerosol-producing device can contain the compound and, optionally, the therapeutic agent in form of a powder, a solution or a dispersion. The powder may contain, for example, one or more of the following auxiliaries: carriers, stabilizers and fillers. The solution may contain in addition to the solvent, for example, one or more of the following auxiliaries: propellants, solubilizers (co-solvents), surfactants, stabilizers, buffers, tonicity adjusting agents, preservatives and flavorings. The dispersion may contain in addition to the dispersant, for example, one or more of the following auxiliaries: propellants, surfactants, stabilizers, buffers, preservatives and flavorings. Examples of carriers include, but are not limited to, saccharides, e.g. lactose and glucose. Examples of propellants include, but are not limited to, fluorohydrocarbons, e.g. 1,1,1,2-tetrafluoroethane and 1,1,1,2,3,3,3-heptafluoropropane.

The particle size of the aerosol particles (solid, liquid or solid/liquid particles) is preferably less than 100 µm, more preferably it is in the range of from 0.5 to 10 µm, in particular in the range of from 2 to 6 µm (D50 value, measured by laser diffraction).

Specific aerosol-producing devices which may be used for inhaled administration include, but are not limited to, Cyclohaler®, Diskhaler®, Rotadisk®, Turbohaler®, Autohaler®, Turbohaler®, Novolizer®, Easyhaler®, Aerolizer®, Jethaler®, Diskus®, Ultrahaler® and Mystic® inhalers. The aerosol-producing devices may be combined with spacers or expanders, e.g. Aerochamber®, Nebulator®, Volumatic® and Rondo®, for improving inhalation efficiency.

In case of topical administration, suitable pharmaceutical formulations are, for example, ointments, creams, lotions, pastes, gels, powders, solutions, emulsions, suspensions, oils, sprays, and patches (for example, without being limited thereto, transdermal therapeutic systems).

For parenteral modes of administration such as, for example, intravenous, intraarterial, intramuscular, subcutaneous, intracutaneous, intraperitoneal and intrasternal administration, preferably solutions (for example, without being limited thereto, sterile solutions or isotonic solutions) are used. They are preferably administered by injection or infusion techniques.

In case of intranasal administration, for example, sprays and solutions to be applied in drop form are preferred formulations.

For intraocular administration, solutions to be applied in drop form, gels and ointments are exemplified formulations.

Generally, the pharmaceutical compositions according to the invention can be administered such that the dose of the compound of the invention is in the range customary for PIk1 inhibitors. In particular, a dose in the range of from 0.01 to 5000 mg of the compound of the invention per day is preferred for an average adult patient having a body weight of 70 kg. In this respect, it is to be noted that the dose is dependent, for example, on the specific compound used, the species treated, age, body weight, general health, sex and diet of the subject treated, mode and time of administration, rate of excretion, severity of the disease to be treated and drug combination. In case the pharmaceutical composition of the invention comprises at least one of the compounds of the invention and at least one therapeutic agent selected from anti-neoplastic agents in general, cytostatic agents, cytotoxic agents, antimetabolites, anti-microtubule agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies, proapoptotic agents, hormones, hormone analogues, antibiotic agents, anti-emetic agents, and analgetic agents, the same dose ranges apply to the therapeutic agent.

The pharmaceutical compositions according to the invention can be administered in a single dose per day or in multiple subdoses, for example, 2 to 4 doses per day. A single dose unit of the pharmaceutical composition can contain e.g. from 0.01 mg to 5000 mg, preferably 0.1 mg to 2000 mg, more preferably 0.5 to 1000 mg, most preferably 1 to 500 mg, of the compound of the invention. In case the pharmaceutical composition of the invention comprises at least one of the compounds of the invention and at least one therapeutic agent selected from anti-neoplastic agents in general, cytostatic agents, cytotoxic agents, antimetabolites, anti-microtubule agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies, proapoptotic agents, hormones, hormone analogues, antibiotic agents, anti-emetic agents, and analgetic agents, a single dose unit of the pharmaceutical composition can contain e.g. from 0.01 mg to 5000 mg, preferably 0.1 mg to 2000 mg, more preferably 0.5 to 1000 mg, most preferably 1 to 500 mg, of the therapeutic agent. Furthermore, the pharmaceutical composition can be adapted to weekly, monthly or even more infrequent administration, for example by using an implant, e.g. a subcutaneous or intramuscular implant, by using the compound of the invention in form of a sparingly soluble salt, or by using the compound of the invention coupled to a polymer. Administration of the pharmaceutical composition in a single dose per day is preferred.

In case the pharmaceutical composition of the invention comprises at least one of the compounds of the invention and at least one therapeutic agent selected from anti-neoplastic agents in general, cytostatic agents, cytotoxic agents, antimetabolites, anti-microtubule agents, platinum derivatives, alkylating agents, topoisomerase I inhibitors, topoisomerase II inhibitors, signal transduction inhibitors, cell cycle signalling inhibitors, angiogenesis inhibitors including antibodies and receptor tyrosine kinase inhibitors, immunotherapeutic agents, monoclonal antibodies, proapoptotic agents, hormones, hormone analogues, antibiotic agents, anti-emetic agents, and analgetic agents, administration of the compound of the invention and administration of the therapeutic agent can be made simultaneously or sequentially. In case of sequential administration, the compound of the invention can be administered before or after administration of the therapeutic agent.

### Biological investigations

The inhibition of PIk1 kinase activity can be measured using recombinant PIk1 protein expressed as an N-terminally histidine-tagged protein in SF9 insect cells and alpha casein as substrate. The PIk1 assay is run in 96 well plates by incubating 50 to 100 ng per well recombinant PIk1, 500 ng per well alpha casein (Sigma, # C-6780) as substrate, 10 µl of compound of invention (test compounds were dissolved as 20 mM solutions in dimethylsulfoxide (DMSO) and subsequently diluted), and 100 nM Li-ATP (including ³³P-ATP) in 100 µl of reaction buffer (50 nM HEPES, pH 7.5; 3 mM MgCl₂; 3 mM MnCl₂; 3 µM Na-Orthovanadat; 1 mM DTT; 1 µg/ml PEG 8000) for 80 minutes at 30°C. The reactions are terminated by adding 100 µl stopping buffer (2% H₃PO₄ for 5 minutes) and are washed 3 times with washing solution (0,9% NaCl). Determining ³³P incorporated into the protein substrate alpha casein, the detection is based on the adhesion of the phosphorylated protein to the surface of scintillator-coated flash plates (Perkin Elmer, USA; # SMP-200). Phosphorylation of alpha casein is measured by counting the radioactivity bound to the plate for 60 sec. using a plate reader (Perkin Elmer, USA; Wallac Microbeta). By this method, the IC₅₀ value of the PIk1 inhibition is determined as described above. Preferred compounds are characterized by an IC₅₀ value of PIk1 inhibition of below 1 µM.

**Table 1**

| Example | inhibition of PIk1 | | Example | inhibition of PIk1 | | Example | inhibition of PIk1 |
|---|---|---|---|---|---|---|---|
| 1 | ++ | | 27 | +++ | | 53 | ++++ |
| 2 | +++ | | 28 | ++ | | 54 | ++++ |
| 3 | +++ | | 29 | +++ | | 55 | ++++ |
| 4 | ++++ | | 30 | ++++ | | 56 | ++++ |
| 5 | +++ | | 31 | +++ | | 57 | ++++ |
| 6 | +++ | | 32 | +++ | | 58 | ++++ |
| 7 | +++ | | 33 | ++ | | 59 | ++++ |
| 8 | +++ | | 34 | ++ | | 60 | ++++ |
| 9 | ++ | | 35 | ++ | | 61 | ++++ |
| 10 | +++ | | 36 | +++ | | 62 | ++++ |
| 11 | +++ | | 37 | +++ | | 63 | ++++ |
| 12 | ++ | | 38 | ++++ | | 64 | ++++ |
| 13 | ++ | | 39 | ++++ | | 65 | ++++ |
| 14 | +++ | | 40 | +++ | | 66 | ++++ |
| 15 | ++ | | 41 | ++++ | | 67 | ++++ |
| 16 | ++ | | 42 | +++ | | 68 | ++++ |
| 17 | ++ | | 43 | ++++ | | 69 | ++++ |
| 18 | +++ | | 44 | +++ | | 70 | ++++ |
| 19 | +++ | | 45 | +++ | | 71 | +++ |
| 20 | +++ | | 46 | +++ | | 72 | ++++ |
| 21 | ++ | | 47 | ++++ | | 73 | ++++ |
| 22 | +++ | | 48 | ++++ | | 74 | ++++ |
| 23 | ++ | | 49 | ++++ | | 75 | ++++ |
| 24 | ++ | | 50 | +++ | | 76 | ++++ |
| 25 | ++ | | 51 | ++++ | | 77 | n.d. |
| 26 | +++ | | 52 | ++++ | | | |

| | | | | | | | |
|---|---|---|---|---|---|---|---|
| ++++ pIC50 >8 + pIC50 >=5 +++ pIC50 >7 - pIC50 <5 ++ pIC50 >6 n.d. not determined | | | | | | | |

## Claims

1. Compound of formula (I) 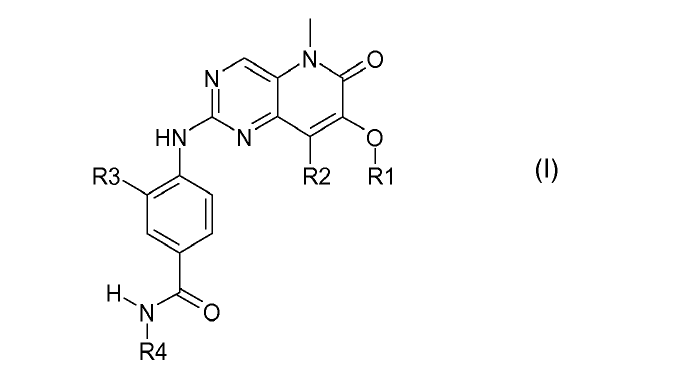 wherein
R1 is C1-C6 alkyl optionally substituted with C1-C4 alkoxy or with 1 to 3 F atoms, C3-C6 cycloalkyl optionally substituted with C1-C4 alkyl or C1-C4 alkoxy, C3-C6 cycloalkyl-C1-C4 alkyl optionally substituted with C1-C4 alkyl, C4-C5 [O]heterocyclyl-C1-C4 alkyl optionally substituted with C1-C4 alkyl, benzyl with phenyl optionally substituted with one or two groups independently selected from R5, or heteroaryl-C1-C2 alkyl with heteroaryl optionally being substituted with one or two groups independently selected from R5;
R2 is H, halogen, C1-C6 alkyl optionally substituted with C1-C4 alkoxy or with 1 to 3 F atoms, C3-C6 cycloalkyl-C1-C4 alkyl, C4-C5 [O]heterocyclylmethyl, C3-C6 cycloalkyl optionally substituted with C1-C4 alkyl or C1-C4 alkoxy, C2-C6 alkenyl, ethinyl optionally substituted with C1-C4 alkyl, C5-C6 cycloalkenyl optionally substituted with C1-C4 alkyl, phenyl optionally substituted with one or two groups independently selected from R5, or heteroaryl optionally substituted with R5;
R3 is halogen, cyano, C1-C4 alkyl optionally substituted with OH or with 1 to 3 F atoms, or C1-C4 alkoxy optionally substituted with OH or with 1 to 3 F atoms;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, cyclopropyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

2. Compound according to claim 1, wherein
R1 is C1-C4 alkyl optionally substituted with C1-C4 alkoxy or with 1 to 3 F atoms, C3-C6 cycloalkyl optionally substituted with C1-C4 alkyl or C1-C4 alkoxy, C3-C6 cycloalkyl-C1-C4 alkyl optionally substituted with C1-C4 alkyl, C4-C5 [O]heterocyclyl-C1-C4 alkyl, benzyl with phenyl optionally substituted with R5, or heteroaryl-C1-C2 alkyl with heteroaryl optionally being substituted with one or two groups independently selected from R5;
R2 is H, halogen, C1-C6 alkyl optionally substituted with C1-C4 alkoxy or with 1 to 3 F atoms, C3-C6 cycloalkyl-C1-C4 alkyl, C4-C5 [O]heterocyclylmethyl, C3-C6 cycloalkyl, C2-C6 alkenyl, ethinyl optionally substituted with C1-C4 alkyl, C5-C6 cycloalkenyl optionally substituted with C1-C4 alkyl, phenyl optionally substituted with R5, or heteroaryl optionally substituted with R5;
R3 is halogen, cyano, C1-C4 alkyl optionally substituted with 1 to 3 F atoms, or C1-C4 alkoxy optionally substituted with 1 to 3 F atoms;
R4 is C1-C4 alkyl, C2-C3 alkyl optionally substituted with -OR6, C2-C3 alkyl optionally substituted with -N(R6)R7, C3-C6 cycloalkyl, C1-C3 alkyl substituted with heteroaryl, the heteroaryl optionally being substituted with one or two groups independently selected from R5, 3-tetrahydropyranyl, 4-tetrahydropyranyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, cyclopropyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

3. Compound according to claim 1, wherein
R1 is C1-C4 alkyl optionally substituted with -F, C3-C6 cycloalkyl, C3-C6 cycloalkyl-C1-C4 alkyl optionally substituted with methyl, C4-C5 [O]heterocyclylmethyl, benzyl or heteroaryl-C1-C2 alkyl with heteroaryl optionally being substituted with one or two groups independently selected from R5;
R2 is H, halogen, C1-C6 alkyl optionally substituted with C1-C4 alkoxy or with 1 to 3 F atoms, C3-C6 cycloalkyl-C1-C4 alkyl, C4-C5 [O]heterocyclylmethyl, C3-C6 cycloalkyl, C2-C6 alkenyl, ethinyl optionally substituted with C1-C4 alkyl, C5-C6 cycloalkenyl optionally substituted with C1-C4 alkyl, phenyl optionally substituted with R5, or heteroaryl optionally substituted with R5;
R3 is F, Cl, C1-C4 alkyl optionally substituted with 1 to 3 F atoms, or C1-C4 alkoxy optionally substituted with 1 to 3 F atoms;
R4 is C2-C3 alkyl optionally substituted with -OR6, C2-C3 alkyl optionally substituted with -N(R6)R7, C3-C6 cycloalkyl, C1-C3 alkyl substituted with heteroaryl, the heteroaryl optionally being substituted with R5, 3-tetrahydropyranyl, 4-tetrahydropyranyl, 4-piperidyl optionally N-substituted by C1-C4 alkyl or C3-C6 cycloalkyl, 4-piperidyl-C1-C2 alkyl, the piperidyl optionally being N-substituted by C1-C4 alkyl or C3-C6 cycloalkyl, or 2-pyrrolidinyl-C1-C2 alkyl, the pyrrolidinyl optionally being N-substituted by C1-C4 alkyl or C3-C6 cycloalkyl;
R5 is halogen, cyano, C1-C4 alkyl, cyclopropyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

4. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl optionally substituted with C1-C4 alkoxy or with 1 to 3 F atoms, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is -Cl, C1-C4 alkyl, or C1-C4 alkoxy;
R4 is C1-C3 alkyl substituted with heteroaryl, the heteroaryl optionally being substituted with R5, 3-tetrahydropyranyl, 4-tetrahydropyranyl, 4-piperidyl optionally N-substituted by C1-C4 alkyl or cyclopropyl, 4-piperidyl-C1-C2 alkyl, the piperidyl optionally being N-substituted by C1-C4 alkyl or cyclopropyl, or 2-pyrrolidinyl-C1-C2 alkyl, the pyrrolidinyl optionally being N-substituted by C1-C4 alkyl or C3-C6 cycloalkyl;
and
R5 is halogen, cyano, C1-C4 alkyl, cyclopropyl, or C1-C4 alkoxy;
a salt of one of the compounds, a stereoisomer of one of the compounds, or a salt of a stereoisomer of one of the compounds.

5. Compound according to claim 1, wherein
R1 is C1-C4 alkyl, cyclopropyl, cyclobutyl, cyclopropylmethyl, 1-methylcyclopropylmethyl, cyclobutylmethyl, or 1-cyclopropylethyl;
R2 is cyclopropyl, (1 E)-prop-1-en-1-yl, isopropenyl, cyclohex-1-en-1-yl optionally substituted with C1-C4 alkyl, cycolopent-1-en-1-yl optionally substituted with C1-C4 alkyl, phenyl optionally substituted with C1-C4 alkyl or F, 2-thienyl optionally substituted with C1-C4 alkyl, 3-thienyl optionally substituted with C1-C4 alkyl, 2-furyl optionally substituted with C1-C4 alkyl, 3-furyl optionally substituted with C1-C4 alkyl, 1H-pyrrol-3-yl optionally substituted with C1-C4 alkyl, or 1H-pyrrol-2-yl optionally substituted with C1-C4 alkyl;
R3 is Cl, methyl, ethyl, methoxy, or ethoxy;
and
R4 is 5-imidazolylmethyl, the imidazolyl optionally being N-substituted by C1-C4 alkyl or cyclopropyl, 4-piperidyl optionally N-substituted by C1-C4 alkyl or cyclopropyl, 4-piperidylmethyl, the piperidyl optionally being N-substituted by C1-C4 alkyl or cyclopropyl, or 2-pyrrolidinylethyl, the pyrrolidinyl optionally being N-substituted by C1-C4 alkyl or cyclopropyl;
a salt of one of the compounds, a stereoisomer of one of the compounds, or a salt of a stereoisomer of one of the compounds.

6. Compound according to claim 1, wherein
R1 is 1-4C-Alkyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

7. Compound according to claim 1, wherein
R1 is cyclopropylmethyl, cyclobutylmethyl, 1-cyclopropylethyl, 1-methylcyclopropylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, heteroaryl optionally substituted with C1-C4 alkyl or halogen;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

8. Compound according to claim 1, wherein
R1 is cyclopropyl or cyclobutyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, heteroaryl optionally substituted with C1-C4 alkyl or halogen;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

9. Compound according to claim 1, wherein
R1 is C1-C4 alkyl optionally substituted with F, C3-C6 cycloalkyl, C3-C6 cycloalkyl-C1-C4 alkyl optionally substituted with methyl, C4-C5 [O]heterocyclylmethyl, benzyl, or heteroaryl-C1-C2 alkyl with heteroaryl optionally being substituted with one or two groups independently selected from R5;
R2 is C3-C6 cycloalkyl;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

10. Compound according to claim 1, wherein
R1 is C1-C4 alkyl optionally substituted with F, C3-C6 cycloalkyl, C3-C6 cycloalkyl-C1-C4 alkyl optionally substituted with methyl, C4-C5 [O]heterocyclylmethyl, benzyl, or heteroaryl-C1-C2 alkyl with heteroaryl optionally being substituted with one or two groups independently selected from R5;
R2 is C2-C6 alkenyl;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

11. Compound according to claim 1, wherein
R1 is C1-C4 alkyl optionally substituted with F, C3-C6 cycloalkyl, C3-C6 cycloalkyl-C1-C4 alkyl optionally substituted with methyl, C4-C5 [O]heterocyclylmethyl, benzyl, or heteroaryl-C1-C2 alkyl with heteroaryl optionally being substituted with one or two groups independently selected from R5;
R2 is C5-C6 cycloalkenyl optionally substituted with C1-C4 alkyl;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

12. Compound according to claim 1, wherein
R1 is C1-C4 alkyl, cyclopropyl, cyclobutyl, cyclopropylmethyl, 1-methylcyclopropylmethyl, cyclobutylmethyl, 1-cyclopropylethyl;
R2 is phenyl optionally substituted with one or two groups independently selected from R5 or heteroaryl optionally substituted with R5;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

13. Compound according to claim 1, wherein
R1 is C1-C4 alkyl, cyclopropyl, cyclobutyl, cyclopropylmethyl, 1-methylcyclopropylmethyl, cyclobutylmethyl, or 1-cyclopropylethyl;
R2 is 2-thienyl optionally substituted with C1-C4 alkyl, 3-thienyl optionally substituted with C1-C4 alkyl, 2-furyl optionally substituted with C1-C4 alkyl, 3-furyl optionally substituted with C1-C4 alkyl, 1H-pyrrol-3-yl optionally substituted with C1-C4 alkyl, or 1H-pyrrol-2-yl optionally substituted with C1-C4 alkyl;
R3 is C1-C4 alkyl or C1-C4 alkoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

14. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is methyl or ethyl;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

15. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is methoxy or ethoxy;
R4 is C1-C4 alkyl, C2-C3 alkyl optionally substituted with -OR6, C2-C3 alkyl optionally substituted with -N(R6)R7, C3-C6 cycloalkyl, C1-C4 alkyl substituted with heteroaryl, the heteroaryl optionally being substituted with one or two groups independently selected from R5, C4-C5 [O]heterocyclyl optionally substituted with C1-C4 alkyl or C3-C6 cycloalkyl, C4-C5 [O]heterocyclyl-C1-C4 alkyl, the C4-C5 [O]heterocyclyl optionally being substituted with C1-C4 alkyl or C3-C6 cycloalkyl, C4-C6 [N]heterocyclyl optionally substituted with C1-C4 alkyl or C3-C6 cycloalkyl, or C4-C6 [N]heterocyclyl-C1-C3 alkyl, the C4-C6 [N]heterocyclyl optionally being substituted with C1-C4 alkyl or C3-C6 cycloalkyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

16. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is C1-C4 alkyl substituted with heteroaryl, the heteroaryl optionally being substituted with one or two groups independently selected from R5;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

17. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is 4-piperidyl, 4-piperidylmethyl, the piperidyl optionally being N-substituted by C1-C4 alkyl or cyclopropyl, or 4-piperidylethyl, the piperidyl optionally being N-substituted by C1-C4 alkyl or cyclopropyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

18. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is 2-pyrrolidinylmethyl, the pyrrolidinyl optionally being N-substituted by C1-C4 alkyl or cyclopropyl, or 2-pyrrolidinylethyl, the pyrrolidinyl optionally being N-substituted by C1-C4 alkyl or cyclopropyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

19. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is N-(1-methylpiperidin-4-yl) or N-(1-methylpiperidin-4-yl)methyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

20. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is N-[2-(1-methylpyrrolidin-2-yl)ethyl];
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

21. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is 3-tetrahydropyranyl or 4-tetrahydropyranyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

22. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is 5-imidazolylmethyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

23. Compound according to claim 1, wherein
R1 is C1-C4 alkyl, C3-C6 cycloalkyl, C3-C6 cycloalkylmethyl optionally substituted with methyl, or C4-C5 [O]heterocyclylmethyl;
R2 is C1-C4 alkyl, C3-C6 cycloalkyl, C2-C4 alkenyl, C5-C6 cycloalkenyl optionally substituted with C1-C4 alkyl, phenyl optionally substituted with C1-C4 alkyl or halogen, or heteroaryl optionally substituted with C1-C4 alkyl or halogen;
R3 is C1-C4 alkyl or C1-C4 alkoxy;
R4 is 2-pyrrolidinylmethyl, the pyrrolidinyl optionally being N-substituted by C1-C4 alkyl or cyclopropyl, or 2-pyrrolidinylethyl, the pyrrolidinyl optionally being N-substituted by C1-C4 alkyl or cyclopropyl;
R5 is halogen, cyano, C1-C4 alkyl, or C1-C4 alkoxy;
R6 is H or C1-C4 alkyl;
and
R7 is H, methyl, or ethyl;
a salt of one of the compounds, a stereoisomer of one of the compounds, or a salt of a stereoisomer of one of the compounds.

24. Compound, pharmaceutically acceptable salt thereof, stereoisomer of the compound or of a pharmaceutically acceptable salt thereof according to any of claims 1 to 23 for use in the treatment or prophylaxis of diseases.

25. Pharmaceutical composition comprising at least one of the compounds, pharmaceutically acceptable salts thereof, stereoisomers of the compound or of a pharmaceutically acceptable salt thereof according to any of claims 1 to 23 together with at least one pharmaceutically acceptable auxiliary.

26. Pharmaceutical composition according to claim 25, further comprising at least one therapeutic agent being an anti-neoplastic agent, a cytostatic agent, a cytotoxic agent, an antimetabolite, a microtubule interfering agent, a platinum derivative, an alkylating agent, a topoisomerase I inhibitor, a topoisomerase II inhibitor, a signal transduction inhibitor, a cell cycle signalling inhibitor, an angiogenesis inhibitor including an antibody or a receptor tyrosine kinase inhibitor, an immunotherapeutic agent, a monoclonal antibody, a proapoptotic agent, a hormone, a hormone analogue, an antibiotic agent, an anti-emetic agent, or an analgetic agent.

27. Use of a compound, a pharmaceutically acceptable salt thereof, a stereoisomer of the compound or of a pharmaceutically acceptable salt thereof according to any of claims 1 to 23 in the manufacture of a pharmaceutical composition inhibiting Plk1.

28. Use of a compound, a pharmaceutically acceptable salt thereof, a stereoisomer of the compound or of a pharmaceutically acceptable salt thereof according to any of claims 1 to 23 in the manufacture of a pharmaceutical composition for the treatment or prophylaxis of neoplasms or other proliferative diseases.

29. Use according to claim 28, wherein the neoplasm is a cancer of the colon including colorectal carcinoma, ovaries, breast, prostate, bladder, or lung, gastrointestinal carcinoma, gastrointestinal stromal tumor, small cell lung cancer, head and neck cancer, a cancer of cervix, pancreas, esophagus, kidney, larynx and hypopharynx, liver, endocrine glands, soft tissue, testis, retinoblastoma and Wilms tumor, endometrial tumors, malignant melanoma, non-Hodgekins lymphoma, Hodgekins lymphoma or other lymphomas, chronic and acute myeloic leukemia, acute lymphoblastic leukemia, T-cell lymphoma, plasma cell neoplasia, and leukemias, hematological malignancies including multiple myeloma, soft tissue sarcoma, osteosarcoma, fibrosarcoma, and other tumors of mesothelial origin, glioma, astrocytoma, cancers of unknown primary site, glioblastoma and other brain tumors and tumors of neuronal origin, germ cell cancers, myelodysplastic syndromes, myeloproliferative syndromes, disorders such as polycythemia vera, essential thrombocytopenia, myelofibrosis, hypereosinophilic syndrome, or other paraneoplastic syndromes.

30. Method of treating or preventing a neoplasm or another proliferative disease comprising administering to a patient in need thereof a therapeutically effective amount of a compound, a pharmaceutically acceptable salt thereof, a stereoisomer of the compound or of a pharmaceutically acceptable salt thereof according to any of claims 1 to 23.

31. Method of treating or preventing a neoplasm according to claim 30, in which the neoplasm is is a cancer of the colon including colorectal carcinoma, ovaries, breast, prostate, bladder, or lung, gastrointestinal carcinoma, gastrointestinal stromal tumor, small cell lung cancer, head and neck cancer, a cancer of cervix, pancreas, esophagus, kidney, larynx and hypopharynx, liver, endocrine glands, soft tissue, testis, retinoblastoma and Wilms tumor, endometrial tumors, malignant melanoma, non-Hodgekins lymphoma, Hodgekins lymphoma or other lymphomas, chronic and acute myeloic leukemia, acute lymphoblastic leukemia, T-cell lymphoma, plasma cell neoplasia, and leukemias, hematological malignancies including multiple myeloma, soft tissue sarcoma, osteosarcoma, fibrosarcoma, and other tumors of mesothelial origin, glioma, astrocytoma, cancers of unknown primary site, glioblastoma and other brain tumors and tumors of neuronal origin, germ cell cancers, myelodysplastic syndromes, myeloproliferative syndromes, disorders such as polycythemia vera, essential thrombocytopenia, myelofibrosis, hypereosinophilic syndrome, or other paraneoplastic syndrome.
